# EUROPEAN PATENT APPLICATION

(11) **EP 2 033 953 A1**
(43) Date of publication of application: **11.03.2009**
(21) Application number: 08170191.4
(22) Date of filing: 13.02.2003
(51) Int. Cl.: C07D 215/38, A61K 31/47, C07D 409/12, C07D 401/12

(54) **Vanilloid receptor modulators**

(30) Priority: 15.02.2002 GB 0203680; 15.02.2002 GB 0203677; 15.02.2002 GB 0203673; 19.04.2002 GB 0209003; 19.04.2002 GB 0209032; 19.04.2002 GB 0209035; 13.09.2002 GB 0221318
(62) Divisional of application: 03739564.7
(71) Applicant: Glaxo Group Limited, Greenford, Middlesex UB6 0NN (GB)
(72) Inventor: Rami, Harshad Kantilal, Harlow, Essex CM19 5AW (GB); Thompson, Mervyn, Harlow, Essex CM19 5AW (GB); MacDonald, Gregor James, Harlow, Essex CM19 5AW (GB); Westaway, Susan Marie, Harlow, Essex CM19 5AW (GB); Mitchell, Darren Jason, Harlow, Essex CM19 5AW (GB)
(74) Representative: Thornley, Rachel Mary

(57) **Abstract**

Certain compounds of formula (I), 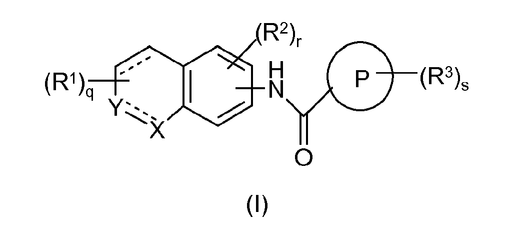 or a pharmaceutically acceptable salt or solvate thereof, wherein R¹, R², R³, P, X, Y, q, r ans s are as defined in the specification, a process for preparing such compounds, a pharmaceutical composition comprising such compounds and the use of such compounds in medicine.

## Description

This invention relates to novel amide derivatives having pharmacological activity, processes for their preparation, to compositions containing them and to their use in medicine, especially in the treatment of various disorders.

Vanilloids are a class of natural and synthetic compounds that are characterised by the presence of a vanillyl (4-hydroxy 3-methoxybenzyl) group or a functionally equivalent group. Vanilloid Receptor (VR-1), whose function is modulated by such compounds, has been widely studied and is extensively reviewed by Szallasi and Blumberg (The American Society for Pharmacology and Experimental Therapeutics, 1999, Vol. 51, No. 2.).

A wide variety of Vanilloid compounds of different structures are known in the art, for example those disclosed in European Patent Application Numbers, EP 0 347 000 and EP 0 401 903, UK Patent Application Number GB 2226313 and International Patent Application, Publication Number WO 92/09285. Particularly notable examples of vanilloid compounds or vanilloid receptor modulators are capsaicin or trans 8-methyl-N-vanillyl-6-nonenamide which is isolated from the pepper plant, capsazepine (Tetrahedron, 53, 1997, 4791) and olvanil or - N-(4-hydroxy-3-methoxybenzyl)oleamide (J. Med. Chem., 36, 1993, 2595).

International Patent Application, Publication Number WO 02/08221 discloses diaryl piperazine and related compounds which bind with high selectivity and high affinity to vanilloid receptors, especially Type I Vanilloid receptors, also known as capsaicin or VR1 receptors. The compounds are said to be useful in the treatment of chronic and acute pain conditions, itch and urinary incontinence.

International Patent Application, Publication Numbers WO 02/16317, WO 02/16318 and WO 02/16319 suggest that compounds having a high affinity for the vanilloid receptor are useful for treating stomach-duodenal ulcers.

US Patent Numbers, US 3,424,760 and US 3,424,761 both describe a series of 3-Ureidopyrrolidines that are said to exhibit analgesic, central nervous system, and pyschopharmacologic activities. These patents specifically disclose the compounds 1-(1-phenyl-3-pyrrolidinyl)-3-phenyl urea and 1-(1-phenyl-3-pyrrolidinyl)-3-(4-methoxyphenyl)urea respectively.

International Patent Applications, Publication Numbers WO 01/62737 and WO 00/69849 disclose a series of pyrazole derivatives which are stated to be useful in the treatment of disorders and diseases associated with the NPY receptor subtype Y5, such as obesity. WO 01/62737 specifically discloses the compound 5-amino-*N*-isoquinolin-5-yl-1-[3-(trifluoromethyl)phenyl]-1*H*-pyrazole-3-carboxamide. WO 00/69849 specifically discloses the compounds 5-methyl-*N-*quinolin-8-yl-1-[3-(trifluoromethyl)phenyl]-1*H*-pyrazole-3-carboxamide, 5-methyl-*N*-quinolin-7-yl-1-[3-trifluoromethyl)phenyl]-1*H*-pyrazole-3-carboxamide, 5-methyl-*N*-quinolin-3-yl-1-[3-(trifluoromethyl)phenyl]-1*H*-pyrazole-3-carboxamide, *N*-isoquinolin-5-yl-5-methyl-1-[3-(trifluoromethyl)phenyl]-1*H*-pyrazole-3-carboxamide, 5-methyl-*N*-quinolin-5-yl-1-[3-(trifluoromethyl)phenyl]-1*H*-pyrazole-3-carboxamide, 1-(3-chlorophenyl)-*N*-isoquinolin-5-yl-5-methyl-1*H*-pyrazole-3-carboxamide, *N*-isoquinolin-5-yl-1-(3-methoxyphenyl)-5-methyl-1*H*-pyrazole-3-carboxamide, 1-(3-fuorophenyl)-*N*-isoquinolin-5-yl-5-methyl-1*H*-pyrazole-3-carboxamide, 1-(2-chloro-5-trifluoromethylphenyl)-*N*-isoquinolin-5-yl-5-methyl-1*H*-pyrazole-3-carboxamide, 5-methyl-*N*-(3-methylisoquinolin-5-yl)-1-[3-(trifluoromethyl)phenyl]-1*H*-pyrazole-3-carboxamide, 5-methyl-*N*-(1,2,3,4-tetrahydroisoquinolin-5-yl)-1-[3-(trifluoromethyl)phenyl]-1*H*-pyrazole-3-carboxamide.

German Patent Application Number 2502588 describes a series of piperazine derivatives. This application specifically discloses the compound N-[3-[2-(diethylamino)ethyl]-1,2-dihydro-4-methyl-2-oxo-7-quinolinyl]-4-phenyl-1-piperazinecarboxamide.

We have now discovered that certain compounds falling within the scope of International Patent Application, Publication Number WO 02/08221 have surprising potency and selectivity as VR-1 antagonists. The compounds of the present invention are considered to be particularly beneficial as VR-1 antagonists as certain compounds exhibit improved aqueous solubility and metabolic stability relative to the compounds disclosed in WO 02/08221.

According to a first aspect of the present invention, there is provided a compound of formula (I), 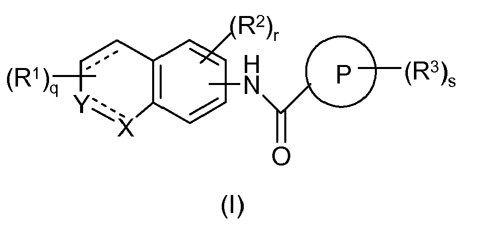 or a pharmaceutically acceptable salt or solvate thereof, wherein, P is selected from phenyl, heteroaryl or heterocyclyl;
R¹ and R² are independently selected from halo, alkyl, alkoxy, cycloalkyl, aralkyl, aralkoxy, cycloalkylalkyl, cycloalkylalkoxy, -CN, -NO₂, -OH, =O, -OCF₃, -CF₃, - NR⁴R⁵, -S(O)ₘR⁶, -S(O)₂NR⁴R⁵, -OS(O)₂R⁶, -OS(O)₂CF₃, -O(CH₂)ₙNR⁴R⁵, -C(O)CF₃, -C(O)alkyl, -C(O)cycloalkyl, -C(O)aralkyl, -C(O)Ar, -C(O)(CH₂)ₙOR⁶, - C(O)(CH₂)ₙNR⁴R⁵, -C(O)alkoxy, -C(O)NR⁴R⁵, -(CH₂)ₙC(O)alkoxy, - (CH₂)ₙOC(O)R⁶, -O(CH₂)ₙOR⁶, -(CH₂)ₙOR⁶, -(CH₂)ₙR⁴R⁵,-(CH₂)ₙC(O)NR⁴R⁵, -(CH₂)ₙN(R⁴)C(O)R⁶, -(CH₂)ₙS(O)₂NR⁴R⁵, - (CH₂)ₙN(R⁴)S(O)₂R⁶, -ZAr, -(CH₂)ₙS(O)₂R⁶, -(OCH₂)ₙS(O)₂R⁶, - N(R⁴)S(O)₂R⁶, -N(R⁴)C(O)R⁶, -(CH₂)ₙN(R⁴)S(O)₂R⁶, -(CH₂)ₙN(R⁴)C(O)R⁶ or -(CH₂)ₙC(O)alkyl;

R³ is selected from alkyl, alkoxy, -CF₃, halo, -O(CH₂)ₙOR⁶, -O(CH₂)ₙNR⁴R⁵, phenyl, cyclohexyl, benzo[1,3]dioxolyl, morpholinyl, pyridyl, pyrimidinyl, pyrazinyl, piperazinyl, piperidinyl, pyridizinyl, thienyl, furyl, pyrazolyl, pyrrolyl, triazolyl, indanyl, imidazolyl, oxazolyl, thiazolyl, oxadiazolyl, isothiazolyl, isoxazolyl or thiadiazolyl; wherein said alkyl, alkoxy, phenyl, cyclohexyl, benzo[1,3]dioxolyl, morpholinyl, pyridyl, pyrimidinyl, pyrazinyl, piperazinyl, piperidinyl, pyridizinyl, thienyl, furyl, pyrazolyl, pyrrolyl, triazolyl, indanyl, imidazolyl, oxazolyl, thiazolyl, oxadiazolyl, isothiazolyl, isoxazolyl and thiadiazolyl groups may be optionally substituted by one or more groups, which may be the same or different, selected from R²;
R⁴ and R⁵ may be the same or different and represent -H or alkyl or R⁴ and R⁵ together with the nitrogen atom to which they are attached form a heterocyclic ring;
R⁶ is -H, alkyl or aryl;
R⁷ is -H, alkyl or aryl;
R⁸ is selected from -H, alkyl, hydroxyalkyl, cycloalkyl, aralkyl, alkoxyalkyl, cycloalkylalkyl, heterocyclylalkyl, -S(O)ₘR⁶, -C(O)CF₃, -C(O)alkyl, - C(O)cycloalkyl, -C(O)aralkyl, -C(O)Ar, -C(O)(CH₂)ₙOR⁶, -C(O)(CH₂)ₙNR⁴R⁵, - C(O)alkoxy, -C(O)NR⁴R⁵, -(CH₂)ₙC(O)alkoxy, -(CH₂)ₙOC(O)R⁶, -(CH₂)ₙOR⁶, - (CH₂)ₙR⁴R⁵, -(CH₂)ₙC(O)NR⁴R⁵, -(CH₂)ₙN(R⁴)C(O)R⁶, -(CH₂)ₙS(O)₂NR⁴R⁵, -(CH₂)ₙN(R⁴)S(O)₂R⁶, -(CH₂)ₙS(O)₂R⁶, -(CH₂)ₙN(R⁴)S(O)₂R⁶,-(CH₂)ₙN(R⁴)C(O)R⁶ or -(CH₂)ₙC(O)alkyl; or where X is NR⁸ and Y is C(R⁹)₂, R⁸ may combine with R¹ to form a benzoquinuclidine group;
R⁹ is -H or R¹;
Ar is aryl or heteroaryl, each of which may be optionally substituted by R²;
Z is a bond, O, S, NR⁷ or CH₂;
m is 0, 1 or 2;
n is an integer value from 1 to 6;
q and r are independently selected from 0, 1, 2 or 3;
s is 0, 1, 2 or 3; and
X and Y are selected from the following combinations:

| X | Y |
|---|---|
| N | CR⁹ |
| NR⁸ | C(R⁹)₂ |
| CR⁹ | N |
| C(R⁹)₂ | NR⁸ |

with the proviso that said compound of formula (I) is not a compound selected from:
*N*-{3-[(*N,N*-Dimethylamino)methyl]-1,2,3,4-tetrahydro-7-quinolinyl}-4-biphenylcarboxamide;
*N*-{3-[(*N,N*-Dimethylamino)methyl]-1-formyl-1,2,3,4-tetrahydro-7-quinolinyl}-4-biphenylcarboxamide;
*N*-{1-Acetyl-3-[(*N,N*-dimethylamino)methyl]-1,2,3,4-tetrahydro-7-quinolinyl}-4-biphenylcarboxamide;
*N*-{3-[(*N,N*-Dimethylamino)methyl]-1-methylsulfonyl-1,2,3,4-tetrahydro-7-quinolinyl}-4-biphenylcarboxamide;
   5-amino-*N*-isoquinolin-5-yl-1-[3-(trifluoromethyl)phenyl]-1H-pyrazole-3-carboxamide;
   5-methyl-*N*-quinolin-8-yl-1-[3-(trifluoromethyl)phenyl]-1*H*-pyrazole-3-carboxamide,
   5-methyl-*N*-quinolin-7-yl-1-[3-trifluoromethyl)phenyl]-1*H*-pyrazole-3-carboxamide,
   5-methyl-*N*-quinolin-3-yl-1-[3-(trifluoromethyl)phenyl]-1*H*-pyrazole-3-carboxamide,
*N*-isoquinolin-5-yl-5-methyl-1-[3-(trifluoromethyl)phenyl]-1*H*-pyrazole-3-carboxamide,
   5-methyl-*N*-quinolin-5-yl-1-[3-(trifluoromethyl)phenyl]-1*H*-pyrazole-3-carboxamide,
   1-(3-chlorophenyl)-*N*-isoquinolin-5-yl-5-methyl-1*H*-pyrazole-3-carboxamide,
*N*-isoquinolin-5-yl-1-(3-methoxyphenyl)-5-methyl-1*H*-pyrazole-3-carboxamide,
   1-(3-fuorophenyl)-*N*-isoquinolin-5-yl-5-methyl-1*H*-pyrazole-3-carboxamide,
   1-(2-chloro-5-trifluoromethylphenyl)-*N*-isoquinolin-5-yl-5-methyl-1*H*-pyrazole-3-carboxamide,
   5-methyl-*N*-(3-methylisoquinolin-5-yl)-1-[3-(trifluoromethyl)phenyl]-1*H*-pyrazole-3-carboxamide,
   5-methyl-*N*-(1,2,3,4-tetrahydroisoquinolin-5-yl)-1-[3-(trifluoromethyl)phenyl]-1*H-*pyrazole-3-carboxamide; and
N-[3-[2-(d iethylam ino)ethyl]-1,2-d ihyd ro-4-methyl-2-oxo-7-q u inol inyl]-4-phenyl-1-piperazinecarboxamide.
Suitably, P is phenyl, pyridyl, furanyl, thienyl, piperazinyl, piperidinyl or fluorenyl. Suitably, P is phenyl or pyridyl. Suitably, P is furanyl, thienyl, piperazinyl, piperidinyl or fluorenyl. More suitably, P is phenyl. More suitably, P is pyridyl. Suitably, R¹ is =O or alkyl. More suitably, R¹ is =O or methyl. Suitably, R² is halo. More suitably, R² is bromo or chloro. Suitably, R³ is alkyl, alkoxy, halo, -CF₃, -O(CH₂)ₙOR⁶, -O(CH₂)ₙNR⁴R⁵, phenyl, thienyl, imidazolyl, pyridyl, pyrazinyl, indanyl, piperazinyl, pyrazolyl, benzo[1,3]dioxolyl, morpholinyl, piperidinyl, cyclohexyl or thiazolyl; wherein said alkyl, phenyl, thienyl, imidazolyl, pyridyl, pyrazinyl, indanyl, piperazinyl, pyrazolyl, benzo[1,3]dioxolyl, morpholinyl, piperidinyl, cyclohexyl and thiazolyl groups may be optionally substituted by one or more groups, which may be the same or different, selected from R². Suitably, R³ is phenyl, alkyl, alkoxy, halo, -CF₃, - O(CH₂)ₙOR⁶, -O(CH₂)ₙNR⁴R⁵, phenyl, thienyl, imidazolyl, pyridyl, pyrazinyl, indanyl, piperazinyl, pyrazolyl, benzo[1,3]dioxolyl, morpholinyl, piperidinyl, cyclohexyl or thiazolyl; wherein said alkyl, phenyl, thienyl, imidazolyl, pyridyl, pyrazinyl, indanyl, piperazinyl, pyrazolyl, benzo[1,3]dioxolyl, morpholinyl, piperidinyl, cyclohexyl and thiazolyl groups may be optionally substituted by one or more groups, which may be the same or different, selected from -H, halo, - CF₃, alkyl, alkoxy, =O, -CONR⁴R⁵, -N(R⁴)C(O)R⁶, -C(O)alkyl, -S(O)₂NR⁴R⁵, - C(O)alkoxy, -O(CH₂)ₙOR⁶ and -O(CH₂)ₙR⁴R⁵. Suitably, R³ is phenyl or pyridyl; each of which may be optionally substituted by one or more groups, which may be the same or different, selected from R². Suitably, R³ is phenyl or pyridyl; each of which may be optionally substituted by one or more groups, which may be the same or different, selected from -H, halo, -CF₃, alkyl, alkoxy, =O, -CONR⁴R⁵, -N(R⁴)C(O)R⁶, -C(O)alkyl, -S(O)₂NR⁴R⁵, -C(O)alkoxy, - O(CH₂)ₙOR⁶ and -O(CH₂)ₙR⁴R⁵. Suitably, R⁴ is -H or alkyl. Suitably, R⁵ is -H or alkyl. Suitably, R⁶ is alkyl. Suitably, R⁸ is -H, alkyl, hydroxyalkyl, alkoxyalkyl, heterocyclylalkyl, -C(O)CF₃, - C(O)alkyl, -C(O)(CH₂)ₙOR⁶, -(CH₂)ₙOC(O)R⁶, -(CH₂)ₙC(O)alkoxy or - (CH₂)ₙR⁴R⁵. More suitably, R⁸ is -H, methyl, -C(O)CF₃, -C(O)Me, - C(O)CH₂OMe, -(CH₂)₂OC(O)Me, -(CH₂)₂CO₂Me, -(CH₂)₂OH, - (CH₂)₂O(CH₂)₂CH₃, -(CH₂)₂OMe, -(CH₂)₂NMe₂, -(CH₂)₂N(Prⁱ)₂ or -(CH₂)₂-morpholinyl. Suitably, R⁹ is H. Suitably R⁹ is R¹. Suitably, q and r are independently selected from 0, 1 or 2. Suitably, q and r are independently selected from 0 or 1. Suitably, s is 0, 1 or 2. Suitably, X is N and Y is CR⁹. Suitably, X is NR⁸ and Y is C(R⁹)₂. Suitably, X is CR⁹ and Y is N. Suitably, X is C(R⁹)₂ and Y is NR⁸. In a further aspect of the present invention there is provided a subset of compounds of formula (I), of formula (IA), 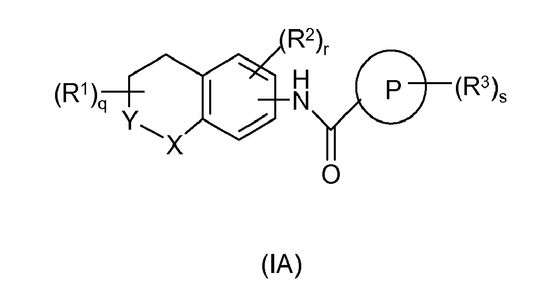 or a pharmaceutically acceptable salt or solvate thereof,
wherein, P is selected from phenyl, heteroaryl or heterocyclyl;
R¹ and R² are independently selected from halo, alkyl, alkoxy, cycloalkyl, aralkyl, aralkoxy, cycloalkylalkyl, cycloalkylalkoxy, -CN, -NO₂, -OH, =O, -OCF₃, -CF₃, - NR⁴R⁵, -S(O)ₘR⁶, -S(O)₂NR⁴R⁵, -OS(O)₂R⁶, -OS(O)₂CF₃, -O(CH₂)ₙNR⁴R⁵, -C(O)CF₃, -C(O)alkyl, -C(O)cycloalkyl, -C(O)aralkyl, -C(O)Ar, -C(O)(CH₂)ₙOR⁶, - C(O)(CH₂)ₙNR⁴R⁵, -C(O)alkoxy, -C(O)NR⁴R⁵, -(CH₂)ₙC(O)alkoxy, - (CH₂)ₙOC(O)R⁶, -O(CH₂)ₙOR⁶, -(CH₂)ₙOR⁶, -(CH₂)ₙR⁴R⁵, - (CH₂)ₙC(O)NR⁴R⁵, -(CH₂)ₙN(R⁴)C(O)R⁶, -(CH₂)ₙS(O)₂NR⁴R⁵, - (CH₂)ₙN(R⁴)S(O)₂R⁶, -ZAr, -(CH₂)ₙS(O)₂R⁶, -(OCH₂)ₙS(O)₂R⁶, - N(R⁴)S(O)₂R⁶, -N(R⁴)C(O)R⁶, -(CH₂)ₙN(R⁴)S(O)₂R⁶, -(CH₂)ₙN(R⁴)C(O)R⁶ or -(CH₂)ₙC(O)alkyl; R³ is selected from alkyl, -CF₃, halo, phenyl, cyclohexyl, benzo[1,3]dioxolyl morpholinyl, pyridyl, pyrimidinyl, pyrazinyl, piperazinyl piperidinyl, pyridizinyl, thienyl, furyl, pyrazolyl, pyrrolyl, triazolyl, indanyl, imidazolyl, oxazolyl, thiazolyl, oxadiazolyl, isothiazolyl, isoxazolyl or thiadiazolyl; wherein said alkyl, alkoxy, phenyl, cyclohexyl, benzo[1,3]dioxolyl, morpholinyl, pyridyl, pyrimidinyl, pyrazinyl, piperazinyl, piperidinyl, pyridizinyl, thienyl, furyl, pyrazolyl, pyrrolyl, triazolyl, indanyl, imidazolyl, oxazolyl, thiazolyl, oxadiazolyl, isothiazolyl, isoxazolyl and thiadiazolyl groups may be optionally substituted by one or more groups, which may be the same or different, selected from R²;
R⁴ and R⁵ may be the same or different and represent -H or alkyl or R⁴ and R⁵ together with the nitrogen atom to which they are attached form a heterocyclic ring;
R⁶ is -H, alkyl or aryl;
R⁷ is -H, alkyl or aryl;
R⁸ is selected from -H, alkyl, hydroxyalkyl, cycloalkyl, aralkyl, alkoxyalkyl, cycloalkylalkyl, heterocyclylalkyl, -S(O)ₘR⁶, -C(O)CF₃, -C(O)alkyl, - C(O)cycloalkyl, -C(O)aralkyl, -C(O)Ar, -C(O)(CH₂)ₙOR⁶, -C(O)(CH₂)ₙNR⁴R⁵, - C(O)alkoxy, -C(O)NR⁴R⁵, -(CH₂)ₙC(O)alkoxy, -(CH₂)ₙOC(O)R⁶, -(CH₂)ₙOR⁶, - (CH₂)ₙR⁴R⁵, -(CH₂)ₙC(O)NR⁴R⁵, -(CH₂)ₙN(R⁴)C(O)R⁶, -(CH₂)ₙS(O)₂NR⁴R⁵, -(CH₂)ₙN(R⁴)S(O)₂R⁶, -(CH₂)ₙS(O)₂R⁶, -(CH₂)ₙN(R⁴)S(O)₂R⁶, - (CH₂)ₙN(R⁴)C(O)R⁶ or -(CH₂)ₙC(O)alkyl; or where X is NR⁸ and Y is C(R⁹)₂, R⁸ may combine with R¹ to form a benzoquinuclidine group;
R⁹ is -H or R¹.
Ar is aryl or heteroaryl, each of which may be optionally substituted by R²;
Z is a bond, O, S, NR⁷ or CH₂;
m is 0, 1 or 2;
n is an integer value from 1 to 6;
q and r are independently selected from 0, 1, 2 or 3;
s is 0, 1, 2 or 3; and
X is C(R⁹)₂ and Y is NR⁸ or X is NR⁸ and Y is C(R⁹)₂; with the proviso that said compound of formula (I) is not a compound selected from: *N*-{3-[(*N,N*-Dimethylamino)methyl]-1,2,3,4-tetrahydro-7-quinolinyl}-4-biphenylcarboxamide; *N*-{3-[(*N,N*-Dimethylamino)methyl]-1-formyl-1,2,3,4-tetrahydro-7-quinolinyl}-4-biphenylcarboxamide; *N*-{1-Acetyl-3-[(*N,N*-dimethylamino)methyl]-1,2,3,4-tetrahydro-7-quinolinyl}-4-biphenylcarboxamide; *N*-{3-[(*N,N*-Dimethylamino)methyl]-1-methylsulfonyl-1,2,3,4-tetrahydro-7-quinolinyl}-4-biphenylcarboxamide; and 5-methyl-*N*-(1,2,3,4-tetrahydroisoquinolin-5-yl)-1-[3-(trifluoromethyl)phenyl]-1*H-*pyrazole-3-carboxamide. Suitably, P is phenyl, pyridyl, furyl, thienyl or piperazinyl. Suitably, P is phenyl. Suitably, P is pyridyl. Suitably, R¹ is alkyl. More suitably, R¹ is methyl. Suitably, R² is halo or alkyl. Suitably, R³ is alkyl, phenyl, indanyl, pyridyl, pyrazinyl, pyrazolyl or thienyl; each of which may be optionally substituted by one or more groups, which may be the same or different, selected from R². More suitably, R³ is alkyl, phenyl or pyridyl; which phenyl and pyridyl groups may be optionally substituted by alkyl, halo, - CF₃, -CONHMe, -NHCOMe, -CONMe₂, -C(O)Me, -SO₂NHMe, -CONH₂. Suitably, R⁸ is -H, alkyl, hydroxyalkyl, alkoxyalkyl, heterocyclylalkyl, -C(O)CF₃, - C(O)alkyl, -C(O)(CH₂)ₙOR⁶, -(CH₂)ₙOC(O)R⁶, -(CH₂)ₙC(O)alkoxy or - (CH₂)ₙR⁴R⁵. More suitably, R⁸ is -H, methyl, -C(O)CF₃, -C(O)Me, - C(O)CH₂OMe, -(CH₂)₂OC(O)Me, -(CH₂)₂CO₂Me, -(CH₂)₂OH, - (CH₂)₂O(CH₂)₂CH₃, -(CH₂)₂OMe, -(CH₂)₂NMe₂, -(CH₂)₂N(Prⁱ)₂ or -(CH₂)₂-morpholinyl. Suitably, R⁹ is H. Suitably, R⁹ is R¹. Suitably, m is 2. Suitably, n is 1 or 2. Suitably, q and r are independently selected from 0, 1 or 2. Suitably, s is 0, 1 or 2. Suitably, X is C(R⁹)₂ and Y is NR⁸. Suitably, or X is NR⁸ and Y is C(R⁹)₂.

In a further aspect of the present invention there is provided a subset of compounds of formula (I), of formula (IB), 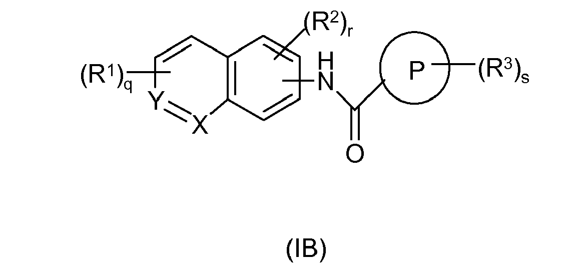 or a pharmaceutically acceptable salt or solvate thereof,
wherein, P is selected from phenyl, heteroaryl or heterocyclyl;
R¹ and R² are independently selected from halo, alkyl, alkoxy, cycloalkyl, aralkyl, aralkoxy, cycloalkylalkyl, cycloalkylalkoxy, -CN, -NO₂, -OH, -OCF₃, -CF₃, - NR⁴R⁵, -S(O)ₘR⁶, -S(O)₂NR⁴R⁵, -OS(O)₂R⁶, -OS(O)₂CF₃, -O(CH₂)ₙNR⁴R⁵, -C(O)CF₃, -C(O)alkyl, -C(O)cycloalkyl, -C(O)aralkyl, -C(O)Ar, -C(O)(CH₂)ₙOR⁶, - C(O)(CH₂)ₙNR⁴R⁵, -C(O)alkoxy, -C(O)NR⁴R⁵, -(CH₂)ₙC(O)alkoxy, - (CH₂)ₙOC(O)R⁶, -(CH₂)ₙOR⁶, -(CH₂)ₙR⁴R⁵, -(CH₂)ₙC(O)NR⁴R⁵, - (CH₂)ₙN(R⁴)C(O)R⁶, -(CH₂)ₙS(O)₂NR⁴R⁵, -(CH₂)ₙN(R⁴)S(O)₂R⁶, -ZAr, - (CH₂)ₙS(O)₂R⁶, -(OCH₂)ₙS(O)₂R⁶, -N(R⁴)S(O)₂R⁶, -N(R⁴)C(O)R⁶, - (CH₂)ₙN(R⁴)S(O)₂R⁶, -(CH₂)ₙN(R⁴)C(O)R⁶ or -(CH₂)ₙC(O)alkyl;

R³ is selected from halo, -CF₃, alkyl, alkoxy, -O(CH₂)ₙOR⁶, -O(CH₂)ₙNR⁴R⁵, phenyl, cyclohexyl, benzo[1,3]dioxolyl, morpholinyl, pyridyl, pyrimidinyl, pyrazinyl, piperazinyl, piperidinyl, pyridizinyl, thienyl, furyl, pyrazolyl, pyrrolyl, triazolyl, imidazolyl, oxazolyl, thiazolyl, oxadiazolyl, isothiazolyl, isoxazolyl or thiadiazolyl; which alkyl, alkoxy, phenyl, cyclohexyl, benzo[1,3]dioxolyl, morpholinyl, pyridyl, pyrimidinyl, pyrazinyl, piperazinyl, piperidinyl, pyridizinyl, thienyl, furyl, pyrazolyl, pyrrolyl, triazolyl, imidazolyl, oxazolyl, thiazolyl, oxadiazolyl, isothiazolyl, isoxazolyl and thiadiazolyl groups may be optionally substituted by one or more groups, which may be the same or different, selected from R²;
R⁴ and R⁵ may be the same or different and represent -H or alkyl or R⁴ and R⁵ together with the nitrogen atom to which they are attached form a heterocyclic ring;
R6 is -H, alkyl or aryl;
R⁷ is -H, alkyl or aryl;
Ar is aryl or heteroaryl; each of which may be optionally substituted by R²;
X and Y are selected from CR⁹ and N with the proviso that X and Y may not be the same;
Z is a bond, O, S, NR⁷ or CH₂;
m is 0, 1 or 2;
n is an integer value from 1 to 6;
q and r are independently selected from 0, 1, 2 or 3; and
s is 0, 1, 2 or 3; with the proviso that said compound of formula (IB) is not a compound selected from: 5-amino-*N*-isoquinolin-5-yl-1-[3-(trifluoromethyl)phenyl]-1*H*-pyrazole-3-carboxamide; 5-methyl-*N*-quinolin-8-yl-1-[3-(trifluoromethyl)phenyl]-1*H*-pyrazole-3-carboxamide, 5-methyl-*N*-quinolin-7-yl-1-[3-trifluoromethyl)phenyl]-1*H*-pyrazole-3-carboxamide, 5-methyl-*N*-quinolin-3-yl-1-[3-(trifluoromethyl)phenyl]-1*H*-pyrazole-3-carboxamide, *N*-isoquinolin-5-yl-5-methyl-1-[3-(trifluoromethyl)phenyl]-1*H*-pyrazole-3-carboxamide, 5-methyl-*N*-quinolin-5-yl-1-[3-(trifluoromethyl)phenyl]-1*H*-pyrazole-3-carboxamide, 1-(3-chlorophenyl)-*N*-isoquinolin-5-yl-5-methyl-1*H*-pyrazole-3-carboxamide, *N*-isoquinolin-5-yl-1-(3-methoxyphenyl)-5-methyl1-1*H*-pyrazole-3-carboxamide, 1-(3-fuorophenyl)-*N*-isoquinolin-5-yl-5-methyl-1*H*-pyrazole-3-carboxamide, 1-(2-chloro-5-trifluoromethylphenyl)-*N*-isoquinolin-5-yl-5-methyl-1*H*-pyrazole-3-carboxamide, 5-methyl-*N*-(3-methylisoquinolin-5-yl)-1-[3-(trifluoromethyl)phenyl]-1*H*-pyrazole-3-carboxamide; and N-[3-[2-(d iethylam ino)ethyl]-1,2-d ihyd ro-4-methyl-2-oxo-7-q u inol inyl]-4-phenyl-1-piperazinecarboxamide. Suitably, P is phenyl, pyridine, piperazine, piperidine or fluorene. Suitably, P is phenyl. Suitably, P is pyridine. Suitably, R¹ is alkyl. More suitably, R¹ is methyl. Suitably, R² is halo. More suitably, R² is chloro. Suitably, R³ is halo, -CF₃, alkyl, alkoxy, -O(CH₂)ₙOR⁶, -O(CH₂)ₙNR⁴R⁵, phenyl, cyclohexyl, benzo[1,3]dioxolyl, morpholinyl, pyridyl, piperazinyl, piperidinyl, pyrazolyl, thienyl, isothiazolyl; which alkyl, phenyl, cyclohexyl, benzo[1,3]dioxolyl, morpholinyl, pyridyl, piperazinyl, piperidinyl, pyrazolyl, thienyl, and isothiazolyl groups may be optionally substituted by one or more groups, which may be the same or different, selected from R². More suitably, R³ is halo, -CF₃, alkyl, alkoxy, -O(CH₂)ₙOR⁶, -O(CH₂)ₙNR⁴R⁵, phenyl, cyclohexyl, benzo[1,3]dioxolyl, morpholinyl, pyridyl, piperazinyl, piperidinyl, pyrazolyl, thienyl, isothiazolyl; which alkyl, phenyl, cyclohexyl, benzo[1,3]dioxolyl, morpholinyl, pyridyl, piperazinyl, piperidinyl, pyrazolyl, thienyl, and isothiazolyl groups may be optionally substituted by one or more groups, which may be the same or different, selected from halo, -CF₃, alkyl, alkoxy, -C(O)alkyl, -C(O)alkoxy and - S(O)₂NR⁴R⁵. Suitably, q is 0 or 1. Suitably, r is 0 or 1. Suitably, s is 0, 1 or 2. Suitably, X is CR⁹ and Y is N. Suitably, X is N and Y is CR⁹.

Preferred compounds according to this invention include Examples 1-133 (as shown below) or pharmaceutically acceptable salts or solvates thereof.

| | | |
|---|---|---|
| Example 1 | Example 2 | Example 3 |
| 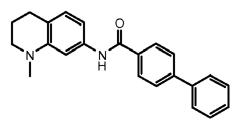 | 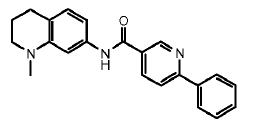 | 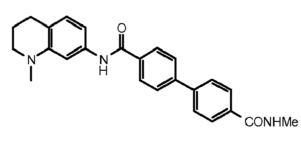 |
| Example 4 | Example 5 | Example 6 |
| 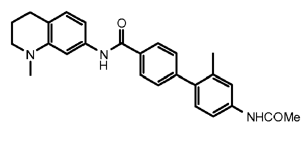 | 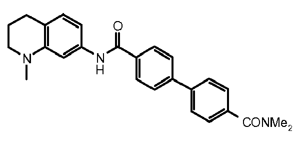 | 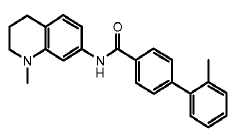 |
| Example 7 | Example 8 | Example 9 |
| 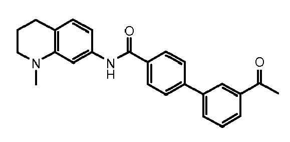 | 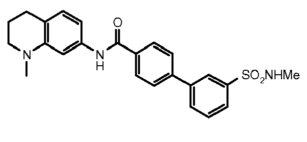 | 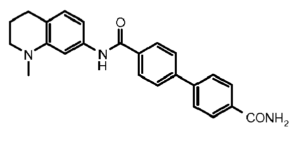 |
| Example 10 | Example 11 | Example 12 |
| 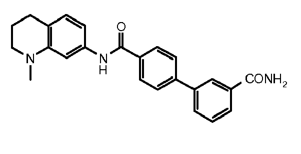 | 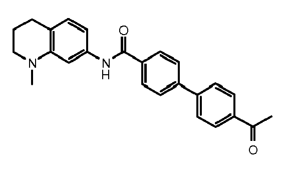 | 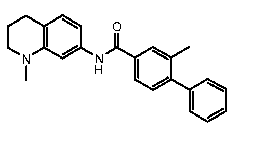 |
| Example 13 | Example 14 | Example 15 |
| 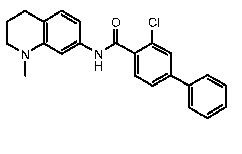 | 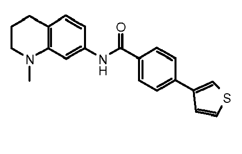 | 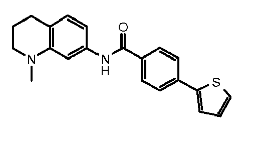 |
| Example 16 | Example 17 | Example 18 |
| 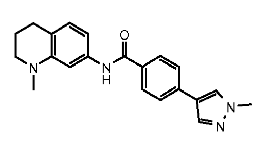 | 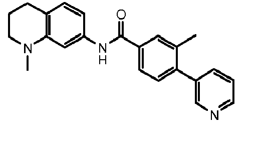 | 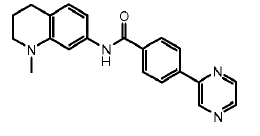 |
| Example 19 | Example 20 | Example 21 |
| 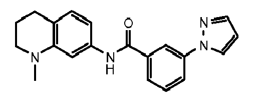 | 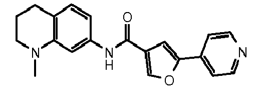 | 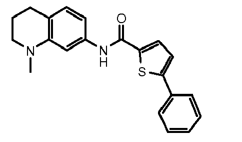 |
| Example 22 | Example 23 | Example 24 |
| 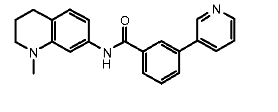 | 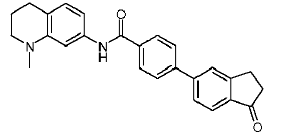 | 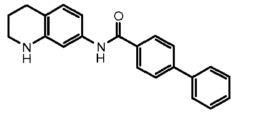 |
| Example 25 | Example 26 | Example 27 |
| 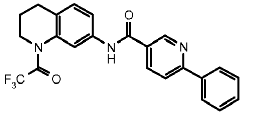 | 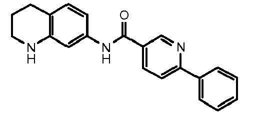 | 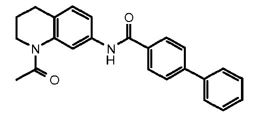 |
| Example 28 | Example 29 | Example 30 |
| 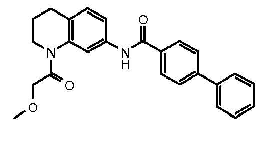 | 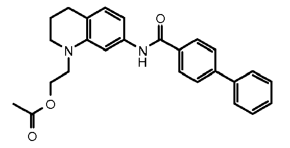 | 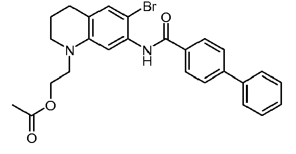 |
| Example 31 | Example 32 | Example 33 |
| 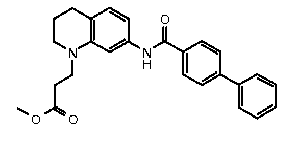 | 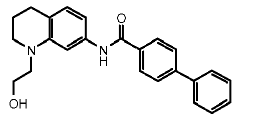 | 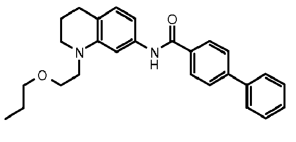 |
| Example 34 | Example 35 | Example 36 |
| 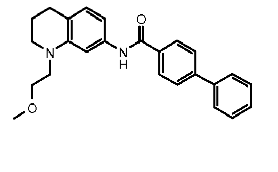 | 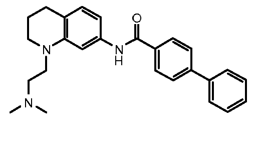 | 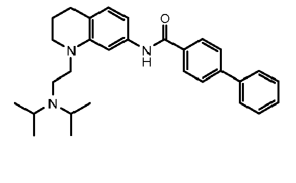 |
| Example 37 | Example 38 | Example 39 |
| 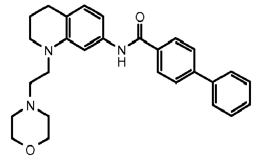 | 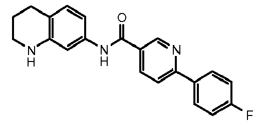 | 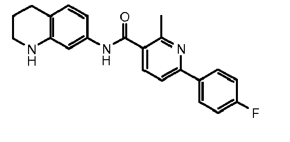 |
| Example 40 | Example 41 | Example 42 |
| 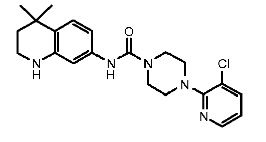 | 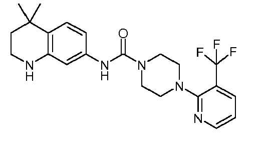 | 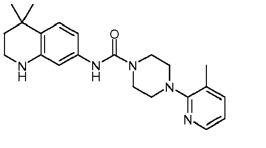 |
| Example 43 | Example 44 | Example 45 |
| 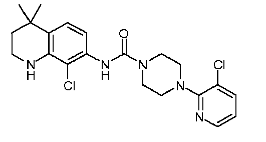 | 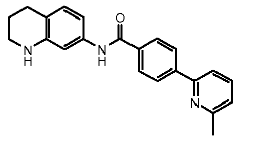 | 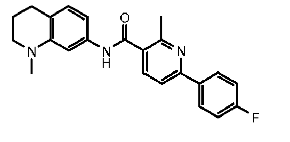 |
| Example 46 | Example 47 | Example 48 |
| 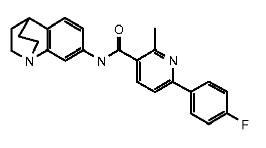 | 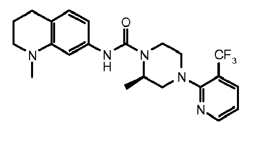 | 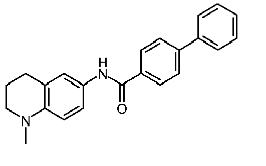 |
| Example 49 | Example 50 | Example 51 |
| 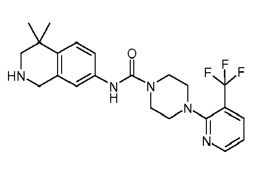 | 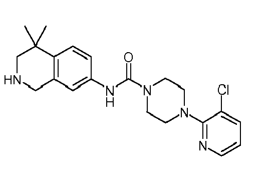 | 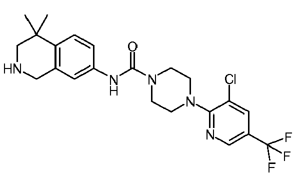 |
| Example 52 | Example 53 | Example 54 |
| 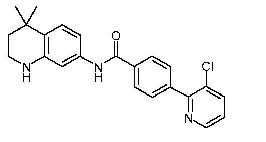 | 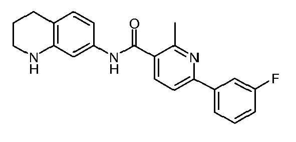 | 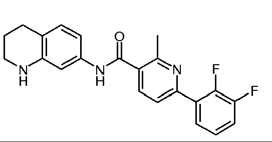 |
| Example 55 | Example 56 | Example 57 |
| 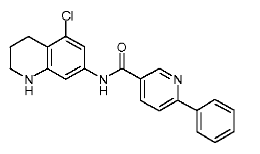 | 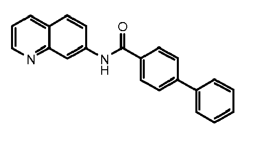 | 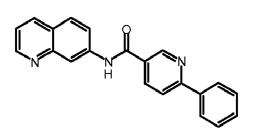 |
| Example 58 | Example 59 | Example 60 |
| 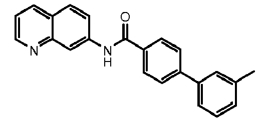 | 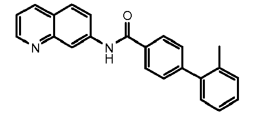 | 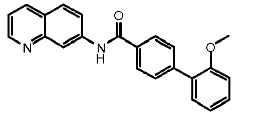 |
| Example 61 | Example 62 | Example 63 |
| 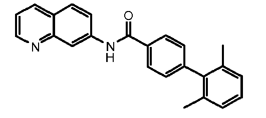 | 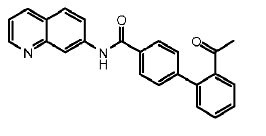 | 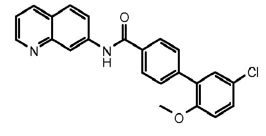 |
| Example 64 | Example 65 | Example 66 |
| 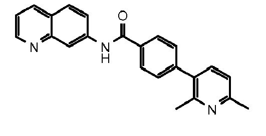 | 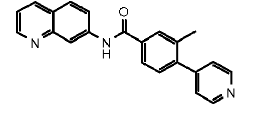 | 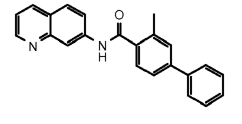 |
| Example 67 | Example 68 | Example 69 |
| 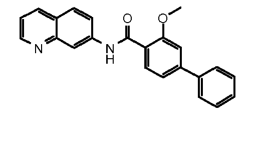 | 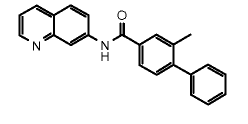 | 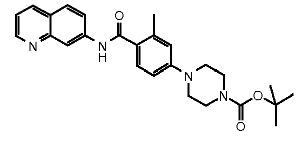 |
| Example 70 | Example 71 | Example 72 |
| 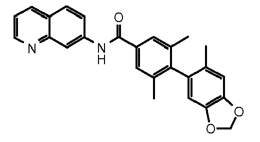 | 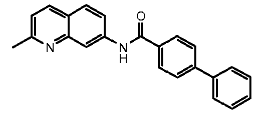 | 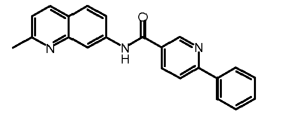 |
| Example 73 | Example 74 | Example 75 |
| 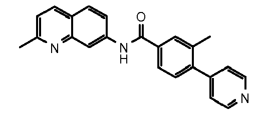 | 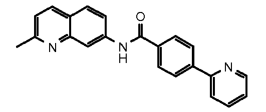 | 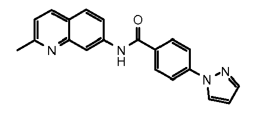 |
| Example 76 | Example 77 | Example 78 |
| 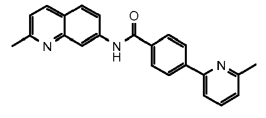 | 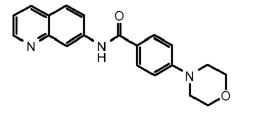 | 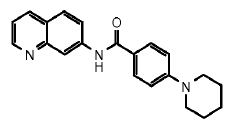 |
| Example 79 | Example 80 | Example 81 |
| 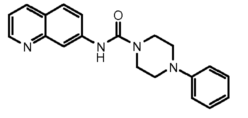 | 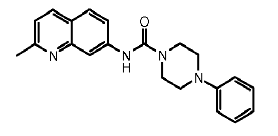 | 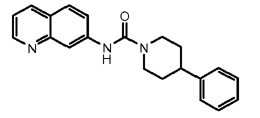 |
| Example 82 | Example 83 | Example 84 |
| 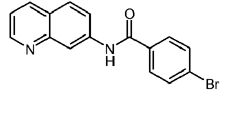 | 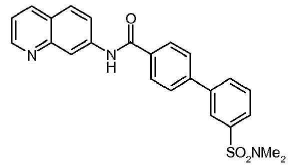 | 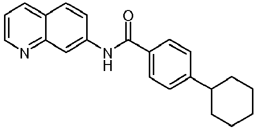 |
| Example 85 | Example 86 | Example 87 |
| 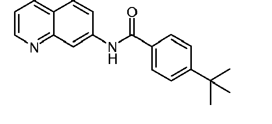 | 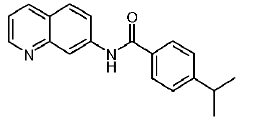 | 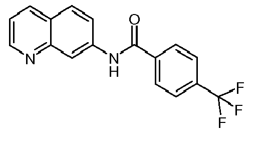 |
| Example 88 | Example 89 | Example 90 |
| 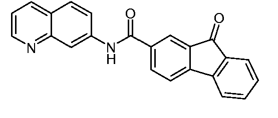 | 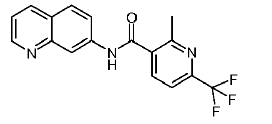 | 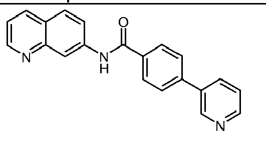 |
| Example 91 | Example 92 | Example 93 |
| 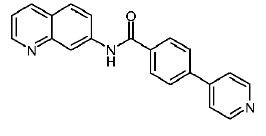 | 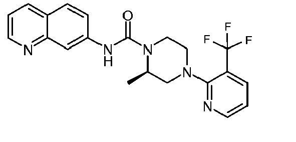 | 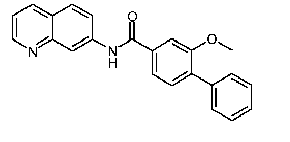 |
| Example 94 | Example 95 | Example 96 |
| 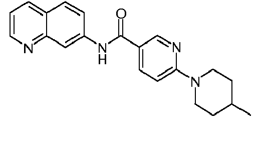 | 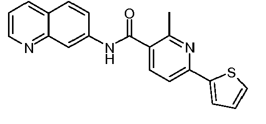 | 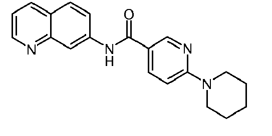 |
| Example 97 | Example 98 | Example 99 |
| 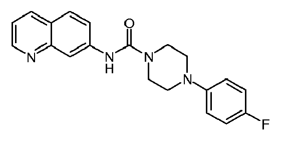 | 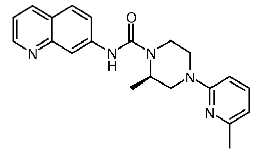 | 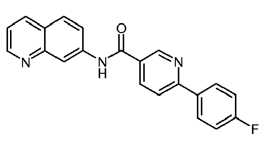 |
| Example 100 | Example 101 | Example 102 |
| 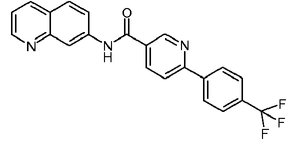 | 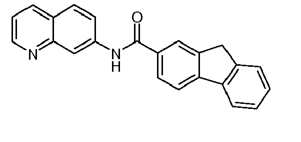 | 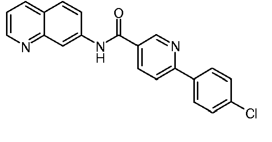 |
| Example 103 | Example 104 | Example 105 |
| 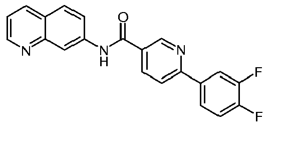 | 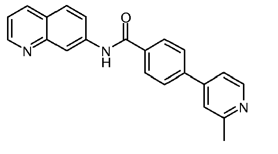 | 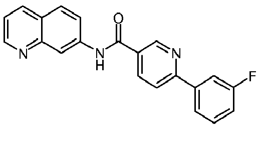 |
| Example 106 | Example 107 | Example 108 |
| 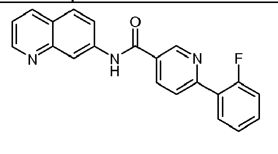 | 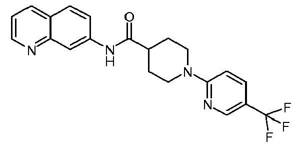 | 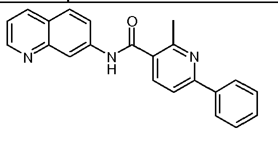 |
| Example 109 | Example 110 | Example 111 |
| 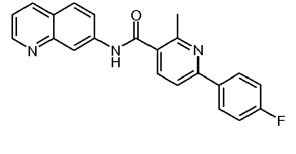 | 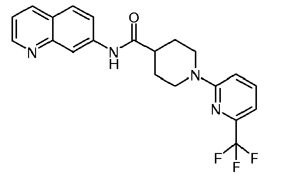 | 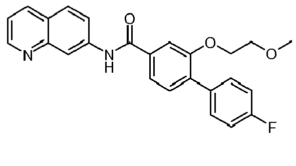 |
| Example 112 | Example 113 | Example 114 |
| 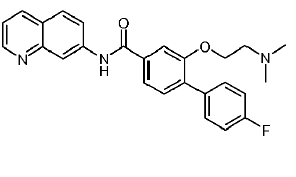 | 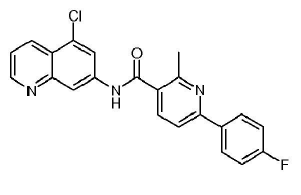 | 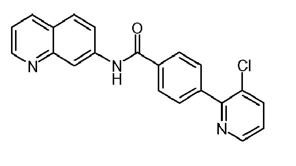 |
| Example 115 | Example 116 | Example 117 |
| 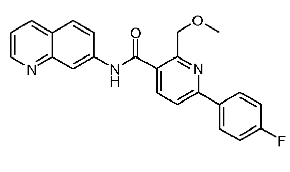 | 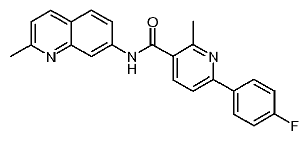 | 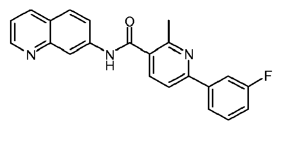 |
| Example 118 | Example 119 | Example 120 |
| 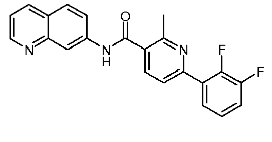 | 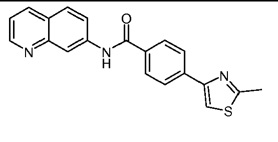 | 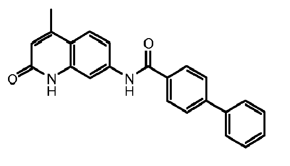 |
| Example 121 | Example 122 | Example 123 |
| 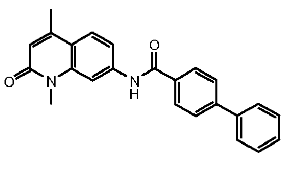 | 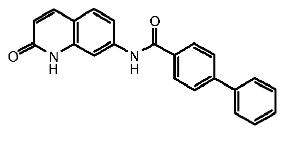 | 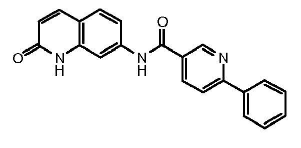 |
| Example 124 | Example 125 | Example 126 |
| 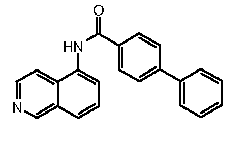 | 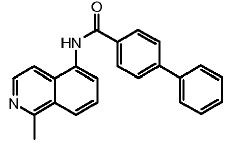 | 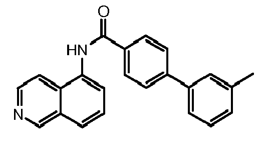 |
| Example 127 | Example 128 | Example 129 |
| 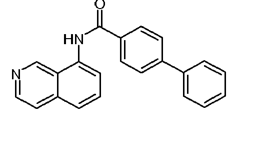 | 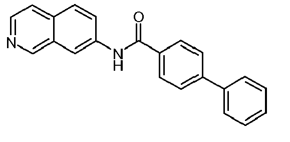 | 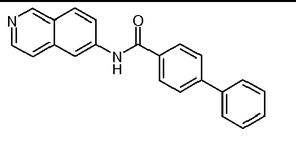 |
| Example 130 | Example 131 | Example 132 |
| 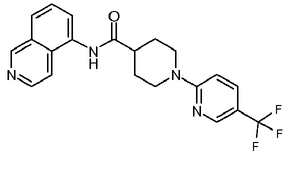 | 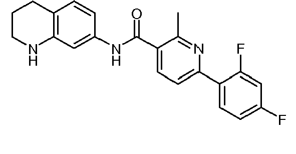 | 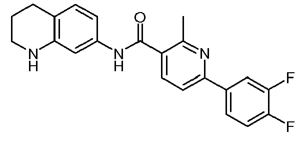 |
| Example 133 | | |
| 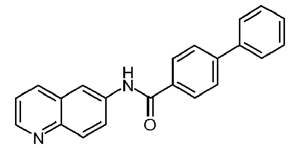 | | |

Certain of the carbon atoms of formula (I) are chiral carbon atoms, and therefore compounds of formula (I) may exist as stereoisomers. The invention extends to all optical isomers such as stereoisomeric forms of the compounds of formula (I) including enantiomers and mixtures thereof, such as racemates. The different stereoisomeric forms may be separated or resolved one from the other by conventional methods or any given isomer may be obtained by conventional stereospecific or asymmetric syntheses.

As indicated above, the compounds of formula (I) can form salts, especially pharmaceutically acceptable salts. Suitable pharmaceutically acceptable salts are those used conventionally in the art and include those described in J. Pharm. Sci., 1977, 66, 1-19, such as acid addition salts.
Suitable pharmaceutically acceptable salts include acid addition salts.
Suitable pharmaceutically acceptable acid addition salts include salts with inorganic acids such, for example, as hydrochloric acid, hydrobromic acid, orthophosphoric acid or sulphuric acid, or with organic acids such, for example as methanesulphonic acid, toluenesulphonic acid, acetic acid, propionic acid, lactic acid, citric acid, fumaric acid, malic acid, succinic acid, salicylic acid, maleic acid, glycerophosphoric acid or acetylsalicylic acid.

The salts and/or solvates of the compounds of the formula (I) which are not pharmaceutically acceptable may be useful as intermediates in the preparation of pharmaceutically acceptable salts and/or solvates of compounds of formula (I) or the compounds of the formula (I) themselves, and as such form another aspect of the present invention.

The compounds of formula (I) may be prepared in crystalline or non-crystalline form, and if crystalline, may be optionally hydrated or solvated. This invention includes in its scope stoichiometric hydrates as well as compounds containing variable amounts of water.

Suitable solvates include pharmaceutically acceptable solvates, such as hydrates.

Solvates include stoichiometric solvates and non-stoichiometric solvates. As used herein the term "alkyl" as a group or part of a group refers to a straight or branched chain saturated aliphatic hydrocarbon radical containing 1 to 12 carbon atoms, suitably 1 to 6 carbon atoms. Such alkyl groups in particular include methyl ("Me"), ethyl ("Et"), n-propyl ("Prⁿ"), *iso*-propyl ("Prⁱ"), n-butyl ("Buⁿ"), *sec*-butyl ("Bu^{s}"), *tert*-butyl ("Bu^{t}"), pentyl and hexyl. Where appropriate, such alkyl groups may be substituted by one or more groups selected from halo (such as fluoro, chloro, bromo), -CN, -CF₃, -OH, -OCF₃, C₂₋₆ alkenyl, C₃₋₆ alkynyl, C₁₋₆ alkoxy, aryl and di-C₁₋₆ alkylamino.

As used herein, the term "alkoxy" as a group or part of a group refers to an alkyl ether radical, wherein the term "alkyl" is defined above. Such alkoxy groups in particular include methoxy, ethoxy, n-propoxy, iso-propoxy, n-butoxy, iso-butoxy, sec-butoxy and *tert*-butoxy. Where appropriate, such alkoxy groups may be substituted by one or more groups selected from halo (such as fluoro, chloro, bromo), -CN, -CF₃, -OH, -OCF₃, C₁₋₆ alkyl, C₂₋₆ alkenyl, C₃₋₆ alkynyl, aryl and di-C₁₋₆ alkylamino.

As used herein, the term "aryl" as a group or part of a group refers to a carbocyclic aromatic radical ("Ar"). Suitably such aryl groups are 5-6 membered monocyclic groups or 8-10 membered fused bicyclic groups, especially phenyl ("Ph"), biphenyl and naphthyl, particularly phenyl.

The term "naphthyl" is used herein to denote, unless otherwise stated, both naphth-1-yl and naphth-2-yl groups.

As used herein, the term "heteroaryl" as a group or part of a group refers to a stable 5- 7-membered monocyclic or 7- to 10-membered bicyclic heterocyclic aromatic ring which consists of carbon atoms and from 1 to 4 heteroatoms independently selected from the group consisting of N, O and S. It is preferred that the total number of S and O atoms in the aromatic heterocycle is not more than 1. Examples of suitable heteroaryl groups include, but are not limited to, acridinyl, azocinyl, benzimidazolyl, benzofuranyl, benzothiofuranyl, benzothiophenyl, benzoxazolyl, benzthiazolyl, benztriazolyl, benztetrazolyl, benzisoxazolyl, benzisothiazolyl, benzimidazolyl, carbazolyl, carbolinyl, chromanyl, chromenyl, cinnolinyl, decahydroquinolinyl, 2H, 6H-1,5,2-dithiazinyl, dihydrobenzofuranyl, furanyl, furazanyl, imidazolyl, 1H-indazolyl, indolinyl, indolyl, isobenzofuranyl, isochromanyl, isoindazolyl, isoindolyl, isoquinolinyl, isothiazolyl, isoxazolyl, naphthyridinyl, oxadiazolyl, 1,2,3-oxadiazolyl, 1,2,4-oxadiazolyl, 1,2,5-oxadiazolyl, 1,3,4-oxadiazolyl, oxazolyl, pyrimidinyl, phthalazinyl, pteridinyl, purinyl, pyrazinyl, pyrazolyl, pyridooxazolyl, pyridoimidazolyl, pyridothiazolyl, pyridyl, pyrrolyl, quinazolinyl, quinolinyl, quinoxalinyl, tetrahydroisoquinolinyl, tetrahydroquinolinyl, 6H-1,2,5-thiadiazinyl, 1,2,3-thiadiazolyl, 1,2,4-thiadiazolyl, 1,2,5-thiadiazolyl, 1,3,4-thiadiazolyl, thiazolyl, thienyl, thienothiazolyl, thienooxazolyl, thienoimidazolyl, triazinyl, 1,2,3-triazolyl, 1,2,4-triazolyl, 1,2,5-triazolyl, 1,3,4-triazolyl and xanthenyl.

As used herein, the terms "heterocyclyl" and "heterocyclic" as a group or part of a group refer to stable heterocyclic non-aromatic single and fused rings containing one or more heteroatoms independently selected from nitrogen, oxygen and sulfur. A fused heterocyclyl ring system may include carbocyclic rings and need include only one heterocyclic ring. Examples of suitable heterocyclyl groups include, but are not limited to, piperazinyl, homopiperazinyl, piperidinyl, pyrrolidinyl and morpholinyl.

The term "halo" is used herein to describe, unless otherwise stated, a group selected from fluorine ("fluoro"), chlorine ("chloro"), bromine ("bromo") or iodine ("iodo").

The present invention also provides a process for the preparation of a compound of formula (I) or a pharmaceutically acceptable salt or solvate thereof, which process comprises: (a) reacting a compound of formula (II): 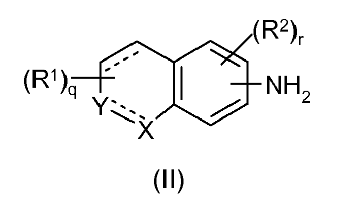 wherein, R¹, R², q, r, X and Y are as defined in relation to formula (I), with a compound of formula (III): 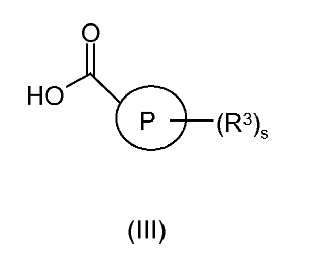 wherein, P, R³ and s are as defined in relation to formula (I) and thereafter, as necessary, carrying out one or more of the following reactions: (i) converting one compound of formula (I) into another compound of formula (I); (ii) removing any protecting group; (iii) preparing a salt or a solvate of the compound so formed.

The reaction between a compound of formula (II) and a compound of formula (III) may be effected using conventional methods for the formation of an amide bond, such as those described in J March, Advanced Organic Chemistry, 4th edition, J Wiley & Sons, 1992, p. 419-421. Typically, the reaction may be carried out in a solvent such as dichloromethane, in the presence of a suitable diimide, such as 1-(3-dimethylaminopropyl)-3-ethylcarbodiimide hydrochloride.

According to a further aspect of the present invention there is provided an alternative process for the preparation of a compound of formula (I) or a pharmaceutically acceptable salt or solvate thereof where P is phenyl or heteroaryl, which process comprises reacting a compound of formula (II) with a compound of formula (IV), 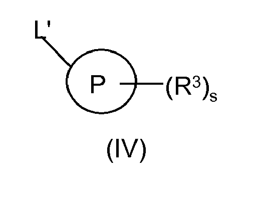 wherein, R³ and s are as defined in relation to formula (I), P is phenyl or heteroaryl and L' is selected from iodo, bromo or -OSO₂CF₃, in the presence of carbon monoxide and a suitable catalyst; and thereafter, as necessary, carrying out one or more of the following reactions:
(i) converting one compound of formula (I) into another compound of formula (I);
(ii) removing any protecting group;
(iii) preparing a salt or a solvate of the compound so formed.
   A suitable catalyst is *trans-bis*-triphenylphosphinepalladium(II)bromide.
   According to still a further aspect of the present invention there is provided an alternative process for the preparation of a compound of formula (I) where P is heterocyclyl, or a pharmaceutically acceptable salt or solvate thereof, which process comprises reacting a compound of formula (II) with a compound of formula (V): 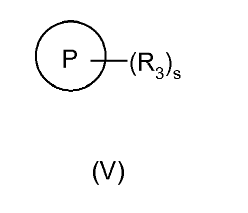
wherein, P is heterocyclyl and R³ and s are as defined in relation to formula (I); and thereafter, as necessary, carrying out one or more of the following reactions:
(i) converting one compound of formula (I) into another compound of formula (I);
(ii) removing any protecting group;
(iii) preparing a salt or a solvate of the compound so formed.

The reaction between a compound of formula (II) and a compound of formula (V) may be effected using conventional methods for the formation of a urea derivative, for example, by treatment of a compound of formula (II) with a suitable activating reagent, such as phosgene,di-tertbutyl tricarbonate,or phenylchloroformate and a suitable base, followed by treatment with a compound of formula (V). The reaction may be carried out in a suitable solvent such as dichloromethane. A suitable base is triethylamine.

According to still a further aspect of the present invention, there is provided an alternative process for the preparation of compounds of formula (I), which process comprises reacting a compound of formula (VI), 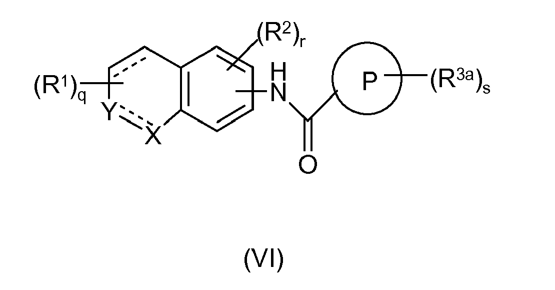 wherein R¹, R², q, r, X and Y are as defined in relation to formula (I), and one R^{3a} represents a group W wherein W is a halogen atom or a trifluoromethylsulfonyloxy group, or W is a group M selected from a boron derivative, for example, a boronic acid function B(OH)₂ or a metal function such as trialkyl stannyl, for example SnBu₃, zinc halide or magnesium halide; and when s is 2 the other R^{3a} is R³; with a compound of formula (VII),

R³-W¹ (VII)

wherein, R³ is as defined in relation to formula (I) and W¹ is a halogen atom or a trifluoromethylsulfonyloxy group when W is a group M or W¹ is a group M when W is a halogen atom or a trifluoromethylsulfonyloxy group; and thereafter, as necessary, carrying out one or more of the following reactions:
(i) converting one compound of formula (I) into another compound of formula (I);
(ii) removing any protecting group;
(iii) preparing a salt or a solvate of the compound so formed.

The reaction of a compound of formula (VI) with a compound of formula (VII) may be effected in the presence of a transition metal catalyst such as tetrakis-triphenylphosphinepalladium (0). When M represents a boronic acid function such as B(OH)₂, the reaction may be carried out under basic conditions, for example using aqueous sodium carbonate in a suitable solvent such as dioxane. When M is trialkylstannyl, the reaction may be carried out in an inert solvent, such as xylene or dioxane optionally in the presence of LiCl. When M is a zinc or magnesium halide, the reaction may be effected in an aprotic solvent such as tetrahydrofuran. The substituent W is preferably a halogen atom such as bromine, or a sulfonyloxy group such as trifluoromethylsulfonyloxy; and W¹ is preferably a group M, such as trialkylstannyl or B(OH)₂.

Compounds of formula (II) may be prepared by the reaction of a compound of formula (VIII), 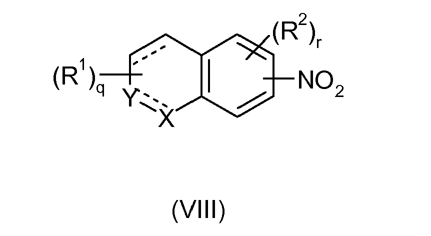 wherein, R¹, R², q and r are as defined in relation to formula (I), with a suitable reducing agent.

The reaction of a compound of formula (VIII) with a reducing agent may be effected by methods well known in the art, such as those described in J March, Advanced Organic Chemistry, 4th edition, J Wiley & Sons, 1992, p. 1216-1218. Suitable reducing agents include (a) iron or zinc metal in hydrochloric acid, or (b) hydrogen in the presence of a suitable catalyst, such as, 5% palladium on charcoal. Reduction using hydrogen may conveniently be performed in a solvent such as methanol or ethanol.

Compounds of formula (VIII) where X is NR⁸ where R⁸ is H and Y is C(R⁹)₂, may be prepared by reaction of a compound of formula (IX), 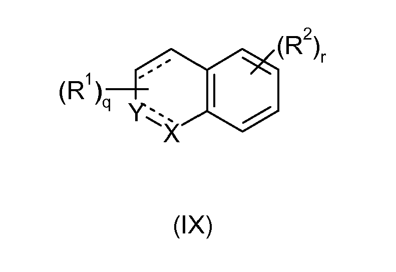 wherein, R¹, R², q and r are as defined in relation to formula (I), X is NR⁸ where R⁸ is H and Y is C(R⁹)₂, with concentrated sulfuric acid and concentrated nitric acid. The reaction of a compound of formula (IX) with concentrated sulfuric acid and concentrated nitric acid may be effected by methods well known in the art, such as those described in J March, Advanced Organic Chemistry, 4th edition, J Wiley & Sons, 1992, p. 522-525.

Compounds of formula (VIII) where X is N and Y is CR⁹ may be prepared by reaction of a compound of formula (VIII) where X is NR⁸ where R⁸ is H and Y is C(R⁹)₂ with (a) a suitable aromatisation reagent, such as a suitable quinone, for example, 2,3-dichloro-5,6-dicyano-1,4-benzoquinone; or (b) a suitable hydrogenation catalyst, for example, 10% Pd on charcoal, in the presence of a suitable solvent such as xylene. The reaction of a compound of formula (VIII) where X is NR⁸ where R⁸ is H and Y is CH₂ with a suitable aromatisation reagent or a suitable hydrogenation catalyst may be effected by methods well known in the art, such as those described in J March, Advanced Organic Chemistry, 4th edition, J Wiley & Sons, 1992, p. 1162-1164.

Compounds of formula (VIII) wherein X is NR⁸ where R⁸ is alkyl, hydroxyalkyl, cycloalkyl, aralkyl, alkoxyalkyl, cycloalkylalkyl, heterocyclylalkyl, - S(O)ₘR⁶, -C(O)CF₃, -C(O)alkyl, -C(O)cycloalkyl, -C(O)aralkyl, -C(O)Ar, - C(O)(CH₂)ₙOR⁶, -C(O)(CH₂)ₙNR⁴R⁵, -C(O)alkoxy, -C(O)NR⁴R⁵, - (CH₂)ₙC(O)alkoxy, -(CH₂)ₙOC(O)R⁶, -(CH₂)ₙOR⁶, -(CH₂)ₙR⁴R⁵, - (CH₂)ₙC(O)NR⁴R⁵, -(CH₂)ₙN(R⁴)C(O)R⁶, -(CH₂)ₙS(O)₂NR⁴R⁵, - (CH₂)ₙN(R⁴)S(O)₂R⁶, -(CH₂)ₙS(O)₂R⁶, -(CH₂)ₙN(R⁴)S(O)₂R⁶,-(CH₂)ₙN(R⁴)C(O)R⁶ or -(CH₂)ₙC(O)alkyl and Y is C(R⁹)₂ where R⁹ is as defined in relation to formula (I) may be prepared by reaction of a compound of formula (VIII) wherein X is NR⁸ where R⁸ is H and Y is C(R⁹)₂ where R⁹ is as defined in relation to formula (I), with
(a) a suitable acylating agent; or
(b) a suitable acylating reagent and thereafter, reacting the product so formed with a suitable reducing agent; or
(c) a suitable alkylating agent.

The reaction between a compound of formula (VIII) with a suitable acylating agent may be effected by methods well known in the art, such as those described in J March, Advanced Organic Chemistry, 4th edition, J Wiley & Sons, 1992, p417. A suitable acylating agent is an acyl chloride. Typically, the acylation is performed in the presence of a suitable base, such as triethylamine, in a suitable solvent, such as, dichloromethane. The reduction of an acylated product so formed may be effected by methods well known in the art such as those descibed in J March, Advanced Organic Chemistry, 4th edition, J Wiley & Sons, 1992, p. 1212. A suitable reducing agent is borane-THF complex. Typically, the reduction is performed in a suitable solvent, such as, tetrahydrofuran.

The reaction between a compound of formula (VIII) with a suitable alkylating agent may be effected by methods well known in the art, such as those described in J March, Advanced Organic Chemistry, 4th edition, J Wiley & Sons, 1992, p411. A suitable alkylating reagent is an alkyl halide. Typically the reaction is performed in the presence of a suitable base, such as, potassium carbonate or cesium carbonate, in a suitable solvent, such as, dimethylformamide.

Compounds of formula (VIII) where Y is NR⁸ where R⁸ is H, and X is C(R⁹)₂ may be prepared by methods described in International Patent Application, Publication Number WO 00/09486.

Compounds of formula (IX) are commercially available. Compounds of formula (III) may be prepared according to a variety of known methods in accordance with the nature of the moiety, P. For example, compounds of formula (III) or their corresponding esters, where P is phenyl or heteroaryl may be prepared in accordance with methods described in J. Hassan et al., Chem. Rev., 2002, 102, 1359. Hydrolysis of the corresponding ester compounds to compounds of formula (III) may be carried out in accordance with methods disclosed in J March, Advanced Organic Chemistry, 4th edition, J Wiley & Sons, 1992, p. 378-383. Compounds of formula (III) where P is heteroaryl or heterocyclyl may be prepared in accordance with, for example, methods disclosed in the following references: H. Vorbruggen, Adv. Het. Chem., 1990, 49, 117 and E. Graf et al, Synthesis, 1999, 7,1216.

Compounds of formula (IV) may be prepared in accordance with methods disclosed in J. Hassan et al., Chem. Rev., 2002, 102, 1359.

Compounds of formula (V) may be prepared by reaction of a compound of formula (X),

(R³)ₛ-L" (X)

wherein R³ is as defined in relation to compound of formula (I), s is 1, 2 or 3 and L" is halo, such as chloro or bromo, with a compound of formula (XI), 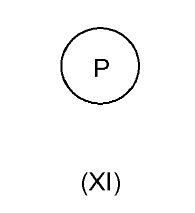 wherein P is heterocyclyl.

Compounds of formula (V) where R³ is heteroaryl may be prepared in accordance with the methods disclosed in H. Vorbruggen et al., Adv. Het. Chem., 1990, 49, 117. Compounds of formula (X) where R³ is heteroaryl and compounds of formula (XI) are commercially available. Compounds of formula (V) where R³ is phenyl are commercially available.

Compounds of formula (VI) may be prepared by analogous methods to those described herein for the preparation of compounds of formula (I).

Compounds of formula (VII) are commercially available.

The above-mentioned conversions of a compound of formula (I) into another compound of formula (I) include any conversion, which may be effected using conventional procedures, but in particular the said conversions include any combination of:
(i) converting one group R¹ into another group R¹;
(ii) converting one group R² into another group R²;
(iii) converting one group R³ into another group R³; and
(iv) converting one group R⁸ into another group R⁸.
The above-mentioned conversions (i), (ii), (iii) and (iv) may be performed using any appropriate method under conditions determined by the particular groups chosen.
Suitable conversions of one group R⁸ into another group R⁸, as in conversion (iv) above, include, (a) converting a group R⁸ which represents -H, into another group R⁸ which represents alkyl, such as methyl. Such a conversion may be performed using an appropriate alkylation procedure, for example, by treating a compound of formula (I) wherein R⁸ is -H with an agent, R⁸-Z, where R⁸ is alkyl and Z is halo, such as bromo, chloro or iodo, or -OSO₂CF₃. Typically, such an interconversion is performed in the presence of a suitable base, such as, potassium carbonate or cesium carbonate. A suitable solvent is dimethylformamide; (b) converting a group R⁸ which represents -H, into another group R⁸ which represents acyl, such as acetyl. Such a conversion may be performed using an appropriate acylation procedure, for example, by treating a compound of formula (I) wherein R⁸ is -H with an agent, R⁸-Z, where R⁸ is acyl and Z is halo, such as chloro. Typically, such an interconversion is performed in the presence of a suitable base, such as, triethylamine. A suitable solvent is dichloromethane.

It will be appreciated by those skilled in the art that it may be necessary to protect certain reactive substituents during some of the above procedures. Standard protection and deprotection techniques, such as those described in Greene T.W. 'Protective groups in organic synthesis', New York, Wiley (1981), can be used. For example, primary amines can be protected as phthalimide, benzyl, benzyloxycarbonyl or trityl derivatives. Carboxylic acid groups can be protected as esters. Aldehyde or ketone groups can be protected as acetals, ketals, thioacetals or thioketals. Deprotection of such groups is achieved using conventional procedures known in the art.

Pharmaceutically acceptable salts may be prepared conventionally by reaction with the appropriate acid or acid derivative.

Compounds of formula (I) and their pharmaceutically acceptable salts and solvates thereof have Vanilloid receptor antagonist (VR1) activity and are believed to be of potential use for the treatment or prophylaxis of certain disorders, or treatment of the pain associated with them, such as: pain, chronic pain, neuropathic pain, postoperative pain, postrheumatoid arthritic pain, osteoarthritic pain, back pain, visceral pain, cancer pain, algesia, neuralgia, dental pain, headache, migraine, neuropathies, carpal tunnel syndrome, diabetic neuropathy, HIV-related neuropathy, post-herpetic neuralgia, fibromyalgia, neuritis, sciatica, nerve injury, ischaemia, neurodegeneration, stroke, post stroke pain, multiple sclerosis, respiratory diseases, asthma, cough, COPD, broncho constriction, inflammatory disorders, oesophagitis, heart burn, Barrett's metaplasia, dysphagia, gastroeosophageal relux disorder (GERD), stomach and duodenal ulcers, functional dyspepsia, irritable bowel syndrome, inflammatory bowel disease, colitis, Crohn's disease, pelvic hypersensitivity, pelvic pain, menstrual pain, renal colic, urinary incontinence, cystitis, burns, itch, psoriasis, pruritis, emesis (hereinafter referred to as the "Disorders of the Invention").

Accordingly, the invention also provides a compound of formula (I) or a pharmaceutically acceptable salt or solvate thereof, for use as an active therapeutic substance, in particular, in the treatment and/or prophylaxis of the Disorders of the Invention.

In particular, the invention provides a compound of formula (I) or a pharmaceutically acceptable salt or solvate thereof for use in the treatment or prophylaxis of pain.

The invention further provides a method for the treatment or prophylaxis of disorders in which antagonism of the Vanilloid (VR1) receptor is beneficial, in particular the Disorders of the Invention, in mammals including humans, which method comprises administering to a mammal in need thereof a therapeutically effective amount of a compound of formula (I) or a pharmaceutically acceptable salt or solvate thereof.

The invention provides for the use of a compound of formula (I) or a pharmaceutically acceptable salt or solvate thereof in the manufacture of a medicament for the treatment or prophylaxis of disorders in which antagonism of the Vanilloid (VR1) receptor is beneficial, particularly the Disorders of the Invention.

In order to use the compounds of the invention in therapy, they will normally be formulated into a pharmaceutical composition in accordance with standard pharmaceutical practice. Thus, the present invention also provides a pharmaceutical composition, which comprises a compound of formula (I) or a pharmaceutically acceptable salt or solvate thereof and a pharmaceutically acceptable carrier or excipient therefor.

A pharmaceutical composition of the invention, which may be prepared by admixture, suitably at ambient temperature and atmospheric pressure, is usually adapted for oral, parenteral, rectal administration or intravesical adminstration to the bladder and, as such, may be in the form of tablets, capsules, oral liquid preparations, powders, granules, lozenges, reconstitutable powders, injectable or infusable solutions, suspensions or suppositories. Orally administrable compositions are generally preferred.

Tablets and capsules for oral administration may be in unit dose form, and may contain conventional excipients, such as binding agents, fillers, tabletting lubricants, disintegrants and acceptable wetting agents. The tablets may be coated according to methods well known in normal pharmaceutical practice.

Oral liquid preparations may be in the form of, for example, aqueous or oily suspension, solutions, emulsions, syrups or elixirs, or may be in the form of a dry product for reconstitution with water or other suitable vehicle before use. Such liquid preparations may contain conventional additives such as suspending agents, emulsifying agents, non-aqueous vehicles (which may include edible oils), preservatives, and, if desired, conventional flavourings or colourants.

For parenteral administration, fluid unit dosage forms are prepared utilising a compound of the invention or pharmaceutically acceptable salt thereof and a sterile vehicle. The compound, depending on the vehicle and concentration used, can be either suspended or dissolved in the vehicle. In preparing solutions, the compound can be dissolved for injection and filter sterilised before filling into a suitable vial or ampoule and sealing. Advantageously, adjuvants such as a local anaesthetic, preservatives and buffering agents are dissolved in the vehicle. To enhance the stability, the composition can be frozen after filling into the vial and the water removed under vacuum. Parenteral suspensions are prepared in substantially the same manner, except that the compound is suspended in the vehicle instead of being dissolved, and sterilization cannot be accomplished by filtration. The compound can be sterilised by exposure to ethylene oxide before suspension in a sterile vehicle. Advantageously, a surfactant or wetting agent is included in the composition to facilitate uniform distribution of the compound.

The composition may contain from 0.1 % to 99% by weight, preferably from 10 to 60% by weight, of the active material, depending on the method of administration.

The dose of the compound used in the treatment of the aforementioned disorders will vary in the usual way with the seriousness of the disorders, the weight of the sufferer, and other similar factors. For systemic administration, dosage levels from 0.01 mg to 100mg per kilogramme of body weight are useful in the treatment of pain. However, as a general guide suitable unit doses may be 0.05 to 1000 mg, more suitably 0.05 to 20, 20 to 250, or 0.1 to 500.0 mg, for example 0.2 to 5 and 0.1 to 250 mg; and such unit doses may be administered more than once a day, for example two or three a day, so that the total daily dosage is in the range of about 0.5 to 1000 mg; and such therapy may extend for a number of weeks or months.

No unacceptable toxicological effects are indicated with compounds of the invention when administered in accordance with the invention.

All publications, including but not limited to patents and patent applications, cited in this specification are herein incorporated by reference as if each individual publication were specifically and individually indicated to be incorporated by reference herein as though fully set forth.

The following Descriptions and Examples illustrate the preparation of the compounds of the invention.

### Abbreviations

AIBN = 2,2'-azobisisobutyronitrile
BINAP = 2,2'-bis(diphenylphosphino)-1,1'-binaphthyl
CDCl₃ = chloroform-d
DCM = Dichloromethane
DME = 1,2-Dimethoxyethane
DMF = DimethylformamideDMSO = dimethylsulfoxide
EtOAc = Ethyl acetate
MeOH = Methanol
MgSO₄ = Magnesium sulfate
Na₂SO₄ = Sodium Sulfate
NCS = N-chlorosuccinimide
Pd₂(dba)₃ -tris(dibenzylideneacetone)dipalladium(0)
SPE = solid phase extraction
THF = Tetrahydrofuran
tic = Thin Layer Chromatography
Xantphos --9,9-dimethyl-4,5-bis(diphenylphosphino)xanthene

### Description 1 (D1)

### 7-Nitro-1,2,3,4-tetrahydroquinoline

To a solution of 1,2,3,4-tetrahydroquinoline (6.5g, 0.049mol) in concentrated sulfuric acid (118ml) cooled to 0°C was added a solution of concentrated nitric acid (4.9ml) in concentrated sulfuric acid (12ml) dropwise over 0.3h so as to maintain the temperature at 0-5°C. On completion of addition, the reaction mixture was poured onto crushed ice then neutralised with solid potassium carbonate. EtOAc (500ml) was added and the mixture was filtered to remove undissolved solids then extracted with further EtOAc (4 x 500ml). The combined extracts were dried over MgSO4 and concentrated *in vacuo* to give the crude product which was purified by flash column chromatography. Elution with 5% EtOAc/60-80°C petroleum ether gave the title compound as an orange solid (5.46g). ¹H NMR (250MHz, CDCl₃) δ(ppm): 7.40 (dd, 1 H), 7.26 (d,1H), 7.02 (d, 1 H), 4.16 (br, 1 H), 3.35 (m, 2H), 2.81 (t, 2H), 1.95 (m, 2H).

### Description 2 (D2)

### 1-Methyl-7-nitro-1,2,3,4-tetrahydroquinoline

To a solution of 7-nitro-1,2,3,4-tetrahydroquinoline (D1) (7.03g, 39.4mmol) in dimethylformamide (50ml) was added potassium carbonate (16.3g, 118mmol) and iodomethane (3.7ml, 59.1 mmol) and the reaction stirred at ambient temperature overnight. The solvent was removed under reduced pressure and the residue was taken up in water (400ml) and extracted into ether (3 x 200ml). The combined ether extracts were washed with brine (100ml), dried over MgSO₄ and concentrated *in vacuo* to give the crude product which was purified by flash column chromatography. Elution with 20-40% EtOAc/40-60°C petroleum ether gave the title compound as an orange solid (5.35g). ¹H NMR (250MHz, CDCl₃) δ (ppm): 7.42 (dd, 1 H), 7.33 (d, 1 H), 7.01 (d, 1 H), 3.31 (m, 2H), 2.96 (s, 3H), 2.80 (t, 2H), 1.99 (m, 2H).

### Description 3 (D3)

### 7-Amino-1-methyl-1,2,3,4-tetrahydroquinoline

A mixture of 1-methyl-7-nitro-1,2,3,4-tetrahydroquinoline (D2) (5.35g, 27.9mmol) and 10% palladium on charcoal (2g, 54% water) in methanol (150ml) was hydrogenated at atmospheric pressure and ambient temperature temperature for 3d. The catalyst was filtered off and washed with further methanol. The combined filtrated and washings were concentrated *in vacuo* to give the crude product, which was purified by flash column chromatography. Elution with 5-10% EtOAc/DCM gave the title compound as a brown oil (2.58g). ¹H NMR (250MHz, CDCl₃) δ (ppm): 6.73 (dd, 1 H), 5.99 (m, 2H), 3.49 ( br, 1 H), 3.18 (m, 2H), 2.84 (s, 3H), 2.66 (t, 2H), 1.93 (m, 2H).

### Description 4 (D4)

### 7-Nitro-1-trifluoroacetyl-1,2,3,4-tetrahydroquinoline

To solution of 7-nitro-1,2,3,4-tetrahydroquinoline (D1) (1 g, 5.6mmol) and triethylamine (1.2ml, 8.6mmol) in DCM (30ml) at 0°C was added trifluoroacetic anhydride (0.8ml, 5.7mmol) and the reaction was then stirred at ambient temperature overnight. The reaction mixture was diluted with DCM (30ml), washed with water (100ml), dried over MgSO₄ and concentrated *in vacuo* to give the title compound as a yellow solid (1.52g). ¹H NMR (250MHz, CDCl₃) δ (ppm): 8.64 (br, 1 H), 8.04 (dd, 1 H), 7.36 (d, 1 H), 3.90 (m, 2H), 2.99 (t, 2H), 1.98 (m, 2H).

### Description 5 (D5)

### 7-Amino-1-trifluoroacetyl-1,2,3,4-tetrahydroquinoline

A mixture of 7-nitro-1-trifluoroacetyl-1,2,3,4-tetrahydroquinoline (D4) (1.51 g, 5.5mmol) and 10% palladium on charcoal (150mg, 54% water) in methanol (80ml) was hydrogenated at atmospheric pressure and ambient temperature for 24h. The catalyst was removed by filtration and was washed with further methanol. The combined filtrated and washings were concentrated *in vacuo* to give the title compound as a pale orange solid (1.29g). ¹H NMR (250MHz, CDCl₃) δ (ppm): 7.05 (br, 1 H), 6.95 (d, 1 H), 6.54 (dd, 1 H), 3.78 (m, 2H), 3.65 (br, 2H), 2.74 (br, 2H), 2.03 (m, 2H).

### Description 6 (D6)

### 1-(2-Methoxyacetyl)-7-nitro-1,2,3,4-tetrahydroquinoline

Using the procedure outlined in Description 4, the title compound was prepared from 7-nitro-1,2,3,4-tetrahydroquinoline (D1) (178mg, 1mmol) and methoxyacetyl chloride (101ul, 1.1mmol) as a yellow gum (212mg). ¹H NMR (250MHz, CDCl₃) δ (ppm): 8.54 (br, 1 H), 7.96 (dd, 1 H), 7.30 (d, 1 H), 4.25 (s, 2H), 3.81 (m, 2H), 3.48 (s, 3H), 2.88 (t, 2H), 2.04 (qn, 2H).

### Description 7 (D7)

### 1-(2-Dimethylaminoacetyl)-7-nitro-1,2,3,4-tetrahydroquinoline

Using the procedure outlined in Description 4 followed by silica SPE chromatography eluting with 20% EtOAc/MeOH the title compound was prepared from 7-nitro-1,2,3,4-tetrahydroquinoline (D1) (178mg, 1mmol) and dimethylaminoacetyl chloride hydrochloride (174mg, 1.1mmol) as a yellow solid (103mg). ¹H NMR (250MHz, CDCl₃) δ (ppm): 8.74 (d, 1 H), 7.92 (dd, 1 H), 7.28 (d, 1 H), 3.87 (m, 2H), 3.25 (s, 2H), 2.87 (t, 2H), 2.35 (s, 6H), 1.95 (m, 2H).

### Description 8 (D8)

### 1-(2-Chloroacetyl)-7-nitro-1,2,3,4-tetrahydroquinoline

Using the procedure outlined in Description 4, the title compound was prepared from 7-nitro-1,2,3,4-tetrahydroquinoline (D1) (4.41 g, 25mmol) and chloroacetyl chloride (2.2ml, 27.6mmol) as a dark brown solid (5.853g). ¹H NMR (250MHz, CDCl₃) δ (ppm): 8.49 (br, 1 H), 8.00 (dd, 1 H), 7.33 (d, 1 H), 4.27 (s, 2H), 3.86 (m, 2H), 2.90 (t, 2H), 2.09 (m, 2H).

### Description 9 (D9)

### 1-(2-Diisopropylaminoacetyl)-7-nitro-1,2,3,4-tetrahydroquinoline

A mixture of 1-(2-chloroacetyl)-7-nitro-1,2,3,4-tetrahydroquinoline (D8) (6.7g, 26mmol) and diisopropylamine (50ml) in THF (50ml) was heated at reflux for 5d then cooled to room temperature. Aqueous work-up yielded a crude oil which was purified by flash column chromatography. Elution with 0-5% methanol/DCM gave the title compound as a dark oil (6.56g). ¹H NMR (250MHz, CDCl₃) δ (ppm): 8.62 (br, 1 H), 7.91 (dd, 1 H), 7.27 (d, 1 H), 3.95 (m, 2H), 3.54 (s, 2H), 3.02 (sp, 2H), 2.87 (t, 2H), 2.02 (m, 2H), 1.02 (d, 12H).

### Description 10 (D10)

### 1-(2-Methoxyethyl)-7-nitro-1,2,3,4-tetrahydroquinoline

To a solution of 1-(2-methoxyacetyl)-7-nitro-1,2,3,4-tetrahydroquinoline (D6) (212mg, 0.85mmol) in dry THF (9ml) at 0°C under an argon atmosphere, was added borane/THF complex (4.5ml, 4.5mmol). The reaction was stirred at 0°C for 0.5h then ambient temperature for 3h. 2M Hydrochloric acid (3ml) was added cautiously followed by water (10ml). The mixture was extracted with EtOAc (2 x 10ml) which was dried over MgSO₄ and concentrated *in vacuo* to give the desired product as an orange solid in quantitative yield. ¹H NMR (250MHz, CDCl₃) δ (ppm): 7.39 (m, 2H), 7.00 (m, 1 H), 3.61 (dd, 2H), 3.52 (dd. 2H), 3.41 (m, 2H), 3.37 (s, 3H), 2.80 (t, 2H), 1.95 (m, 2H).

### Description 11 (D11)

### 1-(2-Dimethylaminoethyl)-7-nitro-1,2,3,4-tetrahydroquinoline

Using the procedure outlined in Description 10, the title compound was prepared from 1-(2-dimethylaminoacetyl)-7-nitro-1,2,3,4-tetrahydroquinoline (D7) (103mg, 0.39mmol) as an orange solid (80mg). ¹H NMR (250MHz, CDCl₃) δ (ppm): 7.43 (dd, 1 H), 7.35 (d, 1 H), 7.04 (d, 1 H), 3.83 (m, 2H), 3.39 (m, 2H), 2.91 (m, 2H), 2.81 (t, 2H), 2.72 (s, 6H), 1.98 (m, 2H).

### Description 12 (D12)

### 1-(2-Diisopropylaminoethyl)-7-nitro-1,2,3,4-tetrahydroquinoline

Using the procedure outlined in Description 10, the title compound was prepared from 1-(2-diisopropylaminoacetyl)-7-nitro-1,2,3,4-tetrahydroquinoline (D9) (6.56g, 21mmol) as an orange oil (4.85g). ¹H NMR (250MHz, CDCl₃) δ (ppm): 7.42 (d, 1 H), 7.33 (dd, 1 H), 6.98 (d, 1 H), 3.41 (m, 2H), 3.31 (m, 2H), 3.05 (sp, 2H), 2.78 (t, 2H), 2.62 (m, 2H), 1.92 (m, 2H), 1.04 (d, 12H).

### Description 13 (D13)

### 7-Amino-1-(2-methoxyethyl)-1,2,3,4-tetrahydroquinoline

Using the procedure outlined in Description 5, the title compound was prepared from 1-(2-methoxyethyl)-7-nitro-1,2,3,4-tetrahydroquinoline (D10) (215mg, 0.91mmol) as a colourless gum, (152mg). ¹H NMR (250MHz, CDCl₃) δ (ppm): 6.71 (m, 1 H), 5.96 (m, 2H), 3.55 (t, 2H), 3.41 (t, 2H), 3.35 (s, 3H), 3.31 (m, 2H), 2.95 (br), 2.64 (t, 2H), 1.89 (m, 2H).

### Description 14 (D14)

### 7-Amino-1-(2-dimethylaminoethyl)-1,2,3,4-tetrahydroquinoline

Using the procedure outlined in Description 5, the title compound was prepared from 1-(2-dimethylaminoethyl)-7-nitro-1,2,3,4-tetrahydroquinoline (D11) (75mg, 0.3mmol) as a crude yellow solid, (79mg). ¹H NMR (250MHz, CDCl₃) δ (ppm): 6.75 (d, 1 H), 6.35 (d, 1 H), 6.05 (dd, 1 H), 3.81 (m, 2H), 3.27 (m, 2H), 3.16 (t, 2H), 2.85 (s, 6H), 2.64 (t, 2H), 1.90 (m, 2H).

### Description 15 (D15)

### 7-Amino-1-(2-diisopropylaminoethyl)-1,2,3,4-tetrahydroquinoline

Using the procedure outlined in Description 5, the title compound was prepared from 1-(2-diisopropylaminoethyl)-7-nitro-1,2,3,4-tetrahydroquinoline (D12) (4.70g, 17mmol) as a brown oil (2.85g). ¹H NMR (400MHz, CDCl₃) δ (ppm): 6.71 (d, 1 H), 5.95 (m, 2H), 3.43 (br, 2H), 3.29 (m, 2H), 3.20 (m, 2H), 3.02 (sp, 2H), 2.62 (m, 4H), 1.89 (m, 2H), 1.04 (d, 12H).

### Description 16 (D16)

### 1-(2-Morpholin-4-ylethyl)-7-nitro-1,2,3,4-tetrahydroquinoline

To a solution of 7-nitro-1,2,3,4-tetrahydroquinoline (D1) (246mg, 1.38mmol) in DMF (2.7ml) was added potassium carbonate (574mg, 4.15mmol) followed by a solution of 4-(2-iodoethyl)morpholine (500mg, 2.07mmol) in DMF (2ml) and the reaction heated to 70°C. After cooling to ambient temperature the reaction mixture was diluted with water and extracted with EtOAc which was washed with water, dried over MgSO₄ and concentrated *in vacuo* to give the crude product which was purified by silica SPE chromatography. Elution with 80% EtOAc/petroleum ether gave the title compound as an orange gum (29mg). ¹H NMR (250MHz, CDCl₃) δ (ppm): 7.39 (m, 2H), 7.00 (d, 1H), 3.73 (m, 4H), 3.47 (m, 2H), 3.38 (m, 2H), 2.79 (t, 2H), 2.49-2.59 (m, 6H), 1.96 (m, 2H).

### Description 17 (D17)

### 7-Amino-1-(2-morpholin-4-ylethyl)-1,2,3,4-tetrahydroquinoline

Using the procedure outlined in Description 5, the title compound was prepared from 1-(2-morpholin-4-ylethyl)-7-nitro-1,2,3,4-tetrahydroquinoline (D16) (29mg, 0.1 mmol) as a pink gum, which was used directly in the next step.

### Description 18 (D18)

### Ethyl 2-methyl-6-phenylnicotinate

The title compound was prepared according to E. Graf & R. Troschutz, Synthesis, 1999, 7, 1216.

### Description 19 (D19)

### Ethyl 6-(4-fluorophenyl)-2-methylnicotinate

The title compound was prepared from dimethylamino-(4-fluorophenyl)propan-1-one and ethyl 3-aminocrotonate using the general procedure outlined in D18. MS (ES): MH⁺ 260.

### Description 20 (D20)

### Ethyl 6-(3-fluorophenyl)-2-methylnicotinate

The title compound was prepared from dimethylamino-(3-fluorophenyl)propan-1-one and ethyl 3-aminocrotonate using the general procedure outlined in D18. ¹H NMR (250MHz, CDCl₃) δ (ppm): 8.27 (d, 1H), 7.83 (m, 2H), 7.61 (d, 1 H), 7.44 (m, 1 H), 7.13 (m, 1 H), 4.41 (q, 2H), 2.91 (s, 3H), 1.42 (t, 3H).

### Description 21 (D21)

### Ethyl 6-(2,3-difluorophenyl)-2-methylnicotinate

The title compound was prepared from dimethylamino-(2,3-difluorophenyl)-propan-1-one and ethyl 3-aminocrotonate using the general procedure outlined in D18. ¹H NMR (250MHz, CDCl₃) δ (ppm): 8.28 (d, 1H), 7.83 (m, 1 H), 7.70 (dd, 1 H), 7.22 (m, 2H), 4.41 (q, 2H), 2.91 (s, 3H), 1.42 (t, 3H).

### Description 22 (D22)

### (R)-2-Methyl-4-(3-trifluoromethyl-2-pyridyl)piperazine

The title compound was prepared according to R. Bakthavalatcham, International Patent Application, Publication Number, WO 02/08221).

### Description 23 (D23)

### 2-Methyl-6-phenylnicotinic acid

Ethyl 2-methyl-6-phenylnicotinate (D18) (284mg, 1.2mmol) was treated with aq. 2M NaOH in ethanol at reflux giving the title compound as an off white solid (108mg). MS (AP): MH⁺ 214, M-H⁺ 212.

### Description 24 (D24)

### 6-(4-Fluorophenyl)-2-methylnicotinic acid

Using the procedure outlined in Description 23, the title compound was prepared from ethyl 6-(4-fluorophenyl)-2-methylnicotinate (D19) (500mg, 1.9mmol) as an off white solid (250mg). ¹H NMR (400MHz, DMSO) δ (ppm): 8.25 (d, 1 H), 8.21 (dd,2H), 7.92 (d, 1 H), 7.35(t, 2H), 2.80(s, 3H).

### Description 25 (D25)

### 6-(3-Fluorophenyl)-2-methylnicotinic acid

Using the procedure outlined in Description 23, the title compound was prepared from ethyl 6-(3-fluorophenyl)-2-methylnicotinate (D20) (500mg, 1.9mmol) as an off white solid (254mg). ¹H NMR (250MHz, MeOH-d₄) δ (ppm): 8.13 (d, 1 H), 7.80 (m, 2H), 7.69 (d, 1 H), 7.47 (m, 1 H), 7.16 (m, 1 H), 2.81 (s, 3H).

### Description 26 (D26)

### 6-(2,3-Difluorophenyl)-2-methylnicotinic acid

Using the procedure outlined in Description 23, the title compound was prepared from ethyl 6-(2,3-difluorophenyl)-2-methylnicotinate (D21) (500mg, 1.8mmol) as an off white solid (344mg). ¹H NMR (250MHz, CDCl₃) δ (ppm): 8.42(d, 1 H), 7.87 (m, 1 H), 7.76 (m, 1 H), 7.24 (m, 2H), 2.97 (s, 3H).

### Description 27 (D27)

### 4,4-Dimethyl-7-nitro-1-trifluoroacetyl-1,2,3,4-tetrahydroquinoline

To a suspension of 4,4-dimethyl-1,2,3,4-tetrahydroquinoline (W.W. Hoffman, A.R. Kraska, European Patent Application No. EP 0 130 795) (470mg, 2.92mmol) in concentrated sulfuric acid (5ml) at 0°C was added dropwise a mixture of concentrated nitric acid (0.3ml) in concentrated sulfuric acid (2.8ml) such that the temperature of the mixture remained below 5°C. The reaction was allowed to warm to ambient temperature then poured onto ice, basified with 12M NaOH solution and extracted with EtOAc. The extracts were dried and concentrated *in vacuo* to give the crude 4,4-dimethyl-7-nitro-1,2,3,4-tetrahydroquinoline (514mg) which was then dissolved in DCM (12ml) and triethylamine (533ul, 3.82mmol) and trifluoroacetic anhydride (357ul, 2.51 mmol) were added. The mixture was stirred at ambient temperature for 18h then diluted with further DCM, washed with water, dried over MgSO₄ and concentrated *in vacuo* to give the crude product. Purification by column chromatography eluting with 0-30% EtOAc/40-60°C petroleum ether gave the title compound as an orange solid (341mg). ¹H NMR (250MHz, CDCl₃) δ (ppm): 8.62 (br, 1 H), 8.06 (dd, 1 H), 7.55 (d, 1 H), 3.91 (m, 2H), 1.96 (m, 2H), 1.39 (s, 6H).

### Description 28 (D28)

### 7-Amino-4,4-dimethyl-1-trifluoroacetyl-1,2,3,4-tetrahydroquinoline

Using the procedure outlined in Description 5, the title compound was prepared from 4,4-dimethyl-7-nitro-1-trifluoroacetyl-1,2,3,4-tetrahydroquinoline (D27) (282mg, 0.93mmol) as a green oil (237mg). ¹H NMR (400MHz, DMSO) δ (ppm): 7.09 (d, 1 H), 6.70-6.80 (br, 1 H), 6.48 (dd, 1 H), 5.08 (br, 2H), 3.70-3.80 (m, 2H), 1.70-1.80 (m, 2H), 1.20 (s, 6H). MS (ES): MH⁺ 273.

### Description 29 (D29)

### 4-(6-Methyl-2-pyridyl)benzoic acid

To a stirred, degassed mixture of 2-bromo-6-methylpyridine (3g, 17mmol), sodium carbonate (10.8g, 100mmol) and 4-carboxybenzeneboronic acid (2.3g, 14mmol) in DME (150ml) and water (150ml) under an argon atmosphere was added tetrakis(triphenylphosphine) palladium (0) (350mg) and the mixture heated to reflux for 18h. On cooling, ~50% of the solvent was removed *in vacuo* and the residual aqueous solution was washed with EtOAc, then acidified to pH1 with concentrated HCl and washed with further EtOAc. The aqueous was then adjusted to pH 5 by addition of potassium carbonate causing formation of a white precipitate which was collected by filtration, washed with water and dried to give the title compound as a white solid (2.3g). MS (ES): MH⁺ 212.

### Description 30 (D30)

### 7-Amino-3,4-dihydro-2H-1,4-ethanoquinoline

Using the procedure outlined in Description 5, the title compound was prepared from 3,4-dihydro-2*H*-1,4-ethano-7-nitroquinoline (R.P. Duke et al, Tetrahedron Lett., 1970, 21, 1809) (239mg, 0.113mmol) as a white solid (199mg). ¹H NMR (400MHz, CDCl₃) δ (ppm): 6.96 (d, 1 H), 6.59 (d, 1 H), 6.55 (dd, 1 H), 3.28 (br, 2H), 3.18 (ddd, 2H), 3.02 (m, 1 H), 2.70 (m, 2H), 1.81 (m, 2H), 1.52 (m, 2H).

### Description 31 (D31)

### N-Methyl-4'-carboxamido-1,1'-biphenyl-4-carboxylic acid

Using the procedure outlined in Description 29, the title compound was prepared from N-methyl-4-bromobenzamide (0.76g, 3.6mmol) and 4-carboxybenzeneboronic acid (0.56g, 3.4mmol) as a white solid (0.63g). MS (API): MH⁺ 254.

### Description 32 (D32)

### N,N-Dimethyl-4'-carboxamido-1,1'-biphenyl-4-carboxylic acid

Using the procedure outlined in Description 29, the title compound was prepared from N,N-dimethyl-4-bromobenzamide (2.0g, 8.7mmol) and 4-carboxybenzeneboronic acid (1.38g, 8.3mmol) as a white solid (1.46g). MS (API): MH⁺ 268.

### Description 33 (D33)

### N-Methyl-3'-sulfonamido-1,1'-biphenyl-4-carboxylic acid

Using the procedure outlined in Description 29, the title compound was prepared from N-methyl-3-bromobenzenesulfonamide (1 g, 4mmol) and 4-carboxybenzeneboronic acid (0.56g, 3.3mmol) as a cream solid (0.93g). MS (API): MH⁺ 290.

### Description 34 (D34)

### 2-(4-Pyridyl)furan-4-carboxylic acid

Using the procedure outlined in Description 29, the title compound was prepared from 2-bromo-furan-4-carboxylic acid (S.W. En, M.C. Yuen, H.N.C. Wong, Tetrahedron, 1996, 52(37), 12137) and 4-pyridylboronic acid.
The following acids may be prepared according to literature precedent:

### Description 35 (D35)

### 4'-Acetylamino-2'-methyl-1,1'-biphenyl-4-carboxylic acid

L.M. Gaster, P. Ham, D.F. King and P.A. Wyman, International Patent Application, Publication Number WO 97/34901.

### Description 36 (D36)

### 2'-Methyl-1,1'-biphenyl-4-carboxylic acid

S.I. Klein et al, J. Med. Chem., 1998, 41, 437.

### Description 37 (D37)

### 3'-Acetyl-1,1'-biphenyl-4-carboxylic acid

G. Stemp and A. Johns, International Patent Application, Publication Number WO 97/43262.

### Description 38 (D38)

### 3'-Carboxamido-1,1'-biphenyl-4-carboxylic acid

G. Stemp and A. Johns, International Patent Application, Publication Number WO 97/43262.

### Description 39 (D39)

### 3-Methyl-1,1'-biphenyl-4-carboxylic acid

L.M. Gaster, International Patent Application, Publication Number WO 96/06079.

### Description 40 (D40)

### 2-Chloro-1,1'-biphenyl-4-carboxylic acid

H. Ogawa *et al,* International Patent Application, Publication Number WO 9534540.

### Description 41 (D41)

### 3-(4-Carboxyphenyl)thiophene

G. Stemp and A. Johns, International Patent Application, Publication Number WO 97/43262.

### Description 42 (D42)

### 2-(4-Carboxyphenyl)thiophene

C.A. Axton et al, J. Chem. Soc., Perkin Trans. I, 1992, 2203.

### Description 43 (D43)

### 4-(4-Carboxyphenyl)-1-methylpyrazole

G. Stemp and A. Johns, International Patent Application, Publication Number WO 97/43262.

### Description 44 (D44)

### 2-(4-Carboxyphenyl)pyrazine

L.M. Gaster, H.K. Rami and P.A. Wyman, International Patent Application, Publication Number WO 98/50358.

### Description 45 (D45)

### 1-(3-Carboxyphenyl)pyrazole

M.S. Hadley, C.N. Johnson, G.J. Macdonald, G. Stemp and A.K.K. Vong, International Patent Application, Publication Number WO 00/21951.

### Description 46 (D46)

### 5-Phenylthiophene-2-carboxylic acid

J.K. Myers *et al,* International Patent Application, Publication Number WO 02/17358.

### Description 47 (D47)

### 3-(3-Pyridyl)benzoic acid

U. Hacksell etal, J. Med. Chem., 1981, 24(12), 1475.

### Description 48 (D48)

### 6-Phenylnicotinic acid

L.M. Gaster, H.K. Rami and P.A. Wyman, International Patent Application, Publication Number WO 98/50358.

### Description 49 (D49)

### 6-(4-Fluorophenyl)nicotinic acid

S.A. Baumeister *et al,* International Patent Application, Publication Number WO 02/24636.

### Description 50 (D50)

### 4-(1-oxo-indan-5-yl)-benzoic acid

G. Stemp and A. Johns, International Patent Application, Publication Number WO 97/43262.

### Description 51 (D51)

### 4'-Carboxamido-4-biphenylcarboxylic acid

The title compound may be prepared from 4-carboxybenzeneboronic acid and 4-bromobenzamide using the procedure outlined in International Patent Application, Publication number WO 97/4326, for the synthesis of 3'-carboxamido-1,1'-biphenyl-4-carboxylic acid.

### Description 52 (D52)

### 4'-Acetyl-4-biphenylcarboxylic acid

The title compound may be prepared from 4-carboxybenzeneboronic acid and 4-bromoacetophenone using the procedure outlined in International Patent Application, Publication number WO 97/4326, for the synthesis of 3'-carboxamido-1,1'-biphenyl-4-carboxylic acid.

### Description 53 (D53)

### 3-Methyl-4-(3-pyridyl)benzoic acid

The title compound may be prepared from 4-carboxy-2-methylbenzeneboronic acid (International Patent Application, Publication number WO 97/34901) and 3-bromopyridine using the general procedure outlined in International Patent Application, Publication number WO 00/06085, for the synthesis of 4-(3-pyridyl)benzoic acid.

### Description 54 (D54)

### 7-Nitroquinoline

To a solution of 7-nitro-1,2,3,4-tetrahydroquinoline (D1) (2.20g, 12.3mmol) in toluene (300ml) was added 2,3-dichloro-5,6-dicyanobenzoquinone (5.88g, 25.9mmol) and the reaction was heated to 90°C for 1.5h. After cooling to room temperature, the suspension was filtered and the filtrate concentrated *in vacuo* to give the crude product which was purified by flash column chromatography. Elution with 30% EtOAc in 40-60°C petroleum ether gave the title compound as a cream solid (1.74g). ¹H NMR (250MHz, CDCl₃) δ (ppm): 9.08 (dd, 1H), 8.98 (d, 1 H), 8.30 (dd, 2H), 7.99 (d, 1 H), 7.62 (dd, 1 H).

### Description 55 (D55)

### 7-Aminoquinoline

A mixture of 7-nitroquinoline (D54) (0.65g, 3.71 mmol) and 10% palladium on charcoal (65mg, 54% water) in methanol (20ml) was hydrogenated at 1 atm. and ambient temperature for until complete by tlc. The catalyst was filtered off and washed with further methanol. The combined filtrated and washings were concentrated *in vacuo* to give the title compound as a brown solid (0.53g). ¹H NMR (400MHz, CDCl₃) δ (ppm): 8.75 (dd, 1 H), 7.98 (dd, 1 H), 7.62 (d, 1 H), 7.21 (d, 1 H), 7.14 (dd, 1 H), 6.99 (dd, 1 H), 4.07 (br, 2H).

### Description 56 (D56)

### 4-(2,6-Dimethyl-3-pyridyl)-benzoic acid

The title compound was prepared according to L.M. Gaster, H.K. Rami and P.A. Wyman, International Patent Application, Publication Number WO 98/50358.

### Description 57 (D57)

### 3-Methyl-4-(4-pyridyl)-benzoic acid

The title compound was prepared according to L.M. Gaster and P.A. Wyman, International Patent Application, Publication Number WO 98/50346.

### Description 58 (D58)

### 3-Methyl-1,1'-biphenyl-4-carboxylic acid

The title compound may be prepared from 4-bromo-2-methylbenzoic acid and benzeneboronic acid using the procedure outlined in International Patent Application, Publication number WO 96/06079, for the synthesis of 2-methyl-1,1'-biphenyl-4-carboxylic acid.

### Description (D59) (D59)

### 3-Methoxy-1,1'-biphenyl-4-carboxylic acid

The title compound may be prepared from 4-bromo-2-methoxybenzoic acid and benzeneboronic acid using the procedure outlined in International Patent Application, Publication number WO 96/06079, for the synthesis of 2-methyl-1,1'-biphenyl-4-carboxylic acid.

### Description 60 (D60)

### 2-Methyl-1,1'-biphenyl-4-carboxylic acid

The title compound was prepared according to L.M. Gaster, International Patent Application, Publication Number WO 96/06079.

### Description 61 (D61)

### 4-(4-Carboxyphenyl)-piperazine-1-carboxylic acid tert-butyl ester

The title compound was prepared according to M.E. Duggan et al, US Patent Application number 5,854,245.

### Description 62 (D62)

### 3,5-Dimethyl-4-(4-methyl-benzo[1,3]dioxol-5-yl)-benzoic acid

The title compound may be prepared using the procedure outlined in US Patent Application, Publication number 6,323,227, for the synthesis of 4-(benzo[1,3]dioxol-5-yl)benzoic acid.

### Description 63 (D63)

### 2-Methyl-1,2,3,4-tetrahydroquinoline

A mixture of 2-methylquinoline (584mg, 4.1 mmol), indium powder (4.21 g, 36.7mmol), saturated aqueous ammonium chloride solution (6.3ml) and ethanol (21 ml) were heated at reflux for 3 days. On cooling to room temperature, water was added and the mixture was filtered through Keiselguhr. The filtrate was adjusted to pH 9 with 2M sodium hydroxide solution and extracted with DCM (x2). The extracts were dried over MgSO₄ and concentrated *in vacuo* to give the crude product which was purified by flash column chromatography. Elution with 10% EtOAc in 60-80°C petroleum ether gave the title compound as a pale yellow oil (383mg). ¹H NMR (400MHz, CDCl₃) δ (ppm): 6.96 (m, 2H), 6.60 (t, 1H), 6.47 (d, 1 H), 3.68 (br, 1 H), 3.40 (m, 1 H), 2.84 (ddd, 1 H), 2.72 (ddd, 1 H), 1.92 (m, 1 H), 1.60 (m, 1 H), 1.21 (d, 3H).

### Description 64 (D64)

### 2-Methyl-7-nitro-1,2,3,4-tetrahydroquinoline

To a solution of 2-methyl-1,2,3,4-tetrahydroquinoline (D63) (383mg, 2.6mmol) in concentrated sulfuric acid (7.2ml) at 0-5°C was added concentrated nitric acid (0.26ml) dropwise so as to maintain the temperature at 0-5°C. On completion of addition, the mixture was warmed to room temperature, stirred for 45mins. then poured onto crushed ice and neutralised with 2M sodium hydroxide solution. The mixture was extracted with DCM which was washed with brine, dried over MgSO₄ and concentrated *in vacuo* to give the crude product which was purified by flash column chromatography. Elution with 10% EtOAc in 60-80°C petroleum ether gave the title compound as an orange solid (297mg). ¹H NMR (400MHz, CDCl₃) δ (ppm): 7.40 (dd, 1 H), 7.27 (d, 1 H), 7.03 (d, 1 H), 4.01 (br, 1 H), 3.46 (m, 1 H), 2.87 (m, 2H), 1.92 (m, 1 H), 1.59 (m, 1 H), 1.24 (d, 3H).

### Description 65 (D65)

### 7-Amino-2-methyl-1,2,3,4-tetrahydroquinoline

Using the procedure outlined in Description 55, the title compound was prepared from 2-methyl-7-nitro-1,2,3,4-tetrahydroquinoline (D64) (100mg, 0.52mmol) as a crude oil, (86mg) which was used directly in the next step without further purification. ¹H NMR (400MHz, CDCl₃) δ (ppm): 6.74 (d, 1 H), 6.01 (dd, 1 H), 5.84 (d, 1 H), 3.57 (br, 1 H), 3.36 (m, 1 H), 2.72 (ddd, 1 H), 2.62 (ddd, 1 H), 1.87 (m, 1 H), 1.56 (m, 1 H), 1.24 (d, 3H).

### Description 66 (D66)

### 7-Amino-2-methylquinoline

A mixture of 7-amino-2-methyl-1,2,3,4-tetrahydroquinoline (D65) (86mg) and wet 10% palladium/charcoal (25mg) in xylene (20ml) was heated at reflux for 3.5h. After cooling to room temperature the catalyst was removed via filtration and washed with further xylene. Evaporation of the combined filtrate and washings gave the title compound as a crude off-white solid (87mg) which was used in the next step without further purification. ¹H NMR (400MHz, CDCl₃) δ (ppm): 7.87 (d, 1 H), 7.56 (d, 1 H), 7.14 (d, 1 H), 7.03 (d, 1 H), 6.91 (dd, 1 H), 4.02 (br, 2H), 2.67 (s, 3H).

### Description 67 (D67)

### 4-(2-Pyridyl)-benzoic acid

The title compound was prepared according to N.J. Anthony *et al,* International Patent Application, Publication Number WO 97/36896.

### Description 68 (D68)

### 3'-Dimethylsulfamoyl-1,1'-biphenyl-4-carboxylic acid

The title compound may be prepared from 4-carboxybenzeneboronic acid and 3-bromo-1-(dimethylsulfamoyl)benzene using the procedure outlined in International Patent Application, Publication number WO 97/4326, for the synthesis of 3'-carboxamido-1,1'-biphenyl-4-carboxylic acid.

### Description 69 (D69)

### 4-Bromo-3-methoxy-N-quinolin-7-yl-benzamide

Using the procedure outlined in Example 56, the title compound was prepared from 7-aminoquinoline (D55) (36mg, 0.25mmol) and 4-bromo-3-methoxybenzoic acid (69mg, 0.3mmol) as a white solid (84mg). MS(ES): MH⁺ 357/359, M-H⁺ 355/357.

### Description 70 (D70)

### 6-Chloro-N-quinolin-7-yl-nicotinamide

Using the procedure outlined in Example 45, the title compound was prepared from 7-aminoquinoline (D55) (213mg, 1.48mmol) and 6-chloronicotinic acid (279mg, 1.77mmol) as a cream solid (405mg). ¹H NMR (250MHz, CDCl₃) δ (ppm): 8.94 (m, 2H), 8.24 (m, 2H), 8.16 (d, 1 H), 8.14 (br.s, 1 H), 8.01 (dd, 1 H), 7.87 (d, 1 H), 7.51 (d, 1 H), 7.39 (dd, 1 H).

### Description 71 (D71)

### 9H-Fluorene-2-carboxylic acid

The title compound was prepared according to A. Newman et al, Journal of Medicinal Chemistry, 2001, 44, 3175.

### Description 72 (D72F)

### 4-(2-Methyl-4-pyridyl)-benzoic acid

The title compound was prepared according to L.M. Gaster, H.K. Rami and P.A. Wyman, International Patent Application, Publication Number WO 98/50358.

### Description 73G (D73G)

### 6-(3-Fluorophenyl)nicotinic acid

The title compound may be prepared from 3-fluorophenylboronic acid using the procedure outlined in International Patent Application, Publication Number WO 02/24636, for the synthesis of 6-(4-fluorophenyl)nicotinic acid.

### Description 74 (D74)

### 6-(2-Fluorophenyl)nicotinic acid

The title compound may be prepared from 2-fluorophenylboronic acid using the procedure outlined in International Patent Application, Publication Number WO 02/24636, for the synthesis of 6-(4-fluorophenyl)nicotinic acid.

### Description 75 (D75)

### Methyl 4'-fluoro-2-hydroxy-1,1'-biphenyl-4-carboxylate

Methyl 4-bromo-3-hydroxybenzoate (2.1 g, 9.0mmol), 4-fluorophenylboronic acid (2.52g, 18mmol), tetrakis(triphenylphosphine)palladium (0.52g, 0.45mmol), and 2M aqueous sodium carbonate solution (11ml) in ethanol/toluene (1:5, 30ml) were heated at reflux overnight. After cooling the solvent was removed *in vacuo* and the residue dissolved in EtOAc then washed with sat. aq. sodium bicarbonate and dried over MgSO₄. The crude product was purified by column chromatography eluting with 1%MeOH/DCM giving the title compound as a solid (1.9g). MS(ES): MH⁺ 247, M-H⁺ 245.

### Description 76 (D76)

### Methyl 4'-fluoro-2-(2-methoxyethoxy)-1,1'-biphenyl-4-carboxylate

To a suspension of methyl 4'-fluoro-2-hydroxy-1,1'-biphenyl-4-carboxylate (D75) (400mg, 1.6mmol) in DMF (10ml) was added cesium carbonate (1.27g, 3.9mmol) and 2-methoxyethylbromide (400mg, 1.6mmol) and the reaction was heated at 80°C overnight. The solvent was evaporated and the residue was dissolved in EtOAc, washed with water and brine, then dried over MgSO₄ to give the title compound (494mg) which was used without further purification in the next step. MS(ES): (M-MeOH)H⁺ 273.

### Description 77 (D77)

### 4'-Fluoro-2-(2-methoxyethoxy)-1,1'-biphenyl-4-carboxylic acid

Methyl 4'-fluoro-2-(2-methoxyethoxy)-1,1'-biphenyl-4-carboxylate (D76) (494mg) was treated with aq. 2M sodium hydroxide solution (2ml) in ethanol (3ml) at 90°C overnight. After cooling, the ethanol was evaporated off and the residue dissolved in EtOAc and extracted with sat. aq. sodium bicarbonate solution. This was acidified to pH3 with 2M HCl and extracted with EtOAc which was dried over MgSO₄ and concentrated *in vacuo* to give the title compound as a solid (55mg). MS(ES): M-H⁺ 289.

### Description 78 (D78)

### Methyl 2-(2-dimethylaminoethoxy)- 4'-fluoro-1,1'-biphenyl-4-carboxylate

Using the procedure outlined in Description 76, the title compound was prepared from 4'-fluoro-2-hydroxy-1,1'-biphenyl-4-carboxylate (D75) (150mg, 0.61 mmol)) and 2-(dimethylamino)ethylchloride hydrochloride (114mg, 0.79mmol) as a crude gum (200mg) which was used without further purification in the next step. MS(ES): MH⁺ 318.

### Description 79 (D79)

### 2-(2-Dimethylaminoethoxy)- 4'-fluoro-1,1'-biphenyl-4-carboxylic acid

Using the procedure outlined in Description 77, the title compound was prepared from methyl 2-(2-dimethylaminoethoxy)-4'-fluoro-1,1'-biphenyl-4-carboxylate (D78) (200mg, 0.61 mmol)) and 2-(dimethylamino)ethylchloride hydrochloride (114mg, 0.79mmol) as a solid (122mg). MS(ES): MH⁺ 304, M-H⁺ 302.

### Description 80 (D80)

### 5-lodo-7-nitro-1-trifluoroacetyl-1,2,3,4-tetrahydroquinoline

A solution of 7-nitro-1-trifluoroacteyl-1,2,3,4-tetrahydroquinoline (D4) (2g, 7.3mmol) in conc. sulfuric acid (10ml) was cooled to 0°C and treated with iodine (1.11 g, 4.4mmol) and potassium iodate (0.625g, 2.9mmol). The reaction was stirred at 0°C for 3h then room temperature for 2h. The reaction mixture was slowly poured into water (150ml) at 0°C and extracted with DCM. This was washed with aq. sodium metabisulfite and water then dried over MgSO₄ and concentrated *in vacuo* to give the crude product. Purification by column chromatography gave the title compound (420mg). ¹H NMR (250MHz, CDCl₃) δ (ppm): 8.59 (m, 2H), 3.86 (m, 2H), 2.90 (t, 2H), 2.17 (m, 2H).

### Description 81 (D81)

### 5-Chloro-7-nitro-1,2,3,4-tetrahydroquinoline

A solution of 5-iodo-7-nitro-1-trifluoroacetyl-1,2,3,4-tetrahydroquinoline (D53P) (1.69g, 6.73mmol) in DMF (25ml) was treated with copper (I) chloride (1.66g, 16.8mmol) at 130°C for 7h. On cooling the solution was filtered and the filtrate concentrated *in vacuo.* The residue was dissolved in EtOAc and washed with 5M HCl, then dried over MgSO₄ and concentrated *in vacuo.* The crude product was purified by column chromatography to give a (1:1) mixture of the title compound and 5-chloro-7-nitro-1-trifluoroacetyl-1,2,3,4-tetrahydroquinoline (591 mg). This mixture was treated with potassium carbonate in methanol to yield the title compound (450mg) as a brown solid. ¹H NMR (400MHz, CDCl₃) δ (ppm): 7.49 (d, 1 H), 7.19 (d, 1 H), 4.31 (br.s, 1 H), 3.33 (m, 2H), 2.82 (t, 2H), 1.98 (m, 2H).

### Description 82 (D82)

### 5-Chloro-7-nitroquinoline

Using the procedure outlined in Description 54, the title compound was prepared from 5-chloro-7-nitro-1,2,3,4-tetrahydroquinoline (D81) (60mg, 0.28mmol) as an off white solid (54mg). ¹H NMR (400MHz, CDCl₃) δ (ppm): 9.14 (d, 1 H), 8.96 (d, 1 H), 8.67 (d, 1 H), 8.45 (d, 1 H), 7.72 (dd, 1 H).

### Description 83 (D83)

### 7-Amino-5-chloroquinoline

5-Chloro-7-nitroquinoline (D82) (50mg, 0.24mmol) was treated with tin dichloride dihydrate (216mg, 0.96mmol) and conc. hydrochloric acid (2ml) in ethanol (5ml) at 70°C for 4h. After cooling to room temperature and the ethanol removed *in vacuo* then the residue was dissolved in water and neutralised with potassium carbonate. This was then extracted with EtOAc which was dried over MgSO₄ and concentrated *in vacuo* to give the title compound as a brown solid (18mg). ¹H NMR (400MHz, CDCl₃) δ (ppm): 8.78 (d, 1 H), 8.39 (d, 1 H), 7.25 (dd, 1H), 7.18(d, 1H), 7.11 (d, 1 H).

### Description 84 (D84)

### 4-(3-Chloro-2-pyridyl)-benzoic acid

The title compound may be prepared from 4-carboxybenzeneboronic acid and 2,3-dichloropyridine using the procedure outlined in Description 29 (D29), for the synthesis of 4-(6-methyl-2-pyridyl)-benzoic acid.

### Description 85 (D85)

### Ethyl 6-(4-fluorophenyl)-2-(bromomethyl)nicotinate.

Ethyl 6-(4-fluorophenyl)-2-methylnicotinate (82mg, 0.32mmol), N-bromosuccinimide (67mg, 0.38mmol) and AIBN (5mg, 0.032mmol) in carbon tetrachloride (7ml) were irradiated (150W lamp) for 6h then cooled to room temperature. The solid was filtered off and the filtrate concentated and purified by SPE chromatography to give the title compound (38mg) as a 5:1 mixture with the dibrominated product. ¹H NMR (250MHz, CDCl₃) δ (ppm): 8.32 (d, 1 H), 8.11 (dd, 2H), 7.68 (d, 1 H), 7.15 (t, 2H), 5.09 (s, 2H), 4.45 (q, 2H), 1.45 (t, 3H).

### Description 86 (D86)

### 6-(4-Fluorophenyl)-2-(methoxymethyl)nicotinic acid.

Ethyl 6-(4-fluorophenyl)-2-(bromomethyl)nicotinate (D85) (38mg, 0.11mmol) was treated with sodium methoxide (18mg, 0.34mmol) in methanol (1ml) at room temperature for 1 h. 2M Sodium hydroxide solution was then added and the solution stirred for 1 h. The mixture was diluted with water and extracted with EtOAc which was dried over MgSO₄ and concentrated *in vacuo* to give the title compound as a white solid (19mg). MS(ES): MH⁺ 262, M-H⁺ 260.

### Description 87 (D87)

### 4-(2-Methylthiazol-4-yl)-benzoic acid

The title compound was prepared according to P.J. Sanfilippo et al, US Patent number 5,342,851.

### Description 88 (D88)

### 7-Amino-1,4-dimethyl-1H-quinolin-2-one

To a solution of 7-amino-4-methyl-1*H*-quinolin-2-one (87mg, 0.5mmol) in dry DMF (2ml) was added sodium hydride (24mg, 60% disp. in oil, 0.6mmol) followed by methyl iodide (38µl, 0.6mmol) and the reaction stirred at room temperature for 1.5h. After quenching with water the mixture was extracted with EtOAc and the combined extracts were dried over MgSO₄ and concentrated in *vacuo* to give the crude product. Purification by SPE column chromatography, eluting with 0-10%MeOH/EtOAc gradient gave title compound (64mg) which was used in the next step without further purification. ¹H NMR (250MHz, CDCl₃) δ (ppm): 7.47 (d, 1 H), 6.59 (m, 2H), 6.55 (d, 1 H), 4.45 (br, 2H), 3.61 (s, 3H), 2.38 (s, 3H).

### Description 89 (D89)

### 7-Amino-1H-quinolin-2-one

The title compound may be prepared from 7-nitro-1*H*-quinolin-2-one (M. Nasr et al, J. Med. Chem., 1988, 31 (7), 1347) using the procedure outlined in Description 55 for the synthesis of 7-aminoquinoline.

### Description 90 (D90)

### N-(2,2-Dimethoxyethyl)-(1-phenyl)ethylamine

A solution of α-methylbenzylamine (8.37g, 0.069mol) and bromoacetaldehyde dimethylacetal (11.67g, 0.069mol) in acetonitrile (150ml) containing potassium carbonate (12.39g, 0.09mol) was heated at reflux for 2days then cooled. The resulting precipitate was filtered off and the filtrate was concentrated *in vacuo* to give the crude product as an oil. Chromatography on silica gel eluting with ethyl acetate afforded the title compound as an oil (10.1 g). ¹H NMR (400MHz, CDCl₃) δ(ppm): 7.31 (m, 3H), 7.23 (m, 2H), 4.43 (t, 1H), 3.75 (q, 1 H), 3.35 (s, 3H), 3.31 (s, 3H), 2.63 (dd, 1 H), 2.55 (dd, 1 H), 1.36 (d, 3H).

### Description 91 (D91)

### 1-Methylisoquinoline

To cooled chlorosulfonic acid (-10°C) (16ml) was cautiously added N-(2,2-dimethoxyethyl)-(1-phenyl)ethylamine (D90) (5g, 0.024mol) over a period of 2h. The reaction was allowed to warm to ambient temperature and stirring continued for 3d. The reaction was then poured into ice-water slurry (500ml), basified using solid potassium carbonate followed by extraction with DCM. The organic phase was separated, dried over MgSO₄, filtered and concentrated in *vacuo* to give the crude product as an oil. Chromatography on silica gel eluting with ethyl acetate afforded the title compound as a yellow oil (1.04g). ¹H NMR (400MHz, CDCl₃) δ (ppm): 8.40 (d, 1 H), 8.13 (d, 1 H), 7.81 (d, 1 H), 7.68 (t, 1 H), 7.60 (t, 1 H), 7.51 (d, 1 H), 2.97 (s, 3H).

### Description 92 (D92)

### 1-Methyl-5-nitroisoquinoline

A solution 1-methylisoquinoline (D91) (1 g, 7mmol) in sulfuric acid (2.5ml) was cooled (<4°C) and concentrated nitric acid (1ml) was added over 10 mins. The reaction was stirred for 30mins and then heated at 60°C for 2h. After cooling, the reaction mixture was poured into ice water slurry (100ml) and basified using solid potassium carbonate followed by extraction with DCM. The organic phase was separated, dried over MgSO₄, filtered and concentrated in *vacuo* to afford the product as a white solid (1.05g). ¹H NMR (400MHz, DMSO) δ (ppm): 8.69 (d, 1 H), 8.59 (m, 2H), 8.11 (d, 1 H), 7.88 (t, 1 H), 2.99 (s, 3H).

### Description 93 (D93)

### 1-Methyl-5-aminoisoquinoline

A solution of 1-methyl-5-nitroquinoline (D92) (1.0g, 5.32mmol) in methanol (40ml) with 10% palladium on charcoal (0.15g), was hydrogenated at atmospheric pressure for 5h. The catalyst was removed by filtration and the filtrate concentrated *in vacuo* affording a solid which was resuspended in ether and filtered off to give the title compound (0.82g). ¹H NMR (400MHz, CDCl₃) δ (ppm): 8.36 (d, 1 H), 7.55 (d, 1 H), 7.45 (d, 1 H), 7.39 (t, 1 H), 6.94 (d, 1 H), 4.20 (br, 2H), 2.93 (s, 3H).

### Description 94 (D94)

### 4-Bromo-N-isoquinolin-5-ylbenzamide

A solution of 5-aminoisoquinoline (800mg, 5.54mmol), 4-bromobenzoic acid (1.68g, 8.3mmol), (3-dimethylaminopropyl)-ethyl-carbodiimide hydrochloride (1.64g, 8.3mmol) and 4-dimethylaminopyridine (70mg, 0.6mmol) was stirred at room temperature overnight. The mixture was diluted with DCM, washed with saturated aqueous sodium bicarbonate solution and water, then dried over MgSO₄ and concentrated *in vacuo* to give the crude product. Purification by flash column chromatography eluting with an EtOAc/40-60°C pet. ether gradient gave the title compound as a white solid (1.48g). ¹H NMR (400MHz, CDCl₃) δ (ppm): 9.29 (br, 2H), 8.57 (d, 1 H), 8.11 (d, 1 H), 7.97, (d, 2H), 7.90 (d, 2H), 7.66 (m, 3H).

### Description 95

### 8-Aminoisoquinoline

The title compound was prepared according to W.A. Denny et al, J. Med. Chem., 2002, 45(3), 740.

### Description 96

### 7-Aminoisoquinoline

The title compound was prepared according to J.E. Macdonald *et al,* International Patent Application, Publication Number WO 97/06158.

### Description 97

### 6-Aminoisoquinoline

The title compound was prepared according to J.G. Durant *et al,* European Patent Application, Publication Number EP266949.

### Description 98

### 7-Amino-8-chloro-4,4-dimethyl-1-trifluoroacetyl-1,2,3,4-tetrahydroquinoline

To a stirred solution of 7-amino-4,4-dimethyl-7-1-trifluoroacetyl-1,2,3,4-tetrahydroquinoline (D28) (200mg, 0.735mmol) in DCM was added NCS (118 mg, 0.882 mmol), portion-wise over 15 mins. The reaction was stirred at room temperaure for 18 h. After this period, solvents were evaporated *in vacuo* and the residue purified by column chromatography (0-10% EtOAc / 40-60°C pet. ether) to give the product as a an oil (76mg). ¹H NMR (400MHz, CDCl₃) δ (ppm): 7.08 (d, 1 H), 6.72 (d, 1 H), 4.15-4.25 (m, 1 H), 4.05-4.15 (m, 2H), 3.40-3.50 (m, 1 H), 1.75-2.00 (m, 2H), 1.30 (d, 6H).

### Description 99

### (R)-2-Methyl-4-(6-methyl-2-pyridyl)piperazine

The title compound may be prepared from 2-bromo-6-methylpyridine using the procedure outlined in R. Bakthavalatcham, International Patent Application, Publication number WO 02/0822 for the synthesis of (R)-2-methyl-4-(3-trifluoromethyl-2-pyridyl)piperazine.

### Description 100 (D100)

### (R)-2-Methyl-4-(3-methyl-2-pyridyl)piperazine

The title compound may be prepared from 2-bromo-3-methylpyridine using the procedure outlined in R. Bakthavalatcham, International Patent Application, Publication number WO 02/0822 for the synthesis of (R)-2-methyl-4-(3-trifluoromethyl-2-pyridyl)piperazine.

### Description 101 (D101)

### 1-(5-Trifluoromethlpyrid-2-yl)-piperidine-4-carboxylic acid

The title compound may be prepared from 2-chloro-5-trifluoromethylpyridine and piperidine-4-carboxylic acid using the procedure outlined in German Patent Application, Publication number DE4234295 for the synthesis of 1-(5-cyanopyrid-2-yl)-piperidine-4-carboxylic acid.

### Description 102 (D102)

### 1-(6-Trifluoromethlpyrid-2-yl)-piperidine-4-carboxylic acid

The title compound may be prepared from 2-chloro-6-trifluoromethylpyridine and piperidine-4-carboxylic acid using the procedure outlined in German Patent Application, Publication number DE4234295 for the synthesis of 1-(5-cyanopyrid-2-yl)-piperidine-4-carboxylic acid.

### Description 103 (D103)

### 5-Chloro-7-nitro-1-trifluoroacetyl-1,2,3,4-tetrahydroquinoline

Using the procedure outlined in Description 4, the title compound was prepared from 5-chloro-7-nitro-1,2,3,4-tetrahydroquinoline (D81) (200mg, 0.94mmol) as an orange solid (285mg). ¹H NMR (400MHz, CDCl₃) δ (ppm): 8.58 (br.s, 1 H), 8.15 (d, 1 H), 3.88 (m, 2H), 3.00 (t, 2H), 2.19 (m, 2H).

### Description 104 (D104)

### 7-Amino-5-chloro-1-trifluoroacetyl-1,2,3,4-tetrahydroquinoline

Using the procedure outlined in Description 83, the title compound was prepared from 5-chloro-7-nitro-1-trifluoroacetyl-1,2,3,4-tetrahydroquinoline (D103) (280mg, 0.91mmol) as a white solid (237mg). ¹H NMR (400MHz, CDCl₃) δ (ppm): 7.00 (br.s, 1 H), 6.65 (d, 1 H), 3.77 (m, 2H), 3.70 (br, 2H), 2.78 (t, 2H), 2.06 (m, 2H).

### Description 105 (D105)

### Ethyl 6-(2,4-difluorophenyl)-2-methylnicotinate

The title compound was prepared from dimethylamino-(2,4-difluorophenyl)-propan-1-one and ethyl 3-aminocrotonate using the general procedure outlined in D18. ¹H NMR (250MHz, CDCl₃) δ (ppm): 8.25 (d, 1H), 8.13 (dt, 1 H), 7.67 (dd, 1 H), 6.86-7.05 (m, 2H), 4.40 (q, 2H), 2.90 (s, 3H), 1.41 (t, 3H).

### Description 106 (D106)

### 6-(2,4-Difluorophenyl)-2-methylnicotinic acid

Using the procedure outlined in Description 23, the title compound was prepared from ethyl 6-(2,4-difluorophenyl)-2-methylnicotinate (D105) (2.1 g, 7.6mmol) as a yellow solid (1.4g). ¹H NMR (250MHz, CDCl₃) δ (ppm): 8.30 (d, 1 H), 8.12 (dt, 1 H), 7.66 (dd, 1 H), 6.86-7.05 (m, 2H), 2.91 (s, 3H).

### Description 107 (D107)

### Ethyl 6-(3,4-difluorophenyl)-2-methylnicotinate

The title compound was prepared from dimethylamino-(3,4-difluorophenyl)-propan-1-one and ethyl 3-aminocrotonate using the general procedure outlined in D18. ¹H NMR (250MHz, CDCl₃) δ (ppm): 8.26 (d, 1H), 7.96 (ddd, 1 H), 7.79 (m, 1 H), 7.57 (d, 1 H), 7.20-7.31 (m, 1 H), 4.40 (q, 2H), 2.90 (s, 3H), 1.42 (t, 3H).

### Description 108 (D108)

### 6-(2,4-Difluorophenyl)-2-methylnicotinic acid

Using the procedure outlined in Description 23, the title compound was prepared from ethyl 6-(2,4-difluorophenyl)-2-methylnicotinate (D105) (5.3g, 19.1 mmol) as a yellow solid (1.8g). ¹H NMR (250MHz, CDCl₃) δ (ppm): 8.32 (d, 1 H), 7.96 (ddd, 1 H), 7.79 (m, 1 H), 7.57 (d, 1 H), 7.20-7.31 (m, 1 H), 2.92 (s, 3H).

### EXAMPLES

### Example 1

### N-(1-Methyl-1,2,3,4-tetrahydroquinolin-7-yl)-1,1'-biphenyl-4-carboxamide

To a solution of 7-amino-1-methyl-1,2,3,4-tetrahydroquinoline (D3) (325mg, 2mmol) in DCM (10ml) was added 4-biphenylcarboxylic acid (476mg, 2.4mmol) and 1-(3-dimethylaminopropyl)-3-ethylcarbodiimide hydrochloride (444mg, 2.4mmol) and the reaction stirred at ambient temperature. After 1 h the reaction mixture was filtered to give the title compound as a white solid. The filtrate was diluted with DCM, washed with sat. aq. sodium bicarbonate solution, dried over MgSO₄ and concentrated *in vacuo* to give further crude product which was purified by silica SPE chromatography. Elution with an EtOAc/60-80°C petroleum ether gradient gave a mixture of the title compound and the starting acid. These fractions were washed with further sat. aq. sodium bicarbonate solution, dried over MgSO₄ and concentrated *in vacuo* to give further title compound as a white solid. ¹H NMR (400MHz, CDCl₃) δ (ppm): 7.94 (d, 2H), 7.70 (m, 3H), 7.63 (d, 2H), 7.48 (t, 2H), 7.40 (t, 1 H), 7.07 (d, 1 H), 6.93 (d, 1 H), 6.75 (dd, 1 H), 3.25 (m, 2H), 2.94 (s, 3H), 2.76 (t, 2H), 1.98 (m, 2H).

### Example 2

### N-(1-Methyl-1,2,3,4-tetrahydroquinolin-7-yl)-6-phenylnicotinamide

To a solution of 6-phenylnicotinic acid (D48) (500mg, 2.51 mmol), 1-(3-dimethylaminopropyl)-3-ethylcarbodiimide hydrochloride (963mg, 5.03mmol) and 1-hydroxybenzotriazole hydrate (340mg, 2.51mmol) in DCM (20ml) was added a solution of 7-amino-1-methyl-1,2,3,4-tetrahydroquinoline (D3) (407mg, 2.51mmol) in DCM (5ml). The reaction mixture was stirred overnight then washed with sat. aq. sodium hydrogen carbonate solution (2 x 20ml) and brine (20ml). The organics were dried over MgSO₄ and concentrated *in vacuo* to give the crude product which was purified by flash column chromatography. Elution with 10-20% EtOAc /DCM gave the title compound as a yellow solid. ¹H NMR (250MHz, CDCl₃) δ (ppm): 9.14 (d, 1 H), 8.25 (dd, 1 H), 8.07 (m, 2H), 7.85 (d, 1 H), 7.71 (br, 1 H), 7.48 (m, 3H), 7.03 (br, 1 H), 6.93 (d, 1 H), 6.75 (dd, 1 H), 3.25 (m, 2H), 2.93 (s, 3H), 2.75 (t, 2H), 1.98 (m, 2H).

### Examples 3-23

7-amino-1-methyl-1,2,3,4-tetrahydroquinoline (D3) (0.03mmol) in DCM (0.5ml) was reacted with the appropriate acid (D31-47, D50 & D51-53) (0.03mmol) in DMF (0.25ml) in the presence of hydroxybenzotriazole hydrate (0.06mmol) and excess polymer supported 1,3-dicyclohexylcarbodiimide in 1:1 DCM/THF (0.5ml). On completion, the resin was removed by filtration and the impurities removed by ion-exchange yielding the products given in Table 1.

**Table 1**

| Example | Name | MH⁺ |
|---|---|---|
| 3 | N'-Methyl-N-(1-methyl-1,2,3,4-tetrahydroquinolin-7-yl)-1,1'-biphenyl-4,4'-dicarboxamide | 400 |
| 4 | 4'-Acetamido-2'-methyl-N-(1-methyl-1,2,3,4-tetrahydroquinolin-7-yl)-1,1'-biphenyl-4-carboxamide | 414 |
| 5 | N',N'-Dimethyl-N-(1-methyl-1,2,3,4-tetrahydroquinolin-7-yl)-1,1'-biphenyl-4,4'-dicarboxamide | 414 |
| 6 | 2'-Methyl-N-(1-methyl-1,2,3,4-tetrahydroquinolin-7-yl)-1,1'-biphenyl-4-carboxamide | 357 |
| 7 | 3'-Acetyl-N-(1-methyl-1,2,3,4-tetrahydroquinolin-7-yl)-1,1'-biphenyl-4-carboxamide | 385 |
| 8 | 3'-(Methylsulfamoyl)-N-(1-methyl-1,2,3,4-tetrahydroquinolin-7-yl)-1,1'-biphenyl-4-carboxamide | 436 |
| 9 | N-(1-Methyl-1,2,3,4-tetrahydroquinolin-7-yl)-1,1'-biphenyl-4,4'-dicarboxamide | 386 |
| 10 | N-(1-Methyl-1,2,3,4-tetrahydroquinolin-7-yl)-1,1'-biphenyl-3',4-dicarboxamide | 386 |
| 11 | 4'-Acetyl-N-(1-methyl-1,2,3,4-tetrahydroquinolin-7-yl)-1,1'-biphenyl-4-carboxamide | 385 |
| 12 | 2-Methyl-N-(1-methyl-1,2,3,4-tetrahydroquinolin-7-yl)-1,1'-biphenyl-4-carboxamide | 357 |
| 13 | 3-Chloro-N-(1-methyl-1,2,3,4-tetrahydroquinolin-7-yl)-1,1'-biphenyl-4-carboxamide | 377/379 |
| 14 | N-(1-Methyl-1,2,3,4-tetrahydroquinolin-7-yl)-4-(3-thienyl)benzamide | 349 |
| 15 | N-(1-Methyl-1,2,3,4-tetrahydroquinolin-7-yl)-4-(2-thienyl)benzamide | 349 |
| 16 | 4-(1-Methyl-4-pyrazolyl)-N-(1-methyl-1,2,3,4-tetrahydroquinolin-7-yl)benzamide | 347 |
| 17 | 3-Methyl-N-(1-methyl-1,2,3,4-tetrahydroquinolin-7-yl)-4-(3-pyridyl)benzamide | 358 |
| 18 | N-(1-Methyl-1,2,3,4-tetrahydroquinolin-7-yl)-4-(2-pyrazinyl)benzamide | 345 |
| 19 | N-(1-Methyl-1,2,3,4-tetrahydroquinolin-7-yl)-3-(1-pyrazolyl)benzamide | 333 |
| 20 | N-(1-Methyl-1,2,3,4-tetrahydroquinolin-7-yl)-2-(4-pyridyl)furan-4-carboxamide | 334 |
| 21 | N-(1-Methyl-1,2,3,4-tetrahydroquinolin-7-yl)-5-phenylthiophene-2-carboxamide | 349 |
| 22 | N-(1-Methyl-1,2,3,4-tetrahydroquinolin-7-yl)-3-(3-pyridyl)benzamide | 344 |
| 23 | N-(1-Methyl-1,2,3,4-tetrahydroquinolin-7-yl)-4-(1-oxo-indan-5-yl)-benzamide | 397 |

### Example 24

### N-(1,2,3,4-Tetrahydroquinolin-7-yl)-1,1'-biphenyl-4-carboxamide

To a solution of 7-amino-1-trifluoroacetyl-1,2,3,4-tetrahydroquinoline (D5) (1.17g, 4.78mmol) in DCM (20ml) was added 4-biphenylcarboxylic acid (1.14g, 5.73mmol), 1-(3-dimethylaminopropyl)-3-ethylcarbodiimide hydrochloride (1.06g, 5.73mmol) and 4-dimethylaminopyridine (70mg, 0.57mmol) and the reaction stirred at room temperature. After 3.75h the reaction mixture was treated with 2M sodium hydroxide solution overnight. An acid/base work-up followed by flash column chromatography with an EtOAc/60-80°C petroleum ether gradient gave the title compound as a beige solid. ¹H NMR (250MHz, CDCl₃) δ (ppm): 7.91 (d, 2H), 7.63 (m, 5H), 7.47 (t, 2H), 7.41 (t, 1 H), 7.11 (d, 1 H), 6.91 (d, 1 H), 6.61 (dd, 1 H), 3.94 (br, 1 H), 3.31 (m, 2H), 2.74 (t, 2H), 1.93 (m, 2H), 1.57 (br, 2H).

### Example 25

### 6-Phenyl-N-(1-trifluoroacetyl-1,2,3,4-tetrahydroquinolin-7-yl)nicotinamide

To a solution of 7-amino-1-trifluoroacetyl-1,2,3,4-tetrahydroquinoline (D5) (122mg, 0.5mmol) in DCM (2ml) was added 6-phenylnicotinic acid (D48) (119mg, 0.6mmol), 1-(3-dimethylaminopropyl)-3-ethylcarbodiimide hydrochloride (111mg, 0.6mmol) and 4-dimethylaminopyridine (7mg, 0.06mmol) and the reaction stirred at ambient temperature for until complete by tlc. The mixture was then washed with 2M sodium hydroxide solution (1ml), dried over magnesium sufate and concentrated *in vacuo* to give the crude product which was purified by silica SPE chromatography. Elution with 20% EtOAc/60-80°C petroleum ether gave an off-white solid which was recrystallised from EtOAc/60-80°C petroleum ether giving the title compound as a white solid. ¹H NMR (250MHz, CDCl₃) δ (ppm): 9.10 (d, 1 H), 8.20 (m, 2H), 8.04 (m, 2H), 7.86 (br, 1 H), 7.82 (dd, 1 H), 7.72 (br, 1 H), 7.50 (m, 3H), 7.19 (1 H, d), 3.84 (m, 2H), 2.84 (m, 2H), 2.06 (m, 2H).

### Example 26

### N-(1,2,3,4-Tetrahydroquinolin-7-yl)-6-phenylnicotinamide

6-Phenyl-N-(1-trifluoroacetyl-1,2,3,4-tetrahydroquinolin-7-yl)nicotinamide (Example 25) (155mg, 0.364mmol) and potassium carbonate (252mg, 1.82mmol) in water (2ml) and methanol (8ml) was stirred at ambient temperature for 40mins. The resulting suspension was filtered and the solid was washed with water then dried *in vacuo* to give the title compound as a white solid. ¹H NMR (250MHz, CDCl₃) δ (ppm): 9.11 (m, 1 H), 8.22 (dd, 1 H), 8.03 (m, 2H), 7.82 (d, 1 H), 7.75 (br, 1 H), 7.48 (m, 3H), 7.06 (d, 1 H), 6.91 (d, 1 H), 6.62 (dd, 1 H), 3.95 (br, 1 H), 3.30 (m, 2H), 2.73 (t, 2H), 1.93 (m, 2H).

### Example 27

### N-(1-Acetyl-1,2,3,4-tetrahydroquinolin-7-yl)-1,1'-biphenyl-4-carboxamide

To a solution of N-(1,2,3,4-tetrahydroquinolin-7-yl)-1,1'-biphenyl-4-carboxamide (Example 24) (66mg, 0.2mmol) in DCM (1ml) was added triethylamine (42ul, 0.3mmol) followed by acetyl chloride (20.5ul, 0.3mmol). The reaction was stirred at ambient temperature for 15mins then treated with polymer supported trisamine resin (31 mg, 0.1mmol) and polymer supported isocyanate resin (20mg, 0.04mmol). After 5mins the resins were removed by filtration and the filtrate was washed with 2M hydrochloric acid, dried over MgSO₄ and concentrated *in vacuo* to give the title compound as an off-white solid. ¹H NMR (250MHz, CDCl₃) δ (ppm): 7.94 (d, 2H), 7.84 (d, 1 H), 7.78 (br, 1 H), 7.72 (d, 2H), 7.64 (d, 2H), 7.45 (m, 4H), 7.15 (d, 1 H), 3.80 (t, 2H), 2.74 (t, 2H), 2.33 (s, 3H), 1.98 (qn, 2H).

### Example 28

### N-(1-Methoxyacetyl-1,2,3,4-tetrahydroquinolin-7-yl)-1,1'-biphenyl-4-carboxamide

Using the procedure outlined in Example 27, the title compound was prepared from N-(1,2,3,4-tetrahydroquinolin-7-yl)-1,1'-biphenyl-4-carboxamide (Example 24) (66mg, 0.2mmol) and methoxyacetyl chloride (27.5ul, 0.3mmol) as a pale pink solid. ¹H NMR (250MHz, CDCl₃) δ (ppm): 8.09 (br, 1 H), 7.97 (d, 2H), 7.80 (s,1 H), 7.71 (d, 2H), 7.64 (d, 2H), 7.45 (m, 4H), 7.16 (d, 1 H), 4.31 (s, 2H), 3.79 (t, 2H), 3.46 (s, 3H), 2.74 (t, 2H), 1.98 (t, 2H).

### Examples 29 & 30

### N-[1-(2-Acetoxyethyl)-1,2,3,4-tetrahydroquinolin-7-yl]-1,1'-biphenyl-4-carboxamide & N-[1-(2-Acetoxyethyl)-6-bromo-1,2,3,4-tetrahydroquinolin-7-yl]-1,1'-biphenyl-4-carboxamide

N-(1,2,3,4-Tetrahydroquinolin-7-yl)-1,1'-biphenyl-4-carboxamide (Example 24) (66mg, 0.2mmol), potassium carbonate (164mg,1.2mmol) and 2-bromoethyl acetate (132ul, 1.2mmol) in dimethylformamide (1ml) were heated at 80°C for 20h then 120°C for 24h. Further 2-bromoethyl acetate (132ul, 1.2mmol) was added and heating was continued at 120°C for 20h. After cooling to ambient temperature the reaction mixture was diluted with EtOAc (10ml), filtered and concentrated *in vacuo* to give the crude product which was purified by silica SPE chromatography. Elution with 10% EtOAc/60-80°C petroleum ether gave the 6-bromo-title compound (Example 30) as a yellow gum.¹H NMR (400MHz, CDCl₃) δ (ppm): 8.38 (br,1 H), 8.01 (s, 1 H), 8.00 (d, 2H), 7.73 (d, 2H), 7.64 (d, 2H), 7.48 (t, 2H), 7.40 (t, 1 H), 7.10 (s, 1 H), 4.34 (t, 2H), 3.62 (t, 2H), 3.36 (m, 2H), 2.72 (m, 2H), 2.06 (s, 3H), 1.93 (m, 2H). MS(ES): MH⁺ 493/495. Elution with 15% EtOAc/60-80°C petroleum ether gave the non-brominated title compound (Example 29) as a white solid. ¹H NMR (400MHz, CDCl₃) δ (ppm): 7.94 (d, 2H), 7.75 (br,1 H), 7.70 (d, 2H), 7.63 (d, 2H), 7.48 (t, 2H), 7.40 (t, 1 H), 7.15 (d, 1 H), 6.92 (d, 1 H), 6.74 (dd, 1 H), 4.30 (t, 2H), 3.57 (t, 2H), 3.36 (m, 2H), 2.74 (t, 2H), 2.06 (s, 3H), 1.94 (m, 2H).

### Example 31

### N-[1-(2-Methoxycarbonylethyl)-1,2,3,4-tetrahydroquinolin-7-yl]-1,1'-biphenyl-4-carboxamide

Using the procedure outlined in Example 29, the title compound was prepared from N-(1,2,3,4-tetrahydroquinolin-7-yl)-1,1'-biphenyl-4-carboxamide (Example 24) (66mg, 0.2mmol) and methyl 3-bromopropionate (264ul, 2.4mmol) as a yellow gum. ¹H NMR (400MHz, CDCl₃) δ (ppm): 7.94 (d, 2H), 7.76 (br,1 H), 7.70 (d, 2H), 7.63 (d, 2H), 7.48 (t, 2H), 7.40 (t, 1 H), 7.04 (d, 1 H), 6.93 (d, 1 H), 6.79 (dd, 1 H), 3.69 (s, 3H), 3.65 (t, 2H), 3.31 (m, 2H), 2.72 (t, 2H), 2.67 (t, 2H), 1.93 (m, 2H).

### Example 32

### N-[1-(2-Hydroxyethyl)-1,2,3,4-tetrahydroquinolin-7-yl]-1,1'-biphenyl-4-carboxamide

N-(1,2,3,4-Tetrahydroquinolin-7-yl)-1,1'-biphenyl-4-carboxamide (Example 24) (100mg, 0.304mmol), potassium carbonate (126mg, 0.913mmol), sodium iodide (9mg, 0.06mmol) and 2-bromoethanol (216ul, 3.04mmol) in dioxane (1 ml) were heated at 60°C for 8d. After cooling to ambient temperature the reaction mixture was diluted with EtOAc (10ml), filtered and concentrated in *vacuo* to give the crude product which was purified on a silica SPE column. Elution with an EtOAc/60-80°C petroleum ether gradient gave the title compound as a beige solid. ¹H NMR (400MHz, CDCl₃) δ (ppm): 7.93 (d, 2H), 7.78 (s,1 H), 7.70 (d, 2H), 7.63 (d, 2H), 7.48 (t, 2H), 7.40 (t, 1 H), 7.19 (d, 1 H), 6.93 (d, 1 H), 6.74 (dd, 1 H), 3.87 (m, 2H), 3.49 (t, 2H), 3.35 (m, 2H), 2.75 (t, 2H), 1.97 (m, 3H).

### Example 33

### N-[1-(2-n-Propyloxyethyl)-1,2,3,4-tetrahydroquinolin-7-yl]-1,1'-biphenyl-4-carboxamide

N-(1,2,3,4-Tetrahydroquinolin-7-yl)-1,1'-biphenyl-4-carboxamide (Example 24) (66mg, 0.2mmol), potassium carbonate (41 mg, 0.3mmol), potassium iodide (100mg, 0.6mmol) and 2-chloroethyl-n-propyl ether (38ul, 0.3mmol) in DMF (1 ml) were heated at 60°C for 17h then 100°C for 48h. After cooling to ambient temperature the reaction mixture was purified on a silica SPE column. Elution with 8% EtOAc/60-80°C petroleum ether gave the title compound as a yellow solid. ¹H NMR (250MHz, CDCl₃) δ (ppm): 7.93 (d, 2H), 7.72 (br,1 H), 7.70 (d, 2H), 7.63 (d, 2H), 7.48 (t, 2H), 7.40 (t, 1 H), 7.03 (d, 1 H), 6.92 (d, 1 H), 6.74 (dd, 1 H), 3.66 (t, 2H), 3.50 (t, 2H), 3.40 (m, 4H), 2.73 (t, 2H), 1.93 (qn, 2H), 1.58 (sx, 2H), 0.91 (t, 3H).

### Example 34

### N-[1-(2-Methoxyethyl)-1,2,3,4-tetrahydroquinolin-7-yl]-1,1'-biphenyl-4-carboxamide

To a solution of 7-amino-1-(2-methoxyethyl)-1,2,3,4-tetrahydroquinoline (D13) (72mg, 0.35mmol) in DCM (2.5ml) was added 4-biphenylcarboxylic acid (104mg, 0.53mmol), 1-(3-dimethylaminopropyl)-3-ethylcarbodiimide hydrochloride (97mg, 0.53mmol) and 4-dimethylaminopyridine (6mg, 0.05mmol) and the reaction stirred at ambient temperature overnight. The mixture was diluted with DCM, washed with 2M sodium hydroxide solution, dried over MgSO₄ and concentrated *in vacuo* to give the crude product which was purified by silica SPE chromatography. Elution with 20% EtOAc/60-80°C petroleum ether gave the title compound as a pale yellow solid. ¹H NMR (250MHz, CDCl₃) δ (ppm): 7.93 (d, 2H), 7.77 (br,1 H), 7.69 (d, 2H), 7.62 (d, 2H), 7.47 (t, 2H), 7.39 (t, 1 H), 7.06 (d, 1 H), 6.91 (d, 1 H), 6.73 (dd, 1 H), 3.63 (t, 2H), 3.49 (t, 2H), 3.37 (s, 3H), 3.36 (t, 2H), 2.73 (t, 2H), 1.91 (m, 2H).

### Example 35

### N-[1-(2-Dimethylaminoethyl)-1,2,3,4-tetrahydroquinolin-7-yl]-4-biphenyl-carboxamide

To a solution of 7-amino-1-(2-dimethylaminoethyl)-1,2,3,4-tetrahydroquinoline (D14) (38mg, 0.152mmol) in DCM (1ml) was added 4-biphenylcarboxylic acid (45mg, 0.22mmol), 1-(3-dimethylaminopropyl)-3-ethylcarbodiimide hydrochloride (42mg, 0.22mmol) and 4-dimethylaminopyridine (2.8mg, 0.022mmol) and the reaction stirred at ambient temperature overnight. The crude reaction mixture was loaded directly onto a silica SPE column and elution with EtOAc followed by 1% triethylamine/EtOAc gave the title compound as a red gum. ¹H NMR (250MHz, CDCl₃) δ (ppm): 7.94 (d, 2H), 7.75 (br,1H), 7.70 (d, 2H), 7.64 (d, 2H), 7.47 (t, 2H), 7.39 (t, 1 H), 7.01 (d, 1 H), 6.92 (d, 1 H), 6.82 (dd, 1 H), 3.43 (m, 2H), 3.33 (m, 2H), 2.73 (t, 2H), 2.55 (m, 2H), 2.33 (s, 6H), 1.95 (m, 2H).

### Example 36

### N-[1-(2-Diisopropylaminoethyl)-1,2,3,4-tetrahydroquinolin-7-yl]-1,1'-biphenyl-4-carboxamide

7-Amino-1-(2-diisopropylaminoethyl)-1,2,3,4-tetrahydroquinoline (D15) (100mg, 0.36mmol), 4-biphenylcarbonyl chloride (258mg, 1.11mmol) and pyridine (0.5ml, 6.2mmol) in DCM (5ml) were stirred at room temperatue for 4 hours. 10% Potassium carbonate solution was then added and the mixture extracted with DCM which was dried over MgSO₄ and concentrated *in vacuo* to give the crude product. Purification by flash column chromatography eluting with 0-5% methanol/DCM gave the title compound as an off-white solid. ¹H NMR (400MHz, CDCl₃) δ (ppm): 7.92 (d, 2H), 7.71 (d, 2H), 7.63 (m, 3H), 7.47 (t, 2H), 7.41 (t, 1 H), 6.99 (br, 1 H), 6.90 (d, 1 H), 6.74 (d, 1 H), 3.37 (m, 2H), 3.30 (m, 2H), 3.05 (sp, 2H), 2.72 (t, 2H), 2.66 (m, 2H), 1.93 (m, 2H), 1.05 (d, 12H).

### Example 37

### N-[1-(2-Morpholin-4-ylethyl)-1,2,3,4-tetrahydroquinolin-7-yl]-1,1'-biphenyl-4-carboxamide

Using the procedure outlined in Example 35, the title compound was prepared from 7-amino-1-(2-morpholin-4-ylethyl)-1,2,3,4-tetrahydroquinoline (D17) (0.1 mmol) and 4-biphenylcarboxylic acid (24mg, 0.12mmol) as a red gum. ¹H NMR (250MHz, CDCl₃) δ (ppm): 7.94 (d, 2H), 7.86 (br,1H), 7.69 (d, 2H), 7.62 (d, 2H), 7.50 (t, 2H), 7.39 (t, 1 H), 7.13 (d, 1 H), 6.91 (d, 1 H), 6.78 (dd, 1 H), 3.75 (m, 4H), 3.46 (m, 2H), 3.33 (m, 2H), 2.80-2.40 (m, 8H), 1.92 (qn, 2H).

### Example 38

### 6-(4-Fluorophenyl)-N-(1,2,3,4-tetrahydroquinolin-7-yl)nicotinamide

To a solution of 7-amino-1-trifluoroacetyl-1,2,3,4-tetrahydroquinoline (D5) (46mg, 0.19mmol) in DCM (1ml) was added 6-(4-fluorophenyl)nicotinic acid (D49) (41 mg, 0.19mmol), 1-(3-dimethylaminopropyl)-3-ethylcarbodiimide hydrochloride (44mg, 0.23mmol) and 4-dimethylaminopyridine (11 mg, 0.09mmol) and the reaction stirred at ambient temperature overnight. The crude reaction mixture was purified on a silica SPE column eluting with 0-2% methanol/DCM to give the 1-trifluoroacetyl- intermediate. This was treated with potasium carbonate (52mg, 0.38mmol) in methanol (2ml) until tlc showed complete deprotection. The reaction mixture was diluted with water and extracted with DCM which was dried over MgSO₄, concentrated *in vacuo* and purified on a silica SPE column to give the title compound as a solid. ¹H NMR (250MHz, CDCl₃) δ (ppm): 9.09 (d, 1 H), 8.23 (dd, 1 H), 8.06 (dd, 2H), 7.81 (d, 1 H), 7.64 (br, 1 H), 7.19 (t, 2H), 7.07 (br, 1 H), 6.92 (d, 1 H), 6.62 (dd, 1 H), 3.95 (br, 1 H), 3.32 (m, 2H), 2.74 (t, 2H), 1.94 (m, 2H).

### Example 39

### 6-(4-Fluorophenyl)-2-methyl-N-(1,2,3,4-tetrahydroquinolin-7-yl)nicotinamide

Using the procedure outlined in Example 38 the title compound was prepared from 7-amino-1-trifluoroacetyl-1,2,3,4-tetrahydroquinoline (D5) (46mg, 0.19mmol) and 2-methyl-6-(4-fluorophenyl)-nicotinic acid (D24) (44mg, 0.19mmol) as a solid. ¹H NMR (250MHz, CDCl₃) δ (ppm): 8.01 (dd, 1 H), 7.81 (d, 1 H), 7.56 (d, 1 H), 7.29 (br, 1 H), 7.16 (t, 2H), 7.08 (br, 1 H), 6.91 (d, 1 H), 6.57 (br, 1 H), 3.96 (br, 1 H), 3.31 (m, 2H), 2.74 (t, 2H), 1.94 (m, 2H).

### Example 40

### N-(4,4-Dimethyl-1,2,3,4-tetrahydroquinolin-7-yl)-4-(3-chloro-2-pyridyl)-piperazine-1-carboxamide

To a stirred solution of 7-amino-4,4-dimethyl-1-trifluoroacetyl-1,2,3,4-tetrahydroquinoline (D28) (75mg, 0.373mmol) and pyridine (33ul, 0.410mmol) in DCM (5ml) was added phenylchloroformate (51 ul, 0.410mmol). The reaction mixture was stirred for 1 h at ambient temperature before triethylamine (57ul, 0.410mmol) was added and then left to stir for a further 30min. After this period, 4-(3-chloro-2-pyridyl)-piperazine (US Patent number 4,456,604) (74mg, 0.373mmol) in DCM (5ml) was added and the reaction stirred at ambient temperature for 18h. On completion, the solvents were evaporated *in vacuo* and the residue purified directly by chromatography, eluting with 10-100% EtOAc/40-60°C petroleum ether, to give *N*-(4,4-dimethyl-1-trifluoroacetyl-1,2,3,4-tetrahydroquinol-7-yl)-4-(3-chloro-2-pyridiyl)-piperazine-1-carboxamide as a colourless oil. ¹H NMR (400MHz, CDCl₃) δ (ppm): 8.20 (dd 1 H), 7.62 (dd, 1 H), 7.50-7.55 (br, 1 H), 7.40-7.45 (Br, 1 H), 7.30 (d, 1 H), 6.89 (dd, 1 H), 6.42 (br, 1 H), 3.80-3.85 (m, 2H), 3.60-3.65 (m, 4H), 3.40-3.45 (m, 4H), 1.33 (s, 6H). MS (ES): MH⁺ 496/498.
A suspension of *N*-(4,4-dimethyl-1-trifluoroacetyl-1,2,3,4-tetrahydroquinol-7-yl)-4-(3-chloro-2-pyridyl)-piperazine-1-carboxamide (125 mg, 0.253 mmol) and potassium carbonate (105 mg, 0.758 mmol) in methanol (5 ml) and water (5 ml) was heated at 50°C for 3h. After this period, the solvents were evaporated in *vacuo* and the residue partitioned between DCM (50ml) and water (50ml). The aqueous layer was re-extracted with DCM (2 x 50 ml) and then the combined organic layers dried (Na₂SO₄) and the solvents evaporated *in vacuo* to give the title compound as a white solid. ¹H NMR (400MHz, CDCl₃) δ (ppm): 8.19 (dd, 1 H), 7.61 (dd, 1 H), 7.07 (d, 1 H), 6.87 (dd, 1 H), 6.75 (d, 1 H), 6.41 (dd, 1 H), 6.19 (br, 1 H), 3.85-3.95 (br, 1 H), 3.60-3.70 (m, 4H), 3.30-3.40 (m, 4H), 3.25-3.35 (m, 2H), 1.65-1.75 (m, 2H), 1.26 (s, 6H). MS (ES): MH⁺ 400 / 402.

### Example 41

### N-(4,4-Dimethyl-1,2,3,4-tetrahydroquinolin-7-yl)-4-(3-trifluoromethyl-2-pyridyl)-piperazine-1-carboxamide

To a stirred solution of 7-amino-4,4-dimethyl-1-trifluoroacetyl-1,2,3,4-tetrahydroquinoline (D28) (50mg, 0.233mmol) and triethylamine (65ul, 0.465mmol) in DCM (2 ml) at 0°C was added triphosgene (23mg, 0.077mmol). The reaction mixture was stirred for 2 min at 0°C and then at ambient temperature for 20 min. After this period, 4-(3-trifluoromethyl-2-pyridyl)-piperazine (54mg, 0.233mmol) in DCM (1 ml) was added and the reaction stirred at ambient temperature for 18h. On completion, the solvents were evaporated in *vacuo* and the residue purified directly by chromatography, eluting with 10-100% EtOAc/40-60°C petroleum ether, to give *N*-(4,4-dimethyl-1-trifluoroacetyl-1,2,3,4-tetrahydroquinol-7-yl)-4-(3-trifluoromethyl-2-pyridiyl)-piperazine-1-carboxamide as a colourless oil. ¹H NMR (400MHz, CDCl₃) δ (ppm): 8.46 (dd 1 H), 7.89 (dd, 1 H), 7.50-7.455 (br-s, 1 H), 7.40-7.45 (m, 1 H), 7.27 (d, 1 H), 7.05 (dd, 1 H), 6.57 (s, 1 H), 3.80-3.85 (m, 2H), 3.60-3.65 (m, 4H), 3.25-3.35 (m, 4H), 1.85-1.90 (m, 2H), 1.33 (s, 6H). MS (ES): MH⁺ 530.
A suspension of *N*-(4,4-dimethyl-1-trifluoroacetyl-1,2,3,4-tetrahydroquinol-7-yl)-4-(3-trifluoromethyl-2-pyridyl)-piperazine-1-carboxamide (50 mg, 0.0955 mmol) and potassium carbonate (40 mg, 0.287 mmol) in methanol (5 ml) and water (5 ml) was heated at 60°C for 4h. After this period, the solvents were evaporated *in vacuo* and the residue partitioned between DCM (50ml) and water (30ml). The aqueous layer was re-extracted with DCM (3 x 50 ml) and then the combined organic layers dried (Na₂SO₄) and the solvents evaporated *in vacuo* to give the title compound as a white solid. ¹H NMR (400MHz, CDCl₃) δ (ppm): 8.45 (dd, 1 H), 7.90 (dd, 1 H), 7.00-7.05 (m, 2H), 6.70 (d, 1 H), 6.70 (d, 1 H), 6.43 (dd, 1 H), 6.32 (br-s, 1 H), 3.60-3.65 (br, 4H), 3.20-3.30 (m, 6H), 1.65-1.70 (m, 2H), 1.26 (s, 6H). MS (ES): MH⁺ 434.

### Example 42

### N-(4,4-Dimethyl-1,2,3,4-tetrahydroquinolin-7-yl)-4-(3-methyl-2-pyridyl)-piperazine-1-carboxamide

To a stirred solution of 7-amino-4,4-dimethyl-1-trifluoroacetyl-1,2,3,4-tetrahydroquinoline (D28) (75mg, 0.276mmol) and triethylamine (56ul, 0.551 mmol) in DCM (3ml) at 0°C was added triphosgene (27ul, 0.092mmol). The reaction mixture was stirred for 2 min at 0°C and then at ambient temperature for 20 min. After this period, 4-(3-methyl-2-pyridyl)-piperazine (D100) (49mg, 0.276mmol) in DCM (2ml) was added and the reaction stirred at ambient temperature for 18h. On completion, the solvents were evaporated in *vacuo* and the residue purified directly by chromatography, eluting with 10-100% EtOAc/40-60°C petroleum ether, to give *N*-(4,4-dimethyl-1-trifluoroacetyl-1,2,3,4-tetrahydroquinol-7-yl)-4-(3-methyl-2-pyridiyl)-piperazine-1-carboxamide as a colourless oil. ¹H NMR (400MHz, CDCl₃) δ (ppm): 8.16 (dd 1 H), 7.50-7.55 (br, 1 H), 7.40-7.45 (m, 2H), 7.26 (d, 1 H), 6.89 (dd, 1 H), 6.60 (br, 1 H), 3.80-3.85 (m, 2H), 3.60-3.65 (m, 4H), 3.15-3.20 (m, 4H), 1.85-1.90 (m, 2H), 1.33 (s, 6H). MS (ES): MH⁺ 476.

A suspension of *N*-(4,4-dimethyl-1-trifluoroacetyl-1,2,3,4-tetrahydroquinol-7-yl)-4-(3-methyl-2-pyridyl)-piperazine-1-carboxamide (100 mg, 0.210 mmol) and potassium carbonate (87 mg, 0.631 mmol) in methanol (5 ml) and water (5 ml) was heated at 60°C for 3h. After this period, the solvents were evaporated in *vacuo* and the residue partitioned between DCM (15ml) and water (10ml). The aqueous layer was re-extracted with DCM (2 x 15 ml) and then the combined organic layers dried (Na₂SO₄) and the solvents evaporated *in vacuo* to give the title compound as a white solid. ¹H NMR (400MHz, CDCl₃) δ (ppm): 8.16 (dd, 1 H), 7.43 (dd, 1 H), 7.05 (d, 1 H), 6.88 (dd, 1 H), 6.75 (d, 1 H), 6.44 (dd, 1 H), 6.29 (br, 1 H), 3.85-3.95 (br, 1 H), 3.55-3.65 (m, 4H), 3.25-3.30 (m, 2H), 3.15-3.25 (m, 4H), 2.29 (s, 3H), 1.65-1.70 (m, 2H), 1.26 (s, 6H). MS (ES): MH⁺ 380.

### Example 43

### N-(8-Chloro-4,4-dimethyl-1,2,3,4-tetrahydroisoquinolin-7-yl)-4-(3-chloro-2-pyridyl)-piperazine-1-carboxamide

Using the procedure outlined in Example 42, the title compound was prepared from 7-amino-8-chloro-4,4-dimethyl-1-trifluoroacetyl-1,2,3,4-tetrahydroquinoline (D98) and 4-(3-chloro-2-pyridyl)-piperazine (US Patent number 4,456,604), as a pale yellow oil. MH+ 434/436

### Example 44

### 4-(6-Methyl-2-pyridyl)-N-(1,2,3,4-tetrahydroquinolin-7-yl)benzamide

Using the procedure outlined in Example 38 the title compound was prepared from 7-amino-1-trifluoroacetyl-1,2,3,4-tetrahydroquinoline (D5) (46mg, 0.19mmol) and 4-(6-methyl-2-pyridyl)benzoic acid (D29) (41 mg, 0.19mmol) as a solid. ¹H NMR (250MHz, CDCl₃) δ (ppm): 8.10 (d, 2H), 7.93 (d, 2H), 7.67 (t, 2H), 7.57 (d, 1 H), 7.15 (d, 1 H), 7.11 (d, 1 H), 6.92 (d, 1 H), 6.62 (dd, 1 H), 3.31 (m, 2H), 2.74 (t, 2H), 1.93 (m, 2H)

### Example 45

### 6-(4-Fluorophenyl)-N-(1-methyl-1,2,3,4-tetrahydroquinolin-7-yl)-2-methyl-nicotinamide

To a solution of 7-amino-1-methyl-1,2,3,4-tetrahydroquinoline (D3) (31 mg, 0.19mmol) in DCM (1ml) was added 6-(4-fluorophenyl)-2-methylnicotinic acid (D24) (44mg, 0.19mmol), 1-(3-dimethylaminopropyl)-3-ethylcarbodiimide hydrochloride (44mg, 0.23mmol) and 4-dimethylaminopyridine (11 mg, 0.09mmol) and the reaction stirred at ambient temperature overnight. The crude reaction mixture was loaded directly onto a silica SPE column and eluted with 0-2% methanol/DCM to give the title compound as a solid. ¹H NMR (400MHz, CDCl₃) δ (ppm): 8.02 (dd, 2H), 7.84 (d, 1 H), 7.57 (d, 1 H), 7.35 (br, 1 H), 7.17 (t, 2H), 7.00 (s, 1 H), 6.93 (d, 1 H), 6.74 (d, 1 H), 3.25 (m, 2H), 2.93 (s, 3H), 2.80 (s, 3H), 2.74 (m, 2H), 1.96 (m, 2H)

### Example 46

### N-(3,4-Dihydro-2H-1,4-ethanoquinolin-7-yl)-6-(4-fluorophenyl)-2-methyl-nicotinamide

Using the procedure outlined in Example 45 the title compound was prepared from 3,4-dihydro-2H-1,4-ethanoquinolin-7-ylamine (D30) (35mg, 0.2mmol) and 6-(4-fluorophenyl)-2-methylnicotinic acid (D24) (46mg, 0.2mmol) as a solid. MS (ES): MH⁺ 388, MH- 386.

### Example 47

### (R)-2-Methyl-4-(3-trifluoromethyl-2-pyridyl)-N-(1-methyl-1,2,3,4-tetrahydro-quinolin-7-yl) piperazine-1-carboxamide

To a solution of 7-amino-1-methyl-1,2,3,4-tetrahydroquinoline (D3) (300mg, 1.85mmol) in DCM (5ml) was added pyridine (164ul, 2mmol) followed by phenyl chloroformate (255ul, 2mmol) and the solution stirred at ambient temperature for 50mins. Triethylamine (516µl, 3.7mmol) was then added followed by a solution of (*R*)-2-methyl-4-(3-trifluoromethyl-2-pyridyl)piperazine (D22) (454mg, 1.85mmol) in DCM (5ml) and the reaction stirred at ambient temperature until complete by tlc. The reaction mixture was washed (1 M HCl, brine), dried over MgSO₄ and concentrated *in vacuo* to give the crude product which was purified by flash chromatography, eluting with an EtOAc/40-60°C petroleum ether gradient, to give the title compound as a white solid. ¹H NMR (250MHz, CDCl₃) δ (ppm): 8.47 (dd, 1 H), 7.90 (dd, 1 H), 7.05 (dd, 1 H), 6.84 (d, 1 H), 6.78 (d, 1 H), 6.49 (dd, 1 H), 6.25 (br, 1 H), 4.34 (m, 1 H), 3.86 (m, 1 H), 3.17-3.62 (m, 6H), 3.05 (m, 1 H), 2.89 (s, 3H), 2.70.(t, 2H), 1.96 (m, 2H), 1.36 (d, 3H). MS (ES): MH⁺ 434.

### Example 48

### N-(1-Methyl-1,2,3,4-tetrahydroquinolin-6-yl)-1,1'-biphenyl-4-carboxamide

Using the procedure outlined in Example 25, the title compound was prepared from N-methyl-6-amino-1,2,3,4-tetrahydroquinoline (International Patent Application, Publication number WO 94/14801) (75mg, 0.46mmol) and 4-biphenylcarboxylic acid (140mg, 0.71mmol) as a yellow gum. ¹H NMR (250MHz, CDCl₃) δ (ppm): 8.03 (d, 2H), 7.6-7.8 (m, 5H), 7.4-7.55 (m, 3H), 7.2-7.35 (m, 2H), 6.59 (d, 1 H), 3.21 (m, 2H), 2.89 (s, 3H), 2.79 (m, 2H), 1.99 (m, 2H).

### Example 49

### N-(4,4-Dimethyl-1,2,3,4-tetrahydroisoquinolin-7-yl)-4-(3-trifluoromethyl-2-pyridyl)-piperazine-1-carboxamide

To a stirred solution of 7-amino-4,4-dimethyl-1-trifluoroacetyl-1,2,3,4-tetrahydroisoquinoline (International Patent Application, Publication number WO 00/09486) (100mg, 0.368mmol) and pyridine (33ul, 0.404mmol) in DCM (5ml) was added phenylchloroformate (51 ul, 0.404mmol). The reaction mixture was stirred for 1 h at ambient temperature before triethylamine (56ul, 0.404mmol) was added and then left to stir for a further 30min. After this period, 4-(3-trifluoromethyl-2-pyridyl)-piperazine (85mg, 0.367mmol) in DCM (5ml) was added and the reaction stirred at ambient temperature for 18h. On completion, the solvents were evaporated *in vacuo* and the residue purified directly by chromatography, eluting with 10-100% EtOAc/40-60°C petroleum ether, to give *N*-(4,4-dimethyl-1-trifluoroacetyl-1,2,3,4-tetrahydroisoquinol-7-yl)-4-(3-trifluoromethyl-2-pyridiyl)-piperazine-1-carboxamide as a colourless oil. ¹H NMR (400MHz, CDCl₃) δ (ppm): 8.46 (dd 1 H), 7.91 (dd, 1 H), 7.05-7.35 (m, 4H), 6.39 (br-s, 1 H), 4.77 (s, 2H), 3.60-3.65 (m, 5H), 3.53 (s, 1 H), 3.30-3.35 (m, 4H), 1.20-1.25 (m, 6H). MS (ES): MH⁺ 530.

A suspension of *N*-(4,4-dimethyl-1-trifluoroacetyl-1,2,3,4-tetrahydroisoquinol-7-yl)-4-(3-trifluoromethyl-2-pyridyl)-piperazine-1-carboxamide (30mg, 0.057 mmol) and potassium carbonate (48 mg, 0.348 mmol) in methanol (4 ml) and water (4 ml) was heated at 50°C for 6h. After this period, the solvents were evaporated *in vacuo* and the residue partitioned between DCM (30ml) and water (30ml). The aqueous layer was re-extracted with DCM (2 x 30 ml) and then the combined organic layers dried (Na₂SO₄) and the solvents evaporated in *vacuo* to give the title compound as a white solid. ¹H NMR (400MHz, DMSO) δ (ppm): 8.45 (dd, 1 H), 7.89 (dd, 1 H), 7.22 (d, 1 H), 7.10 (dd, 1 H), 7.00-7.05 (m, 2H), 6.55 (s, 1 H), 3.95 (s, 2H), 3.60-3.65 (m, 4H), 3.30-3.32 (m, 4H), 2.82 (s, 2H), 2.00 (br-s, 1 H), 1.23 (s, 6H). MS (ES): MH⁺ 434.
The following compounds shown in Table 3 were prepared as outlined above:

**Table 3**

| Example | Name | MH⁺ |
|---|---|---|
| 50 | *N*-(4,4-Dimethyl-1,2,3,4-tetrahydroisoquinolin-7-yl)-4-(3-chloro-2-pyridyl)-piperazine-1-carboxamide | 400/402 |
| 51 | *N*-(4,4-Dimethyl-1,2,3,4-tetrahydroisoquinolin-7-yl)-4-(3-chloro-5-trifluoromethyl-2-pyridyl)-piperazine-1-carboxamide | 468/470 |

### Example 52

### N-[4,4-Dimethyl-1,2,3,4-tetrahydroquinolin-7-yl-(3-chloro-pyridin-2-yl)-benzamide

7-Amino-4,4-dimethyl-1-trifluoroacetyl-1,2,3,4-tetrahydroquinoline (D28) (50 mg, 0.18 mmol) was combined with 4-(3-chloro-pyridin-2-yl)-benzoic acid (D84) (39.3 mg, 0.17 mmol), 1-(3-dimethylaminopropyl)-3-ethylcarbodiimide hydrochloride (38.5mg, 0.2mmol) and dimethylaminopyridine (10.2 mg, 0.08 mmol) in DCM (2 ml). The reaction was stirred for 16 h and then diluted with DCM (18 ml). The solution was washed with 10% citric acid (20 ml), saturated NaHCO₃ (20 ml) and brine (20 ml) then dried with Na₂SO₄ and concentrated. The resulting residue was purified by flash chromatography (EtOAc/40-60°C pet.ether) to yield product as a white solid (19.3 mg). ¹H NMR(400MHz, CDCl₃) δ (ppm): 8.61-8.63(dd, 1 H), 8.11 (s, 1 H), 7.94-7.97(d, 2H), 7.74-7.86(m, 5H), 7.37-7.39(1 H, d), 7.26-7.33(1 H, m), 4.08-4.16(2H, m), 1.88-1.93(2H, m), 1.33 (6H, s). MH⁺ 488/490.

*N*-[4,4-Dimethyl-1-trifluoroacetyl-1,2,3,4-tetrahydroquinolin-7-yl-(3-chloro-pyridin-2-yl)-phenylcarboxamide (19.3 mg, 0.04 mmol) and potassium carbonate (16 mg, 0.12 mmol) in water (2 ml) and methanol (2 ml) were heated at 50°C for 3h. The methanol was then evaporated *in vacuo* and the residue diluted with water (10 ml). The mixture was extracted with DCM (4 x 10 ml) and the combined organics were dried with Na₂SO₄ and the solvents evaporated in vacuo to give an off-white solid. This product was then taken up in methanol and 1 M HCl in ether (41 µl) was added. Evaporation of the solvent gave the final product as an off-white solid. ¹H NMR(400MHz, DMSO) δ (ppm): 10.32(1 H, s), 8.67-8.68(1H, m), 8.04-8.11(4H, m), 7.82-7.85(3H, d), 7.33(2H, bs), 3.31 (2H, bs), 1.77(2H, bs), 1.27(6H, s). MS (ES): MH⁺ 392/394.

### Example 53

### 6-(3-Fluorophenyl)-2-methyl-N-(1,2,3,4-tetrahydroquinolin-7-yl)nicotinamide

Using the procedure outlined in Example 38, the title compound was prepared from 7-amino-1-trifluoroacetyl-1,2,3,4-tetrahydroquinoline (D5) (48mg, 0.20mmol) and 2-methyl-6-(3-fluorophenyl)-nicotinic acid (D25) (50mg, 0.22mmol) as an off-white solid. MS(ES): MH⁺ 362, M-H⁺ 360.

### Example 54

### 6-(2,3-Difluorophenyl)-2-methyl-N-(1,2,3,4-tetrahydroquinolin-7-yl)nicotinamide

Using the procedure outlined in Example 38, the title compound was prepared from 7-amino-1-trifluoroacetyl-1,2,3,4-tetrahydroquinoline (D5) (48mg, 0.20mmol) and 2-methyl-6-(2,3-difluorophenyl)-nicotinic acid (D26) (54mg, 0.22mmol) as an off-white solid. MS(ES): MH⁺ 380, M-H⁺ 378.

### Example 55

### N-(5-Chloro-1,2,3,4-tetrahydro-quinolin-7-yl)-6-phenyl-nicotinamide

Using the procedure outlined in Example 38, the title compound was prepared from 7-amino-5-chloro-1-trifluoroacetyl-1,2,3,4-tetrahydroquinoline (D104) (50 mg, 0.251 mmol) and 6-phenyl nicotinic acid (60 mg, 0.302 mmol) as a white solid (55 mg). ¹H NMR (400 MHz, CDCl₃) δ (ppm): 9.11 (s, 1 H), 8.22 (dd, 1 H), 8.05 (dd, 1 H), 7.85 (d, 1 H), 7.65 (br-s, 1 H), 7.45-7.55 (m, 3H), 7.00 (br.s, 1 H), 6.79 (d, 1 H), 4.10 (br-s, 1 H), 3.25-3.30 (m, 2H), 2.75-2.80 (m, 2H), 1.95-2.00 (m, 1 H), 1.57 (s, 6H). MS(ES): MH⁺ 364.

### Example 56

### N-Quinolin-7-yl-1,1'-biphenyl-4-carboxamide

To a solution of 7-aminoquinoline (D55) (100mg, 0.69mmol) in DCM (3ml) was added 4-biphenylcarboxylic acid (206mg, 1.04mmol), 1-(3-dimethylaminopropyl)-3-ethyl-carbodiimide hydrochloride (197mg, 1.04mmol) and 4-dimethylaminopyridine (10mg, 0.08mmol) and the reaction stirred at room temperature overnight. The mixture was diluted with DCM, washed with sat. aqueous sodium bicarbonate solution, dried over MgSO₄ and concentrated *in vacuo* to give the crude product which was purified by SPE column chromatography. Elution with 50% EtOAc in 40-60°C petroleum ether gave the title compound as an off-white solid. ¹H NMR (400MHz, CDCl₃) δ (ppm): 8.91 (dd, 1 H), 8.19 (d, 1 H), 8.12 (m, 2H), 8.02, (d, 2H), 7.86 (d, 1 H), 7.75 (d, 2H), 7.65 (d, 2H), 7.49 (t, 2H), 7.42 (t, 1 H), 7.36 (dd,1 H).

### Example 57

### 6-Phenyl-N-quinolin-7-ylnicotinamide

Using the procedure outlined in Example 56, the title compound was prepared from 7-aminoquinoline (D55) (100mg, 0.69mmol) and 6-phenylnicotinic acid (D48) (198mg, 1mmol) as a solid. ¹H NMR (400MHz, CDCl₃) δ (ppm): 9.23 (d, 1 H), 8.92 (dd, 1 H), 8.33 (dd, 1 H), 8.23 (s, 1 H), 8.15, (d, 1 H), 8.14 (s,1 H), 8.08 (m, 3H), 7.90 (d, 1 H), 7.87 (d, 1 H), 7.50 (m, 3H), 7.38 (dd, 1 H).

### Example 58

### 3'-Methyl-N-quinolin-7-yl-1,1'-biphenyl-4-carboxamide

To a solution of 4-bromo-N-quinolin-7-ylbenzamide (Example 82) (50mg, 0.153mmol) in toluene (2ml) and ethanol (0.4ml) under an argon atmosphere was added 3-methyl-phenylboronic acid (21 mg, 0.153mmol), 2M sodium carbonate solution (0.15ml) and tetrakis(triphenylphosphine)palladium (0) (5mg, 0.05mmol). The reaction was heated at reflux for 18h, then cooled to room temperature and diluted with EtOAc. The mixture was washed with sat. aq. sodium bicarbonate solution and water, dried over MgSO₄ and concentrated to give the crude product which was purified by SPE column chromatography. Elution with 50% EtOAc in 40-60°C petroleum ether gave the title compound as an off-white solid. ¹H NMR (400MHz, CDCl₃) δ (ppm): 8.92 (dd,1 H), 8.19 (d, 1 H), 8.13 (m, 3H), 8.01, (d, 2H), 7.86 (d, 1 H), 7.75 (d, 2H), 7.46 (m, 2H), 7.36 (m, 2H), 7.25 (m, 1 H), 2.46 (s, 3H).

### Example 59

### 2'-Methyl-N-quinolin-7-yl-1,1'-biphenyl-4-carboxamide

Using the procedure outlined in Example 58, the title compound was prepared from 4-bromo-N-quinolin-7-ylbenzamide (Example 82) (50mg, 0.153mmol) and 2-methyl-phenylboronic acid (23mg, 0.168mmol) as a white solid. ¹H NMR (400MHz, CDCl₃) δ (ppm): 8.92 (dd,1 H), 8.19 (d, 1 H), 8.13 (m, 3H), 8.00, (d, 2H), 7.86 (d, 1 H), 7.49 (d, 2H), 7.36 (dd, 1 H), 7.29 (m, 4H), 2.30 (s, 3H).

### Example 60

### 2'-Methoxy-N-quinolin-7-yl-1,1'-biphenyl-4-carboxamide

Using the procedure outlined in Example 58, the title compound was prepared from 4-bromo-N-quinolin-7-ylbenzamide (Example 82) (50mg, 0.153mmol) and 2-methoxy-phenylboronic acid (25mg, 0.168mmol) as a colourless gum. ¹H NMR (400MHz, CDCl₃) δ (ppm): 8.91 (dd,1 H), 8.17 (d, 1 H), 8.14 (m, 3H), 7.98, (d, 2H), 7.86 (d, 1 H), 7.70 (d, 2H), 7.36 (m, 3H), 7.30 (d, 1 H), 7.07 (t, 1H), 3.85 (s, 3H).

### Example 61

### 2',6'-Dimethyl-N-quinolin-7-yl-1,1'-biphenyl-4-carboxamide

Using the procedure outlined in Example 58, the title compound was prepared from 4-bromo-N-quinolin-7-ylbenzamide (Example 82) (50mg, 0.153mmol) and 2,6-dimethyl-phenylboronic acid (25mg, 0.17mmol) as a white solid. ¹H NMR (400MHz, CDCl₃) δ (ppm): 8.92 (dd,1 H), 8.20 (d, 1 H), 8.14 (m, 3H), 8.02, (d, 2H), 7.87 (d, 1 H), 7.37 (dd, 1 H), 7.34 (d, 2H), 7.21 (t, 1 H), 7.14 (d, 2H), 2.05 (s, 6H).

### Example 62

### 2'-Acetyl-N-quinolin-7-yl-1,1'-biphenyl-4-carboxamide

Using the procedure outlined in Example 58, the title compound was prepared from from 4-bromo-N-quinolin-7-ylbenzamide (Example 82) (50mg, 0.153mmol) and 2-acetyl-phenylboronic acid (28mg, 0.17mmol) as an off-white solid. ¹H NMR (400MHz, CDCl₃) δ (ppm): 8.92 (dd,1 H), 8.21 (d, 1 H), 8.20 (s, 1 H), 8.14 (d, 1 H), 8.10 (dd, 1 H), 7.86 (d, 1 H), 7.63 (dd, 1 H), 7.57 (td, 1 H), 7.48 (m, 3H), 7.40 (dd, 1 H), 7.35 (dd, 1 H), 3.85 (s, 3H).

### Example 63

### 5'-Chloro-2'-methoxy-N-quinolin-7-yl-1,1'-biphenyl-4-carboxamide

Using the procedure outlined in Example 58, the title compound was prepared from from 4-bromo-N-quinolin-7-ylbenzamide (Example 82) (65mg, 0.199mmol) and 5-chloro-2-methoxyphenylboronic acid (42mg, 0.22mmol) as a colourless gum. ¹H NMR (400MHz, CDCl₃) δ (ppm): 8.91 (dd,1H), 8.18 (d, 1 H), 8.13 (m, 3H), 7.98, (d, 2H), 7.86 (d, 1 H), 7.66 (d, 2H), 7.36 (dd, 2H), 7.32 (m, 2H), 6.94 (d, 1H), 3.82 (s, 3H).

### Example 64

### 4-(2, 6-Dimethyl-3-pyridyl)-N-quinolin-7-ylbenzamide

Using the procedure outlined in Example 56, the title compound was prepared as the corresponding hydrochloride salt from 7-aminoquinoline (D55) (25mg, 0.17mmol) and 4-(2,6-dimethyl-3-pyridyl)benzoic acid (D56) (21 mg, 0.09mmol) as a brown solid. ¹H NMR (250MHz, DMSO) δ (ppm): 9.15 (d, 1 H), 9.01 (s, 1 H), 8.94 (d, 1 H), 8.28 (m, 5H), 7.84 (m, 2H), 7.72 (d, 2H), 2.79 (s, 3H), 2.70 (s, 3H).

### Example 65

### 3-Methyl-4-(4-pyridyl)-N-quinolin-7-ylbenzamide

Using the procedure outlined in Example 56, the title compound was prepared from 7-aminoquinoline (D55) (25mg, 0.17mmol) and 3-methyl-4-(4-pyridyl)benzoic acid (D57) (44mg, 0.21mmol) as an orange solid. ¹H NMR (400MHz, CDCl₃) δ (ppm): 8.91 (dd, 1 H), 8.70 (d, 2H), 8.22 (m, 2H), 8.14 (dd, 1 H), 8.11 (dd, 1 H), 7.84 (m, 3H), 7.36 (m, 2H), 7.28 (m, 2H), 2.37 (s, 3H).

### Example 66

### 3-Methyl-N-quinolin-7-yl-1,1'biphenyl-4-carboxamide

Using the procedure outlined in Example 56, the title compound was prepared from 7-aminoquinoline (D55) (31, 0.22mmol) and 3-methyl-1,1'-biphenyl-4-carboxylic acid (D58) (55mg, 0.26mmol) as an off-white solid. ¹H NMR (400MHz, CDCl₃) δ (ppm): 8.91 (dd, 1 H), 8.14 (m, 3H), 7.86 (d, 1 H), 7.78 (br, 1 H), 7.66 (d, 1 H), 7.62 (d, 2H), 7.53 (m, 2H), 7.48 (t, 2H), 7.40 (t, 1 H), 7.36 (dd, 1H), 2.63 (s, 3H).

### Example 67

### 3-Methoxy-N-quinolin-7-yl-1,1'biphenyl-4-carboxamide

Using the procedure outlined in Example 56, the title compound was prepared from 7-aminoquinoline (D55) (26mg, 0.18mmol) and 3-methoxy-1,1'-biphenyl-4-carboxylic acid (D59) (50mg, 0.22mmol) as an off-white solid. MS (ES): MH⁺ 355.

### Example 68

### 2-Methyl-N-quinolin-7-yl-1,1'biphenyl-4-carboxamide

Using the procedure outlined in Example 56, the title compound was prepared from 7-aminoquinoline (D55) (30mg, 0.21mmol) and 2-methyl-1,1'-biphenyl-4-carboxylic acid (D60) (53mg, 0.25mmol) as a white solid. MS(ES): MH⁺ 339

### Example 69

### 4-[(4-tert-Butoxycarbonyl)piperazin-1-yl]-2-methyl-N-quinolin-7-ylbenzamide

Using the procedure outlined in Example 56, the title compound was prepared from 7-aminoquinoline (D55) (17mg, 0.12mmol) and 4-[(4-*tert-*butoxycarbonyl)piperazin-1-yl]-2-methylbenzoic acid (D61) (47mg, 0.14mmol) as a yellow oil. MS(ES): MH⁺ 447, M-H⁺ 445.

### Example 70

### 3,5-Dimethyl-4-(4-methyl-benzo[1,3]dioxol-5-yl)-N-quinolin-7-ylbenzamide

Using the procedure outlined in Example 56, the title compound was prepared from 7-aminoquinoline (D55) (17mg, 0.12mmol) and 3,5-dimethyl-4-(4-methyl-benzo[1,3]-dioxol-5-yl)-benzoic acid (D62) (41 mg, 0.15mmol) as a yellow oil. MS(ES): MH⁺ 411, M-H⁺ 409.

### Example 71

### N-(2-Methylquinolin-7-yl)-1,1'-biphenyl-4-carboxamide

Using the procedure outlined in Example 56, the title compound was prepared from from 7-amino-2-methylquinoline (D66) (80mg, 0.51mmol) and 4-biphenylcarboxylic acid (149mg, 0.75mmol) as an off-white solid. ¹H NMR (400MHz, CDCl₃) δ (ppm): 8.07 (m, 3H), 8.01 (m, 3H), 7.81 (d, 1 H), 7.75 (d, 2H), 7.66 (d, 2H), 7.50 (t, 2H), 7.42 (t, 1 H), 7.24 (d, 1 H), 2.75 (s, 3H).

### Example 72

### N-(2-Methylquinolin-7-yl)-6-phenylnicotinamide

Using the procedure outlined in Example 56, the title compound was prepared from 7-amino-2-methylquinoline (D66) (100mg, 0.63mmol) and 6-phenylnicotinic acid (D48) (151 mg, 0.76mmol) as a cream solid. ¹H NMR (400MHz, DMSO) δ (ppm): 10.75 (s, 1 H), 9.25 (d, 1 H), 8.50 (s, 1 H), 8.45 (dd, 1 H), 8.20 (m, 4H), 7.90 (m, 2H), 7.55 (m, 3H), 7.34 (d, 1 H), 2.65 (s, 3H).

### Example 73

### 3-Methyl-4-(4-pyridyl)-N-(2-methylquinolin-7-yl)-benzamide

Using the procedure outlined in Example 56, the title compound was prepared from 7-amino-2-methylquinoline (D66) (30mg, 0.19mmol) and 3-methyl-4-(4-pyridyl)benzoic acid (D57) (49mg, 0.23mmol) as an orange gum, (59mg, 88%). ¹H NMR (250MHz, CDCl₃) δ (ppm): 8.70 (dd, 2H), 8.10 (s, 2H), 8.03 (m, 2H), 7.87 (s, 1 H), 7.80 (d, 2H), 7.35 (d, 1 H), 7.27 (m, 3H), 2.74 (s, 3H), 2.37 (s, 3H).

### Example 74

### N-(2-Methylquinolin-7-yl)-4-(2-pyridyl)benzamide

Using the procedure outlined in Example 56, the title compound was prepared from 7-amino-2-methylquinoline (D66) (33mg, 0.21mmol) and 4-(2-pyridyl)benzoic acid (D67) (50mg, 0.25mmol) as a white solid. MS (ES): MH⁺ 340, M-H⁺ 338.

### Example 75

### N-(2-Methylquinolin-7-yl)-4-(1-pyrazolyl)benzamide

Using the procedure outlined in Example 56, the title compound was prepared from 7-amino-2-methylquinoline (D66) (33mg, 0.21mmol) and 4-(1-pyrazolyl)benzoic acid (47mg, 0.25mmol) as an off-white solid. MS (ES): MH⁺ 329, M-H⁺ 327.

### Example 76

### N-(2-Methylquinolin-7-yl)-4-(6-methyl-2-pyridyl)benzamide

Using the procedure outlined in Example 56, the title compound was prepared from 7-amino-2-methylquinoline (D66) (33mg, 0.21mmol) and 4-(6-methyl-2-pyridyl)benzoic acid (D29) (47mg, 0.25mmol) as an off-white solid. MS (ES): MH⁺ 354, M-H⁺ 352.

### Example 77

### N-(2-Methylquinolin-7-yl)-4-(N-morpholino)benzamide

A mixture of palladium (II) acetate (14mg, 0.06mmol), cesium carbonate (299mg, 0.92mmol) and BINAP (57mg, 0.09mmol) in dioxan (10ml) was sonicated for 0.75h under an argon atmosphere. To the resulting blood red solution was added a mixture of 4-bromo-N-quinolin-7-ylbenzamide (Example 82) (200mg, 0.61mmol) and morpholine (133mg) in dioxane (10ml) and the reaction was heated at 100°C overnight. The resulting solution was concentrated *in vacuo* and the residue partitioned between DCM and water. The aqueous was further extracted with DCM and the combined organics were washed with sat. aq. sodium bicarbonate solution and brine, then dried over MgSO₄ and concentrated *in vacuo* to give the crude product. Purification by flash chromatography eluting with 5%MeOH/EtOAc gave the title compound as a yellow solid. ¹H NMR (400MHz, CDCl₃) δ (ppm): 8.89 (dd, 1 H), 8.22 (dd, 1 H), 8.16 (m, 2H), 8.09 (s, 1 H), 7.88 (d, 2H), 7.85 (d, 1 H), 7.37 (dd, 1 H), 6.96 (d, 2H), 3.89 (m, 4H), 3.31 (m, 4H).

### Example 78

### N-(2-Methylquinolin-7-yl)-4-(N-piperidino)benzamide

A mixture of 4-bromo-N-quinolin-7-ylbenzamide (Example 82) (200mg, 0.61mmol), Pd₂(dba)₃ (8.4mg, 1.5mol%), Xantphos (21 mg, 6mol%), cesium carbonate (298mg, 0.92mmol) and piperidine (78mg, 0.92mmol) in dioxan (10ml) was heated at reflux under an argon atmosphere overnight. The mixture was concentrated *in vacuo* and the residue was partitioned between 9:1 DCM/MeOH and water. The aqueous was further extracted with 9:1 DCM/MeOH and the combined organics were washed with saturated aqueous sodium bicarbonate solution and brine, then dried over MgSO₄ and concentrated *in vacuo* to give the crude product. Purification by flash chromatography eluting with 50%EtOAc/DCM gave the title compound as a yellow solid. ¹H NMR (400MHz, CDCl₃) δ (ppm): 8.87 (dd, 1 H), 8.13 (m, 4H), 7.83 (d, 2H), 7.80 (d, 1 H), 7.32 (dd, 1 H), 6.92 (d, 2H), 3.32 (m, 4H), 1.66 (m, 6H).

### Example 79

### 4-Phenyl-N-quinolin-7-ylpiperazine-1-carboxamide

To a solution of di-*tert*-butyl tricarbonate (60mg, 0.23mmol) in DCM (1 ml) was added in one portion, a solution of 7-aminoquinoline (D55) (30mg, 0.21mmol) in DCM (1ml). After 5mins, when gas evolution was complete, *tris*amine resin (12mg 0.04mmol) was added, then after 1 h a solution of 4-phenylpiperazine (32ul, 0.21mmol) was added and the reaction stirred at room temperature overnight. The reaction mixture was then purified directly by SPE column chromatography, eluting with an EtOAc/60-80°C-petroleum ether gradient, followed by treatment with excess methyl isocyanate resin to remove unreacted 7-aminoquinoline starting material from the product. On completion the resin was removed by filtration and filtrate concentrated *in vacuo* to give the title compound as an orange gum. ¹H NMR (250MHz, CDCl₃) δ (ppm): 8.85 (dd, 1 H), 8.09 (dd, 1 H), 7.93 (dd, 1 H), 7.82 (s, 1 H), 7.76 (d, 1 H), 7.30 (m, 3H), 6.94 (m, 3H), 6.83 (br, 1 H), 3.72 (m, 4H), 3.28 (m, 4H).

### Example 80

### N-(2-Methylquinolin-7-yl)-4-phenylpiperazine-1-carboxamide

Using the procedure outlined in Example 79, the title compound was prepared from 7-amino-2-methylquinoline (D66) (100mg, 0.63mmol) and 4-phenylpiperazine (123µl, 0.76mmol) as an off-white solid. ¹H NMR (400MHz, CDCl₃) δ (ppm): 7.97 (d, 1 H), 7.84 (dd, 1 H), 7.72 (s, 1 H), 7.69 (dd, 1 H), 7.30 (t, 2H), 7.18 (d, 1 H), 6.95 (d, 2H), 6.92 (t, 1 H), 6.70 (br, 1 H), 3.72 (m, 4H), 3.27 (m, 4H), 2.71 (s, 3H).

### Example 81

### 4-Phenyl-N-quinolin-7-yl-piperidine-1-carboxamide

Using the procedure outlined in Example 79, the title compound was prepared from 7-aminoquinoline (D55) (30mg, 0.21mmol) and 4-phenylpiperidine (40mg, 0.25mmol) as an orange gum. ¹H NMR (400MHz, CDCl₃) δ (ppm): 8.84 (dd, 1 H), 8.08 (dd, 1 H), 7.93 (dd, 1 H), 7.78 (s, 1 H), 7.27 (m, 6H), 6.78 (br, 1 H), 4.29 (m, 2H), 3.06 (td, 2H), 2.76 (tt, 1 H), 1.96 (m, 2H), 1.80 (td, 2H).

### Example 82

### 4-Bromo-N-quinolin-7-yl-benzamide

Using the procedure outlined in Example 56, the title compound was prepared from 7-aminoquinoline (D55) (720mg, 5mmol) and 4-bromobenzoic acid (1.51 g, 7.5mmol) as a white solid. ¹H NMR (400MHz, CDCl₃) δ (ppm): 8.91 (dd, 1 H), 8.18 (d, 1 H), 8.14 (dd, 1 H), 8.06, (m, 2H), 7.85 (d, 1 H), 7.81 (d, 2H), 7.67 (d, 2H), 7.37 (dd,1 H).

### Example 83

### 3'-Dimethylsulfamoyl-N-quinolin-7-yl-1,1'-biphenyl-4-carboxamide

Using the procedure outlined in Example 56, the title compound was prepared from 7-aminoquinoline (D55) (18mg, 0.13mmol) and 3'-dimethylsulfamoyl-1,1'-biphenyl-4-carboxylic acid (D68) (45mg, 0.15mmol) as a yellow oil. MS(ES): MH⁺ 432, M-H⁺ 430

### Example 84

### 4-Cyclohexyl-N-quinolin-7-yl-benzamide

Using the procedure outlined in Example 45, the title compound was prepared from 7-aminoquinoline (D55) (30mg, 21mmol) and 4-cyclohexylbenzoic acid (51 mg, 0.25mmol) as a yellow solid. MS(ES): MH⁺ 331, M-H⁺ 329

### Example 85

### 4-tert-Butyl-N-quinolin-7-yl-benzamide

Using the procedure outlined in Example 45, the title compound was prepared from 7-aminoquinoline (D55) (30mg, 21mmol) and 4-*tert*-butylbenzoic acid (45mg, 0.25mmol) as a yellow solid. MS(ES): MH⁺ 305, M-H⁺ 303

### Example 86

### 4-iso-Propyl-N-quinolinyl-benzamide

Using the procedure outlined in Example 45, the title compound was prepared from 7-aminoquinoline (D55) (30mg, 21mmol) and 4-iso-propylbenzoic acid (41 mg, 0.25mmol) as a yellow solid. MS(ES): MH⁺ 291, M-H⁺ 289

### Example 87

### N-Quinolinyl-4-trifluoromethyl-benzamide

Using the procedure outlined in Example 45, the title compound was prepared from 7-aminoquinoline (D55) (30mg, 21mmol) and 4-trifluoromethylbenzoic acid (48mg, 0.25mmol) as a yellow solid. MS(ES): MH⁺ 317, M-H⁺ 315

### Example 88

### 9-Oxo-9H-fluorene-2-carboxylic acid quinolin-7-yl amide

To a solution of 7-aminoquinoline (D55) (35mg, 0.24mmol) in DCM (3ml) was added 9-oxo-9*H*-fluorene-2-carboxylic acid (60mg, 0.27mmol), (3-dimethylamino-propyl)-ethyl-carbodiimide hydrochloride (68mg, 0.36mmol) and 4-dimethylaminopyridine (5mg, 0.04mmol) and the reaction stirred at room temperature then at reflux until complete by tlc. After cooling to room temperature the resultant precipitate was filtered off to give the title compound as an off-white solid. MS(ES): MH⁺ 351, M-H⁺ 349

### Example 89

### 2-Methyl-N-quinolin-7-yl-6-trifluoromethyl-nicotinamide

Using the procedure outlined in Example 45, the title compound was prepared from 7-aminoquinoline (D55) (30mg, 21mmol) and 2-methyl-6-trifluoromethylnicotinic acid (51 mg, 0.25mmol) as a yellow solid. MS(ES): MH⁺ 332, M-H⁺ 330

### Example 90

### 4-(3-Pyridyl)-N-quinolin-7-yl-benzamide

Using the procedure outlined in Example 58, the title compound was prepared from 4-bromo-N-quinolin-7-ylbenzamide (Example 82) (50mg, 0.15mmol) and 3-pyridylboronic acid (20mg, 0.16mmol) as a white solid. ¹H NMR (400MHz, CDCl₃) δ (ppm): 8.89 (d, 1 H), 8.87 (dd, 1 H), 8.65 (m, 1 H), 8.26 (dd, 1 H), 8.17 (dd, 1 H), 8.14 (d, 1 H), 8.09 (d, 2H), 7.96 (m, 1 H), 7.88 (d, 1 H), 7.74 (d, 2H), 7.45 (dd, 1H), 7.38 (dd, 1 H).

### Example 91

### 4-(4-Pyridyl)-N-quinolin-7-yl-benzamide

Using the procedure outlined in Example 58, the title compound was prepared from 4-bromo-N-quinolin-7-ylbenzamide (Example 82) (50mg, 0.15mmol) and 4-pyridylboronic acid (20mg, 0.16mmol) as a white solid. ¹H NMR (400MHz, CDCl₃) δ (ppm): 8.86 (d, 1 H), 8.70 (d, 2H), 8.33 (dd, 1 H), 8.18 (d, 1 H), 8.12 (d, 2H), 8.07 (s, 1 H), 7.88 (d, 1 H), 7.79 (d, 2H), 7.58(d, 2H), 7.38 (dd, 1 H).

### Example 92

### (R)-2-Methyl-4-(3-trifluoromethyl-2-pyridyl)-N-quinolin-7-yl) piperazine-1-carboxamide

Using the procedure outlined in Example 79, the title compound was prepared from 7-aminoquinoline (D55) (60 mg, 0.417 mmol) and (R)-2-methyl-4-(3-trifluoromethyl-2-pyridyl)piperazine (D22) (123 mg, 0.50 mmol) as a colourless oil. ¹H NMR (400 MHz, CDCl₃) δ (ppm): 8.92 (1 H, d), 8.66-8.69 (2H, m), 8.50 (2H, d), 8.27 (1 H, bs), 7.99 (1 H, d), 7.94 (1 H, dd), 7.65-7.68 (1 H, m), 7.09-7.12 (1 H, m), 4.55 (1 H, m), 4.06 (1 H, d), 3.50-3.56 (2H, m), 3.41 (1 H, d, J=), 3.26 (1 H, dd), 3.01-3.08 (1 H, m), 1.40 (3H, d). MS(ES): MH⁺ 416.

### Example 93

### 2-Methoxy-N-quinolin-7-yl-1,1'-biphenyl-4-carboxamide

Using the procedure outlined in Example 58, the title compound was prepared from 4-bromo-3-methoxy-N-quinolin-7-ylbenzamide (D69) (76mg, 0.21mmol) and phenylboronic acid (28mg, 0.23mmol) as a white solid. MS(ES): MH⁺ 355, M-H⁺ 353.

### Example 94

### 6-(4-Methylpiperidin-1-yl)-N-quinolin-7-yl-nicotinamide

6-Chloro-N-quinolinylnicotinamide (D70) (50mg, 0.18mmol), 4-methylpiperidine (25ul, 0.21mmol) and potassium carbonate (73mg, 0.53mmol) in DMF (2ml) were heated at 120°C overnight. Further 4-methylpiperidine (11ul, 0.09mmol) was added and heating continued overnight. On cooling the reaction mixture was diluted with EtOAc and washed with water, then dried over MgSO₄ and concentrated to give the crude product. Purification by SPE column chromatography gave the title compound as a yellow solid. MS(ES): MH⁺ 347, M-H⁺ 345.

### Example 95

### 2-Methyl-N-quinolin-7-yl-6-(2-thienyl)-nicotinamide

Using the procedure outlined in Example 45, the title compound was prepared from 7-aminoquinoline (D55) (7mg, 0.05mmol) and 2-methyl-6-(2-thienyl)-nicotinic acid (10mg, 0.05mmol) as a yellow solid. ¹H NMR (400MHz, MeOH-d₄) δ (ppm): 8.82 (dd, 1 H), 8.58 (s, 1 H), 8.34 (dd, 1 H), 7.85-8.0 (m, 3H), 7.76 (m, 2H), 7.55 (dd, 1 H), 7.48 (dd, 1 H), 7.16 (dd, 1 H), 2.70 (s, 3H).

### Example 96

### 6-Piperidin-1-yl-N-quinolin-7-yl-nicotinamide

Using the procedure outlined in Example 94, the title compound was prepared from 6-chloro-N-quinolinylnicotinamide (D70) (50mg, 0.18mmol) and piperidine (30ul, 0.30mmol) to give the title compound as a yellow solid. MS(ES): MH⁺ 333, M-H⁺ 331.

### Example 97

### 4-(4-Fluorophenyl)-N-quinolin-7-yl piperazine-1-carboxamide

Using the procedure outlined in Example 47, the title compound was prepared from 7-aminoquinoline (D55) (30mg, 0.21mmol) and 4-(4-fluorophenyl)-piperazine (37mg, 0.21mmol) as an off-white solid. MS(ES): MH⁺ 351, M-H⁺ 349

### Example 98

### (R)-2-Methyl-4-(6-methyl-2-pyridyl)-N-quinolin-7-yl)-piperazine-1-carboxamide

Using the procedure outlined in Example 47, the title compound was prepared from 7-aminoquinoline (D55) (30mg, 0.21mmol) and (R)-2-methyl-4-(6-methyl-2-pyridyl)piperazine (D99) (40mg, 0.21mmol) as an off-white solid. ¹H NMR (400MHz, CDCl₃) δ (ppm): 8.85 (dd, 1 H), 8.09 (dd, 1 H), 7.94 (dd, 1 H), 7.80 (d, 1 H), 7.77 (d, 1 H), 7.40 (dd, 1 H), 7.30 (dd, 1 H), 6.69 (s, 1 H), 6.52 (d, 1 H), 6.44 (d, 1 H), 4.41 (m, 1 H), 4.25 (m, 1 H), 4.06 (m, 1 H), 4.00 (m, 1 H), 3.49 (ddd, 1 H), 3.38 (dd, 1 H), 3.11 (ddd, 1 H), 2.42 (s, 3H), 1.37 (d, 3H).

### Example 99

### 6-(4-Fluorophenyl)-N-quinolin-7-yl-nicotinamide

Using the procedure outlined in Example 56, the title compound was prepared from 7-aminoquinoline (D55) (50mg, 0.35mmol) and 6-(4-fluoro-phenylnicotinic acid (D24) (83mg, 0.38mmol) as a cream solid. ¹H NMR (250MHz, CDCl₃) δ(ppm): 9.23 (dd, 1 H), 8.86 (dd, 1 H), 8.37 (dd, 1 H), 8.26 (dd, 1 H), 8.18 (m, 2H), 8.08 (d, 1 H), 8.06 (d, 1 H), 7.85 (dd, 2H), 7.38 (dd, 1 H), 7.20 (t, 2H).

### Example 100

### N-Quinolin-7-yl-6-(4-trifluoromethylphenyl)-nicotinamide

To a solution of 6-chloro-N-quinolin-7-yl-nicotinamide (D70) (40mg, 0.14mmol) in DME (0.9ml) under an argon atmosphere was added 4-trifluoromethylphenylboronic acid (33mg, 0.17mmol), 2M sodium carbonate solution (0.17ml) and tetrakis(triphenylphosphine)palladium (0) (8mg, 0.007mmol). The reaction was heated at reflux until complete by tlc, then cooled to room temperature and diluted with EtOAc and dried over MgSO₄. The solvent was removed *in vacuo* and the resultant crude product was purified by SPE column chromatography. Elution with 75% EtOAc in 40-60°C petroleum ether gave the title compound as an off-white solid. ¹H NMR (250MHz, CDCl₃) δ (ppm): 9.31 (dd, 1 H), 8.83 (dd, 1 H), 8.46 (dd, 1 H), 8.39 (dd, 1 H), 8.20 (m, 3H), 8.07 (d, 1 H), 7.94 (dd, 1 H), 7.89 (d, 1 H), 7.79 (d, 2H), 7.40 (dd, 1 H).

### Example 101

### 9H-Fluorene-2-carboxylic acid quinolin-7-yl amide

Using the procedure outlined in Example 56, the title compound was prepared from 7-aminoquinoline (D55) (50mg, 0.35mmol) and 9H-fluorene-2-carboxylic acid (D71) (83mg, 0.38mmol) as an off-white solid. MS(ES): MH⁺ 337, M-H⁺ 335.

### Example 102

### 6-(4-Chlorophenyl)-N-quinolin-7-yl-nicotinamide

Using the procedure outlined in Example 100, the title compound was prepared from 6-chloro-N-quinolin-7-yl-nicotinamide (D70) (40mg, 0.14mmol) and 4-chlorophenylboronic acid (27mg, 0.17mmol) as a pale yellow solid. ¹H NMR (250MHz, CDCl₃) δ (ppm): 9.25 (dd, 1 H), 8.82 (dd, 1 H), 8.43 (dd, 1 H), 8.33 (dd, 1 H), 8.22 (br.d, 1 H), 8.11 (d, 1 H), 8.00 (d, 2H), 7.89 (d, 1 H), 7.87 (d, 1 H), 7.51 (d, 2H), 7.40 (dd, 1 H).

### Example 103

### 6-(3,4-Difluorophenyl)-N-quinolin-7-yl-nicotinamide

Using the procedure outlined in Example 100, the title compound was prepared from 6-chloro-N-quinolin-7-yl-nicotinamide (D70) (40mg, 0.14mmol) and 3,4-difluorophenylboronic acid (27mg, 0.17mmol) as a cream solid. ¹H NMR (250MHz, CDCl₃) δ (ppm): 9.24 (dd, 1 H), 8.86 (dd, 1 H), 8.39 (dd, 1 H), 8.28 (dd, 1 H), 8.14 (br.d, 1 H), 8.13 (d, 1 H), 7.97 (ddd, 1 H), 7.85 (m, 3H), 7.39 (dd, 1 H), 7.28 (m, 1 H).

### Example 104

### 4-(2-Methylpyrid-4-yl)-N-quinolin-7-yl-benzamide

To a solution of 7-aminoquinoline (D55) (338mg, 0.26mmol) in DCM (3ml) was added 4-(2-methylpyrid-4-yl)-benzoic acid (D72) (62mg, 0.29mmol), 1-(3-dimethylamino-propyl)-3-ethyl-carbodiimide hydrochloride (74mg, 0.39mmol) and 4-dimethylaminopyridine (6mg, 0.05mmol) and the reaction stirred at room temperature until complete by tlc. The resultant precipitate was filtered off to give the title compound as an off-white solid. MS(ES): MH⁺ 340, M-H⁺ 338

### Example 105

### 6-(3-Fluorophenyl)-N-quinolin-7-yl-nicotinamide

Using the procedure outlined in Example 104, the title compound was prepared from 7-aminoquinoline (D55) (28mg, 0.19mmol) and 6-(3-fluorophenyl)-nicotinic acid (D73) (50mg, 0.23mmol) as a yellow solid. MS(ES): MH⁺ 344, M-H⁺ 342

### Example 106

### 6-(2-Fluorophenyl)-N-quinolin-7-yl-nicotinamide

Using the procedure outlined in Example 104, the title compound was prepared from 7-aminoquinoline (D55) (28mg, 0.19mmol) and 6-(2-fluorophenyl)-nicotinic acid (D74) (50mg, 0.23mmol) as a yellow solid. MS(ES): MH⁺ 344, M-H⁺ 342

### Example 107

### N-Quinolin-7-yl-1-(5-trifluoromethylpyrid-2-yl)-piperidine-4-carboxamide

1-(5-Trifluoromethylpyrid-2-yl)-piperidine-4-carboxylic acid (D101) (100mg, 0.36mmol) was treated with oxalyl chloride (63ul, 0.72mmol) and catalytic DMF in 1,2-dichloroethane (3.5ml) at 60°C for 0.75h. On cooling to room temperature the solvent was removed *in vacuo* and the residue was dissolved in DCM (2ml). Triethylamine (30ul, 0.2mmol) and 7-aminoquinoline (D55) (27mg, 0.19mmol) were added and the reaction stirred at room temperature until complete by tlc. The resultant precipitate was collected by filtration to give the title compound as an off-white solid. MS(ES): MH⁺ 401, M-H⁺ 399.

### Example 108

### 2-Methyl-6-phenyl-N-quinolin-7-yl-nicotinamide

Using the procedure outlined in Example 56, the title compound was prepared from 7-aminoquinoline (D55) (28mg, 0.19mmol) and 2-methyl-6-phenylnicotinic acid (D23) (50mg, 0.24mmol) as a yellow solid. MS(ES): MH⁺ 340, M-H⁺ 338

### Example 109

### 6-(4-Fluorophenyl)-2-methyl-N-quinolin-7-yl-nicotinamide

Using the procedure outlined in Example 45, the title compound was prepared from 7-aminoquinoline (D55) (26mg, 0.18mmol) and 6-(4-fluorophenyl)-2-methylnicotinic acid (D24) (50mg, 0.22mmol) as a white solid. MS(ES): MH⁺ 358, M-H⁺ 356.

### Example 110

### N-Quinolin-7-yl-1-(6-trifluoromethylpyrid-2-yl)-piperidine-4-carboxamide

Using the procedure outlined in Example 107, the title compound was prepared from 1-(6-trifluoromethylpyrid-2-yl)-piperidine-4-carboxylic acid (D102) (100mg, 0.36mmol) and 7-aminoquinoline (D55) (27mg, 0.19mmol) as an off white solid. MS(ES): MH⁺ 401, M-H⁺ 399.

### Example 111

### 4'-Fluoro-2-(2-methoxyethoxy)-N-quinolin-7-yl-1,1'-biphenyl-4-carboxamide

Using the procedure outlined in Example 56, the title compound was prepared from 7-aminoquinoline (D55) (21 mg, 0.14mmol) and 4'-fluoro-2-(2-methoxyethoxy)-1,1'-biphenyl-4-carboxylic acid (D77) (50mg, 0.17mmol) as an orange solid. MS(ES): MH⁺ 417, M-H⁺ 415

### Example 112

### 2-(2-Dimethylaminoethoxy)- 4'-fluoro-N-quinolin-7-yl-1,1'-biphenyl-4-carboxamide

Using the procedure outlined in Example 56, the title compound was prepared from 7-aminoquinoline (D55) (20mg, 0.14mmol) and 2-(2-dimethylaminoethoxy)- 4'-fluoro-1,1'-biphenyl-4-carboxylic acid (D79) (50mg, 0.17mmol) as a yellow solid. MS(ES): MH⁺ 430, M-H⁺ 428

### Example 113

### N-(5-Chloroquinolin-7-yl)-6-(4-Fluorophenyl)-2-methyl-nicotinamide

To a solution of 7-amino-5-chloroquinoline (D83) (50mg, 0.28mmol) in DCM (3ml) was added 6-(4-fluorophenyl)-2-methyl-nicotinic acid (D24) (30mg, 0.13mmol), 1-(3-dimethylaminopropyl)-3-ethyl-carbodiimide hydrochloride (59mg, 0.31mmol) and 4-dimethylaminopyridine (17mg, 0.14mmol) and the reaction stirred at room temperature then at reflux until complete by tlc. The mixture was washed with sat. aq. sodium bicarbonate solution then dried over MgSO₄ and concentrated to give the crude product. Purification by SPE column chromatography gave the title compound as an off white solid. ¹H NMR (400MHz, CDCl₃) δ (ppm): 8.94 (dd, 1 H), 8.54 (d, 1 H), 8.29 (s, 1 H), 8.05 (m, 3H), 7.92 (d, 1 H), 7.78 (br.s, 1 H), 7.62 (d, 1 H), 7.47 (dd, 1 H), 7.18 (t, 2H), 2.84 (s, 3H).

### Example 114

### 4-(3-Chloro-2-pyridinyl)-N-quinolin-7-yl-benzamide

Using the procedure outlined in Example 56, the title compound was prepared from 7-aminoquinoline (D55) (25mg, 0.14mmol) and 4-(3-Chloro-2-pyridyl)-benzoic acid (D84) (50mg, 0.17mmol) as a brown solid. MS(ES): MH⁺ 362/360, M-H⁺ 360/358.

### Example 115

### 6-(4-Fluorophenyl)-2-(methoxymethyl)-N-quinolin-7-yl-nicotinamide

Using the procedure outlined in Example 56, the title compound was prepared from 7-aminoquinoline (D55) (11 mg, 0.076mmol) and 6-(4-fluorophenyl)-2-(methoxymethyl)-nicotinic acid (D86) (19mg, 0.073mmol) as an orange solid. ¹H NMR (250MHz, CDCl₃) δ (ppm): 10.40 (br.s, 1 H), 8.92 (dd, 1 H), 8.41 (d, 1 H), 8.29 (d, 1 H), 8.05-8.20 (m, 4H), 7.85 (m, 2H), 7.36 (dd, 1 H), 7.20 (t, 2H), 4.91 (s, 2H), 3.68 (s, 3H).

### Example 116

### 6-(4-Fluorophenyl)-2-methyl-N-(2-methylquinolin-7-yl)-nicotinamide

Using the procedure outlined in Example 56, the title compound was prepared from 7-amino-2-methylquinoline (D66) (20mg, 0.13mmol) and 6-(4-fluorophenyl)-2-methylnicotinic acid (D24) (30mg, 0.13mmol) then converted to the HCl salt as a beige solid by treatment with ethereal HCl. ¹H NMR (400MHz, DMSO) (HCl salt) δ (ppm): 11.39 (br.s, 1 H), 9.04 (s, 1 H), 8.96 (d, 1 H), 8.30 (d, 1 H), 8.23 (dd, 2H), 8.12 (d, 1 H), 8.00 (m, 2H), 7.84 (d, 1 H), 7.37 (t, 2H), 2.94 (s, 3H), 2.71 (s, 3H)

### Example 117

### 6-(3-Fluorophenyl)-2-methyl-N-quinolin-7-yl-nicotinamide

Using the procedure outlined in Example 56, the title compound was prepared from 7-aminoquinoline (D55) (28mg, 0.20mmol) and 6-(3-fluorophenyl)-2-methyl-nicotinic acid (D25) (50mg, 0.33mmol), as an off-white solid. MS(ES): MH⁺ 358, M-H⁺ 356.

### Example 118

### 6-(2,3-Difluorophenyl)-2-methyl-N-quinolin-7-yl-nicotinamide

Using the procedure outlined in Example 56, the title compound was prepared from 7-aminoquinoline (D55) (28mg, 0.20mmol) and 6-(2,3-difluorophenyl)-2-methyl-nicotinic acid (D26) (54mg, 0.33mmol), as a brown solid. MS(ES): MH⁺ 376, M-H⁺ 374.

### Example 119

### 4-(2-Methylthiazol-4yl)-N-quinolin-7-yl-benzamide

Using the procedure outlined in Example 56, the title compound was prepared from 7-aminoquinoline (D55) (27mg, 0.19mmol) and 4-(2-methylthiazol-4-yl)-benzoic acid (D87) (50mg, 0.23mmol) as a solid. MS(ES): MH⁺ 346, M-H⁺ 344

### Example 120

### N-(4-Methyl-2-oxo-1,2-dihydro-quinolin-7-yl)-1,1'-biphenyl-4-carboxamide

Using the procedure outlined in Example 56, the title compound was prepared from 7-amino-4-methyl-1-*H*-quinolin-2-one (50mg, 0.29mmol) and 4-biphenylcarboxylic acid (68mg, 0.34mmol) as a cream solid. ¹H NMR (250MHz, DMSO) δ (ppm): 11.60 (br, 1 H), 10.55 (br, 1 H), 8.09 (d, 2H), 8.02 (s, 1 H), 7.86 (d, 2H), 7.77 (d, 2H), 7.69 (d, 1 H), 7.55 (dd, 1 H), 7.52 (t, 2H), 7.43 (t, 1 H), 6.29 (s, 1H), 2.41 (s, 3H).

### Example 121

### N-(1,4-Dimethyl-2-oxo-1,2-dihydro-quinolin-7-yl)-1,1'-biphenyl-4-carboxamide

Using the procedure outlined in Example 56, the title compound was prepared from 7-amino-1,4-methyl-1*H*-quinolin-2-one (D89) (64mg, 0.34mmol) and 4-biphenylcarboxylic acid (82mg, 0.41mmol) as a pale pink solid. ¹H NMR (250MHz, DMSO) δ (ppm): 10.58 (br, 1 H), 8.14 (s, 1 H), 8.12 (d, 2H), 7.88 (d, 2H), 7.80 (m, 4H), 7.53 (t, 2H), 7.42 (t, 1 H), 6.44 (d, 1 H), 3.60 (s, 3H), 2.43 (d, 3H).

### Example 122

### N-(2-Oxo-1,2-dihydro-quinolin-7-yl)-1,1'-biphenyl-4-carboxamide

Using the procedure outlined in Example 56, the title compound was prepared from 7-amino-1*H*-quinolin-2-one (D89) (30mg, 0.19mmol) and 4-biphenylcarboxylic acid (44mg, 0.22mmol) as an off-white solid. ¹H NMR (400MHz, DMSO) δ (ppm): 11.80 (br, 1 H), 10.58 (br, 1 H), 8.09 (d, 2H), 8.04 (d, 1 H), 7.85 (m, 3H), 7.77 (d, 2H), 7.62 (d, 1 H), 7.52 (m, 3H), 7.44 (t, 1 H), 6.39 (d, 1 H).

### Example 123

### N-(2-Oxo-1,2-dihydro-quinolin-7-yl)-6-phenylnicotinamide

Using the procedure outlined in Example 56, the title compound was prepared from 7-amino-1*H*-quinolin-2-one (D89) (30mg, 0.19mmol) and 6-phenylnicotinic acid (D48) (45mg, 0.22mmol) as an off-white solid. ¹H NMR (400MHz, DMSO) δ (ppm): 11.80 (br, 1 H), 10.73 (br, 1 H), 9.21 (d, 1 H), 8.42 (dd, 1 H), 8.18 (m, 3H), 8.01 (d, 1 H), 7.84 (d, 1 H), 7.64 (d, 1 H), 7.53 (m, 4H), 6.40 (dd, 1 H).

### Example 124

### N-(Isoquinolin-5-yl)-1,1'-biphenyl-4-carboxamide

To a solution of 5-aminoisoquinoline (72mg, 0.5mmol) in DCM (3ml) was added 4-biphenylcarboxylic acid (149mg, 0.75mmol), 1-(3-dimethylaminopropyl)-3-ethyl-carbodiimide hydrochloride (143mg, 0.75mmol) and 4-dimethylaminopyridine (9mg, 0.08mmol) and the reaction stirred at room temperature overnight. The mixture was diluted with DCM, washed with 2M sodium hydroxide solution and 2M hydrochloric acid causing precipitation of a white solid which was filtered off and dried *in vacuo* to give the HCl salt of the title compound. ¹H NMR (400MHz, DMSO) δ (ppm): 10.94 (s, 1 H), 9.88 (s, 1 H), 8.67 (d, 1 H), 8.41 (d, 1 H), 8.38 (d, 1 H), 8.24 (m, 3H), 8.04 (t, 1 H), 7.91 (d, 2H), 7.80 (d, 2H), 7.54 (t, 2H), 7.45 (t, 1 H), 4.00 (br).

### Example 125

### N-(1-Methylisoquinolin-5-yl)-1,1'-biphenyl-4-carboxamide

To a solution of 1-methyl-5-aminoisoquinoline (94 (75mg, 0.47mmol) in DCM (4ml) was added 4-biphenylcarboxylic acid (141 mg, 0.71 mmol), 1-(3-dimethylaminopropyl)-3-ethyl-carbodiimide hydrochloride (135mg, 0.71 mmol) and 4-dimethylaminopyridine (10mg, 0.08mmol) and the reaction stirred at 38°C for 3 days. The mixture was diluted with DCM, washed with sat. aqueous sodium bicarbonate solution and water, dried over MgSO₄ and concentrated *in vacuo* to give the crude product which was triturated with methanol. The resulting precipitate was collected by filtration, washed with ether and dried *in vacuo* giving the title compound as an off-white solid. ¹H NMR (400MHz, CDCl₃) δ (ppm): 8.48 (d, 1 H), 8.33 (d, 1 H), 8.21 (br, 1 H), 8.06 (m, 3H), 7.78 (d, 2H), 7.68 (m, 3H), 7.58 (d, 1 H), 7.51 (t, 2H), 7.41 (t, 1 H), 3.02 (s, 3H).

### Example 126

### N-(Isoquinolin-5-yl)-3'-methyl-1,1'-biphenyl-4-carboxamide

To a solution of 4-bromo-N-isoquinolin-5-ylbenzamide (D94) (50mg, 0.153mmol) in toluene (2ml) and ethanol (0.4ml) under an argon atmosphere was added 3-methyl-phenylboronic acid (21 mg, 0.153mmol), 2M sodium carbonate solution (0.15ml) and tetrakis(triphenylphosphine)palladium (0) (5mg, 0.05mmol). The reaction was heated at reflux for 18h, then cooled to room temperature and diluted with EtOAc. The mixture was washed with sat. aq. sodium bicarbonate solution and water, dried over MgSO₄ and concentrated to give the crude product which was purified by SPE column chromatography. Elution with 50% EtOAc/40-60°C petroleum ether gave the title compound as an off-white solid. ¹H NMR (400MHz, CDCl₃) δ (ppm): 9.32 (s, 1H), 8.62 (d, 1 H), 8.34 (d, 1 H), 8.22 (br, 1 H), 8.07 (d, 2H), 7.90, (d, 1 H), 7.78 (d, 2H), 7.70 (m, 3H), 7.47 (m, 2H), 7.40 (t, 1 H), 2.47 (s, 3H).

### Example 127

### N-(Isoquinolin-8-yl)-1,1'-biphenyl-4-carboxamide

Using the procedure outlined in Example 56, the title compound was prepared from 8-aminoisoquinoline (D95) (85mg, 0.59mmol) and 4-biphenylcarboxylic acid (177mg, 0.89mmol) as an off-white solid. ¹H NMR (400MHz, CDCl₃) δ (ppm): 9.48 (s, 1 H), 8.61 (d, 1 H), 8.42 (br.s, 1 H), 8.22 (d, 1 H), 8.10 (d, 2H), 7.79 (d, 2H), 7.68-7.76 (m, 5H), 7.51 (t, 2H), 7.43 (t, 1 H).

### Example 128

### N-(Isoquinolin-7-yl)-1,1'-biphenyl-4-carboxamide

To a solution of 7-aminoisoquinoline (D96) (88mg, 0.61 mmol) in DCM (4ml) was added 4-biphenylcarboxylic acid (145mg, 0.73mmol) and 1-(3-dimethylaminopropyl)-3-ethylcarbodiimide hydrochloride (140mg, 0.73mmol) and the reaction stirred at ambient temperature overnight. The reaction mixture was filtered to give the title compound as a white solid. The filtrate was diluted with DCM, washed with 2M sodium hydroxide solution, dried over MgSO₄ and concentrated *in vacuo* to give further crude product which was purified by silica SPE chromatography. Elution with an EtOAc/60-80°C petroleum ether gradient gave a further title compound which was combined with the sample obtained from the filtration. This was dissolved in ethanol and treated with ethereal HCl and the resultant precipitate was collected to give the HCl salt of the title compound as a white solid. ¹H NMR (250MHz, DMSO) δ (ppm): 9.80 (s, 1 H), 9.09 (d, 1 H), 8.58 (d, 1 H), 8.40 (dd, 1 H), 8.33 (d, 1 H), 8.28 (d, 1 H), 8.16 (d, 2H), 7.90 (d, 2H), 7.79 (d, 2H), 7.54 (t, 2H), 7.45 (t, 1 H)

### Example 129

### N-(Isoquinolin-6-yl)-1,1'-biphenyl-4-carboxamide

Using the procedure outlined in Example 56, the title compound was prepared from 6-aminoisoquinoline (D97) (37mg, 0.25mmol) and 4-biphenylcarboxylic acid (75mg, 0.38mmol) as an off-white solid. ¹H NMR (400MHz, CDCl₃) δ (ppm): 9.20 (s, 1 H), 8.52 (d, 1 H), 8.46 (d, 1 H), 8.09 (br.s, 1 H), 8.00 (m, 3H), 7.77 (d, 2H), 7.65-7.70 (m, 4H), 7.50 (t, 2H), 7.43 (t, 1 H).

### Example 130

### N-Isoquinolin-5-yl-1-(5-trifluoromethylpyrid-2-yl)-piperidine-4-carboxamide

Using the procedure outlined in Example 107, the title compound was prepared from 1-(5-trifluoromethylpyrid-2-yl)-piperidine-4-carboxylic acid (D101) (100mg, 0.36mmol) and 5-aminoisoquinoline (27mg, 0.19mmol) as an off white solid. MS(ES): MH⁺ 401, M-H⁺ 399.

### Example 131

### 6-(2,4-Difluorophenyl)-2-methyl-N-(1,2,3,4-tetrahydroquinolin-7-yl)nicotinamide.

Using the procedure outlined in Example 38, the title compound was prepared from 7-amino-1-trifluoroacetyl-1,2,3,4-tetrahydroquinoline (D5) (73mg, 0.30mmol) and 2-methyl-6-(2,4-difluorophenyl)nicotinic acid (D106) (82mg, 0.33mmol) then converted to the HCl salt as an off-white solid. ¹H NMR (400MHz, MeOH-d₄) δ (ppm): 8.46 (d, 1 H), 8.11 (d, 1 H), 7.93-8.01 (m, 2H), 7.81 (d, 1 H), 7.40 (d, 1 H), 7.25 (m, 2H), 3.55 (m, 2H), 2.96 (t, 2H), 2.88 (s, 3H), 2.18 (m, 2H).

### Example 132

### 6-(3,4-Difluorophenyl)-2-methyl-N-(1,2,3,4-tetrahydroquinolin-7-yl)nicotinamide.

Using the procedure outlined in Example 38, the title compound was prepared from 7-amino-1-trifluoroacetyl-1,2,3,4-tetrahydroquinoline (D5) (73mg, 0.30mmol) and 2-methyl-6-(3,4-difluorophenyl)nicotinic acid (D108) (82mg, 0.33mmol) then converted to the HCl salt as a buff solid. ¹H NMR (400MHz, DMSO) δ (ppm): 8.23 (dd, 1 H), 8.04 (m, 3H), 7.87 (d, 1 H), 7.63 (d, 1 H), 7.57 (m, 1 H), 7.29 (d, 1 H), 3.34 (m, 2H), 2.80 (m, 2H), 2.67 (s, 3H), 2.02 (m, 2H).

### Example 133

### N-Quinolin-6-yl-1,1'-biphenyl-4-carboxamide

Using the procedure outlined in Example 56, the title compoudn was prepared from 6-aminoquinoline (72mg, 0.75mmol) and 4-biphenylcarboxylic acid (149mg, 0.75mmol) as a white solid. ¹H NMR (400MHz, CDCl₃) δ (ppm): 9.22 (d, 1 H), 8.88 (dd, 1 H), 8.52 (d, 1 H), 8.33 (dd, 1 H), 8.19 (d, 1 H), 8.13 (d, 1 H), 8.09 (d, 1 H), 8.08 (m, 3H), 7.91 (d, 1 H), 7.72 (dd, 1 H), 7.52 (m, 3H), 7.43 (dd, 1 H).

### Pharmacological Data

### (a) In vitro assay

As referenced above, the compounds of the invention are vanilloid receptor (VR1) antagonists and hence have useful pharmaceutical properties. Vanilloid receptor (VR1) antagonist activity can be confirmed and demonstrated for any particular compound by use of conventional methods, for example those disclosed in standard reference texts such as D. Le Bars, M. Gozarin and S. W. Cadden, Pharmacological Reviews, 2001, 53(4), 597-652] or such other texts mentioned herein.

The screen used for the compounds of this invention was based upon a FLIPR based calcium assay, similar to that described by Smart et al. (British Journal of Pharmacology, 2000, 129, 227-230). Transfected astrocytoma 1321 N1 cells, stably expressing human VR1, were seeded into FLIPR plates at 25,000cells/well (96-well plate) and cultured overnight.

The cells were subsequently loaded in medium containing 4µM Fluo-3 AM (Molecular Probes) for 2 hours, at room temperature, in the dark. The plates were then washed 4 times with Tyrode containing 1.5mM calcium, without probenecid.The cells were pre-incubated with compound or buffer control at room temperature for 30 minutes. Capsaicin (Sigma) was then added to the cells. Compounds having antagonist activity against the human VR1 were identified by detecting differences in fluorescence when measured after capsaicin addition, compared with no compound buffer controls. Thus, for example, in the buffer control capsaicin addition results in an increase in intracellular calcium concentration resulting in fluorescence. A compound having antagonist activity blocks the capsaicin binding to the receptor, there is no signalling and therefore no increase in intracellular calcium levels and consequently lower fluorescence. pKb values are generated from the IC₅₀ values using the Cheng-Prusoff equation.
All compounds tested by the above methodology had pKb > 6, preferred compounds having a pKb > 7.0.

### (b) FCA-induced hyperalgesia in the Guinea pig

100µl of 1mg/ml FCA was injected intraplantar into the left paw of 4 groups of 8 male Dunkin Hartley guinea-pigs (batch: 6282434, average weight 340g). 24 hours later compounds were administered orally at 0 (vehicle), 3, 10 30mg/kg with vehicle as 1 %methylcellulose and dosing volume being 2ml/kg and dosing straight into the stomach. The methylcellulose was added gradually to the compound into the pestle and mortar and ground together.

Behavioural readouts of mechanical hyperalgesia were obtained before FCA administration (naïve reading), after FCA but before drug administration (predose reading) and 1 hour after drug administration. The readout used was paw pressure (Randall-Sellito) and the end point was paw withdrawal. The paw pressure equipment also had one silver disc placed on the point to increase the markings by a factor of 2.
Compounds having a pKb > 7.0 *in vitro,* according to model (a) above, were tested in this model and shown to be active.

## Claims

1. A compound of formula (I), 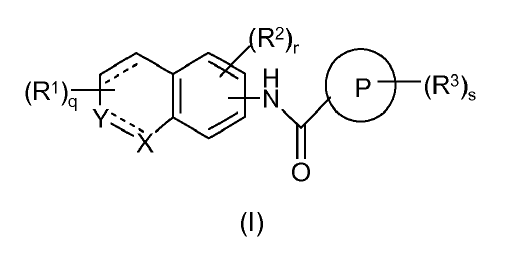 or a pharmaceutically acceptable salt or solvate thereof, wherein, P is selected from phenyl, heteroaryl or heterocyclyl;
R¹ and R² are independently selected from halo, alkyl, alkoxy, cycloalkyl, aralkyl, aralkoxy, cycloalkylalkyl, cycloalkylalkoxy, -CN, -NO₂, -OH, =O, -OCF₃, -CF₃, - NR⁴R⁵, -S(O)ₘR⁶, -S(O)₂NR⁴R⁵, -OS(O)₂R⁶, -OS(O)₂CF₃, -O(CH₂)ₙNR⁴R⁵, -C(O)CF₃, -C(O)alkyl, -C(O)cycloalkyl, -C(O)aralkyl, -C(O)Ar, -C(O)(CH₂)ₙOR⁶, - C(O)(CH₂)ₙNR⁴R⁵, -C(O)alkoxy, -C(O)NR⁴R⁵, -(CH₂)ₙC(O)alkoxy, - (CH₂)ₙOC(O)R⁶, -O(CH₂)ₙOR⁶, -(CH₂)ₙOR⁶, -(CH₂)ₙR⁴R⁵, - (CH₂)ₙC(O)NR⁴R⁵, -(CH₂)ₙN(R⁴)C(O)R⁶, -(CH₂)ₙS(O)₂NR⁴R⁵, - (CH₂)ₙN(R⁴)S(O)₂R⁶, -ZAr, -(CH₂)ₙS(O)₂R⁶, -(OCH₂)ₙS(O)₂R⁶, - N(R⁴)S(O)₂R⁶, -N(R⁴)C(O)R⁶, -(CH₂)ₙN(R⁴)S(O)₂R⁶, -(CH₂)ₙN(R⁴)C(O)R⁶ or -(CH₂)ₙC(O)alkyl;
R³ is selected from alkyl, alkoxy, -CF₃, halo, -O(CH₂)ₙOR⁶, -O(CH₂)ₙNR⁴R⁵, phenyl, cyclohexyl, benzo[1,3]dioxolyl, morpholinyl, pyridyl, pyrimidinyl, pyrazinyl, piperazinyl, piperidinyl, pyridizinyl, thienyl, furyl, pyrazolyl, pyrrolyl, triazolyl, indanyl, imidazolyl, oxazolyl, thiazolyl, oxadiazolyl, isothiazolyl, isoxazolyl or thiadiazolyl; wherein said alkyl, alkoxy, phenyl, cyclohexyl, benzo[1,3]dioxolyl, morpholinyl, pyridyl, pyrimidinyl, pyrazinyl, piperazinyl, piperidinyl, pyridizinyl, thienyl, furyl, pyrazolyl, pyrrolyl, triazolyl, indanyl, imidazolyl, oxazolyl, thiazolyl, oxadiazolyl, isothiazolyl, isoxazolyl and thiadiazolyl groups may be optionally substituted by one or more groups, which may be the same or different, selected from R²;
R⁴ and R⁵ may be the same or different and represent -H or alkyl or R⁴ and R⁵ together with the nitrogen atom to which they are attached form a heterocyclic ring;
R⁶ is -H, alkyl or aryl;
R⁷ is -H, alkyl or aryl;
R⁸ is selected from -H, alkyl, hydroxyalkyl, cycloalkyl, aralkyl, alkoxyalkyl, cycloalkylalkyl, heterocyclylalkyl, -S(O)ₘR⁶, -C(O)CF₃, -C(O)alkyl, - C(O)cycloalkyl, -C(O)aralkyl, -C(O)Ar, -C(O)(CH₂)ₙOR⁶, -C(O)(CH₂)ₙNR⁴R⁵, - C(O)alkoxy, -C(O)NR⁴R⁵, -(CH₂)ₙC(O)alkoxy, -(CH₂)ₙOC(O)R⁶, -(CH₂)ₙOR⁶, - (CH₂)ₙR⁴R⁵, -(CH₂)ₙC(O)NR⁴R⁵, -(CH₂)ₙN(R⁴)C(O)R⁶, -(CH₂)ₙS(O)₂NR⁴R⁵, -(CH₂)ₙN(R⁴)S(O)₂R⁶, -(CH₂)ₙS(O)₂R⁶, -(CH₂)ₙN(R⁴)S(O)₂R⁶, - (CH₂)ₙN(R⁴)C(O)R⁶ or-(CH₂)ₙC(O)alkyl; or where X is NR⁸ and Y is C(R⁹)₂, R⁸ may combine with R¹ to form a benzoquinuclidine group;
R⁹ is -H or R¹;
Ar is aryl or heteroaryl, each of which may be optionally substituted by R²;
Z is a bond, O, S, NR⁷ or CH₂;
m is 0, 1 or 2;
n is an integer value from 1 to 6;
q and r are independently selected from 0, 1, 2 or 3;
s is 0, 1, 2 or 3; and
X and Y are selected from the following combinations:
| X | Y |
|---|---|
| N | CR⁹ |
| NR⁸ | C(R⁹)₂ |
| CR⁹ | N |
| C(R⁹)₂ | NR⁸ |
with the proviso that said compound of formula (I) is not a compound selected from: *N*-{3-[(*N*,*N*-Dimethylamino)methyl]-1,2,3,4-tetrahydro-7-quinolinyl}-4-biphenylcarboxamide; *N*-{3-[(*N*,*N*-Dimethylamino)methyl]-1-formyl-1,2,3,4-tetrahydro-7-quinolinyl}-4-biphenylcarboxamide; *N*-{1-Acetyl-3-[(*N*,*N*-dimethylamino)methyl]-1,2,3,4-tetrahydro-7-quinolinyl}-4-biphenylcarboxamide; *N*-{3-[(*N*,*N*-Dimethylamino)methyl]-1-methylsulfonyl-1,2,3,4-tetrahydro-7-quinolinyl}-4-biphenylcarboxamide; 5-amino-*N*-isoquinolin-5-yl-1-[3-(trifluoromethyl)phenyl]-1H-pyrazole-3-carboxamide; 5-methyl-*N*-quinolin-8-yl-1-[3-(trifluoromethyl)phenyl]-1*H*-pyrazole-3-carboxamide, 5-methyl-*N*-quinolin-7-yl-1-[3-trifluoromethyl)phenyl]-1*H*-pyrazole-3-carboxam ide, 5-methyl-*N*-quinolin-3-yl-1-[3-(trifluoromethyl)phenyl]-1*H*-pyrazole-3-carboxamide, *N*-isoquinolin-5-yl-5-methyl-1-[3-(trifluoromethyl)phenyl]-1*H*-pyrazole-3-carboxamide, 5-methyl-*N*-quinolin-5-yl-1-[3-(trifluoromethyl)phenyl]-1*H*-pyrazole-3-carboxamide, 1-(3-chlorophenyl)-*N*-isoquinolin-5-yl-5-methyl-1*H*-pyrazole-3-carboxam ide, *N*-isoquinolin-5-yl-1-(3-methoxyphenyl)-5-methyl-1*H*-pyrazole-3-carboxam ide, 1-(3-fuorophenyl)-*N*-isoquinolin-5-yl-5-methyl-1*H*-pyrazole-3-carboxam ide, 1-(2-chloro-5-trifluoromethylphenyl)-*N*-isoquinolin-5-yl-5-methyl-1*H*-pyrazole-3-carboxamide, 5-methyl-*N*-(3-methylisoquinolin-5-yl)-1-[3-(trifluoromethyl)phenyl]-1*H*-pyrazole-3-carboxamide, and 5-methyl-*N*-(1,2,3,4-tetrahydroisoquinolin-5-yl)-1-[3-(trifluoromethyl)phenyl]-1*H-*pyrazole-3-carboxamide.

2. A compound of formula (I), as claimed in claim 1, of formula (IA), 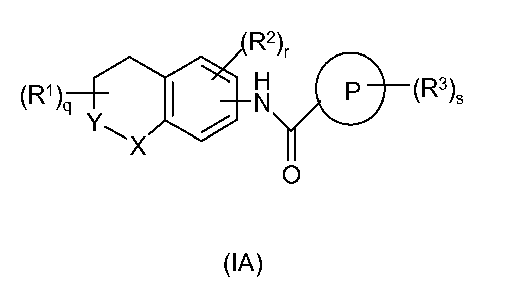 or a pharmaceutically acceptable salt or solvate thereof,
wherein, P is selected from phenyl, heteroaryl or heterocyclyl;
R¹ and R² are independently selected from halo, alkyl, alkoxy, cycloalkyl, aralkyl, aralkoxy, cycloalkylalkyl, cycloalkylalkoxy, -CN, -NO₂, -OH, =O, -OCF₃, -CF₃, - NR⁴R⁵, -S(O)ₘR⁶, -S(O)₂NR⁴R⁵, -OS(O)₂R⁶, -OS(O)₂CF₃, -O(CH₂)ₙNR⁴R⁵, -C(O)CF₃, -C(O)alkyl, -C(O)cycloalkyl, -C(O)aralkyl, -C(O)Ar, -C(O)(CH₂)ₙOR⁶, - C(O)(CH₂)ₙNR⁴R⁵, -C(O)alkoxy, -C(O)NR⁴R⁵, -(CH₂)ₙC(O)alkoxy, - (CH₂)ₙOC(O)R⁶, -O(CH₂)ₙOR⁶, -(CH₂)ₙOR⁶, -(CH₂)ₙR⁴R⁵, - (CH₂)ₙC(O)NR⁴R⁵, -(CH₂)ₙN(R⁴)C(O)R⁶, -(CH₂)ₙS(O)₂NR⁴R⁵, - (CH₂)ₙN(R⁴)S(O)₂R⁶, -ZAr, -(CH₂)ₙS(O)₂R⁶, -(OCH₂)ₙS(O)₂R⁶, - N(R⁴)S(O)₂R⁶, -N(R⁴)C(O)R⁶, -(CH₂)ₙN(R⁴)S(O)₂R⁶, -(CH₂)ₙN(R⁴)C(O)R⁶ or -(CH₂)ₙC(O)alkyl;
R³ is selected from alkyl, -CF₃, halo, phenyl, cyclohexyl, benzo[1,3]dioxolyl morpholinyl, pyridyl, pyrimidinyl, pyrazinyl, piperazinyl piperidinyl, pyridizinyl, thienyl, furyl, pyrazolyl, pyrrolyl, triazolyl, indanyl, imidazolyl, oxazolyl, thiazolyl, oxadiazolyl, isothiazolyl, isoxazolyl or thiadiazolyl; wherein said alkyl, alkoxy, phenyl, cyclohexyl, benzo[1,3]dioxolyl, morpholinyl, pyridyl, pyrimidinyl, pyrazinyl, piperazinyl, piperidinyl, pyridizinyl, thienyl, furyl, pyrazolyl, pyrrolyl, triazolyl, indanyl, imidazolyl, oxazolyl, thiazolyl, oxadiazolyl, isothiazolyl, isoxazolyl and thiadiazolyl groups may be optionally substituted by one or more groups, which may be the same or different, selected from R²;
R⁴ and R⁵ may be the same or different and represent -H or alkyl or R⁴ and R⁵ together with the nitrogen atom to which they are attached form a heterocyclic ring;
R6 is -H, alkyl or aryl;
R⁷ is -H, alkyl or aryl;
R⁸ is selected from -H, alkyl, hydroxyalkyl, cycloalkyl, aralkyl, alkoxyalkyl, cycloalkylalkyl, heterocyclylalkyl, -S(O)ₘR⁶, -C(O)CF₃, -C(O)alkyl, - C(O)cycloalkyl, -C(O)aralkyl, -C(O)Ar, -C(O)(CH₂)ₙOR⁶, -C(O)(CH₂)ₙNR⁴R⁵, - C(O)alkoxy, -C(O)NR⁴R⁵, -(CH₂)ₙC(O)alkoxy, -(CH₂)ₙOC(O)R⁶, -(CH₂)ₙOR⁶, - (CH₂)ₙR⁴R⁵, -(CH₂)ₙC(O)NR⁴R⁵, -(CH₂)ₙN(R⁴)C(O)R⁶, -(CH₂)ₙS(O)₂NR⁴R⁵, -(CH₂)ₙN(R⁴)S(O)₂R⁶, -(CH₂)ₙS(O)₂R⁶, -(CH₂)ₙN(R⁴)S(O)₂R⁶, - (CH₂)ₙN(R⁴)C(O)R⁶ or-(CH₂)ₙC(O)alkyl; or where X is NR⁸ and Y is C(R⁹)₂, R⁸ may combine with R¹ to form a benzoquinuclidine group;
R⁹ is -H or R¹.
Ar is aryl or heteroaryl, each of which may be optionally substituted by R²;
Z is a bond, O, S, NR⁷ or CH₂;
m is 0, 1 or 2;
n is an integer value from 1 to 6;
q and r are independently selected from 0, 1, 2 or 3;
s is 0, 1, 2 or 3; and
X is C(R⁹)₂ and Y is NR⁸ or X is NR⁸ and Y is C(R⁹)₂; with the proviso that said compound of formula (I) is not a compound selected from: *N*-{3-[(*N*,*N*-Dimethylamino)methyl]-1,2,3,4-tetrahydro-7-quinolinyl}-4-biphenylcarboxamide; *N*-{3-[(*N*,*N*-Dimethylamino)methyl]-1-formyl-1,2,3,4-tetrahydro-7-quinolinyl}-4-biphenylcarboxamide; *N*-{1-Acetyl-3-[(*N*,*N*-dimethylamino)methyl]-1,2,3,4-tetrahydro-7-quinolinyl}-4-biphenylcarboxamide; *N*-{3-[(*N*,*N*-Dimethylamino)methyl]-1-methylsulfonyl-1,2,3,4-tetrahydro-7-quinolinyl}-4-biphenylcarboxamide; and 5-methyl-*N*-(1,2,3,4-tetrahydroisoquinolin-5-yl)-1-[3-(trifluoromethyl)phenyl]-1*H-*pyrazole-3-carboxamide.

3. A compound of formula (I), as claimed in claim 1, of formula (IB), 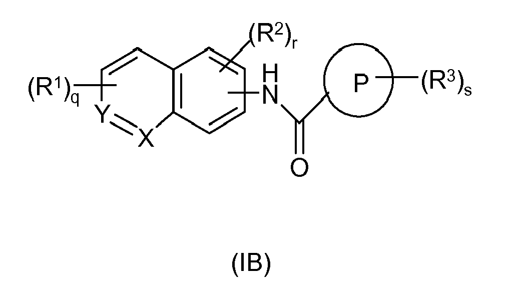 or a pharmaceutically acceptable salt or solvate thereof,
wherein, P is selected from phenyl, heteroaryl or heterocyclyl;
R¹ and R² are independently selected from halo, alkyl, alkoxy, cycloalkyl, aralkyl, aralkoxy, cycloalkylalkyl, cycloalkylalkoxy, -CN, -NO₂, -OH, -OCF₃, -CF₃, - NR⁴R⁵, -S(O)ₘR⁶, -S(O)₂NR⁴R⁵, -OS(O)₂R⁶, -OS(O)₂CF₃, -O(CH₂)ₙNR⁴R⁵, -C(O)CF₃, -C(O)alkyl, -C(O)cycloalkyl, -C(O)aralkyl, -C(O)Ar, -C(O)(CH₂)ₙOR⁶, - C(O)(CH₂)ₙNR⁴R⁵, -C(O)alkoxy, -C(O)NR⁴R⁵, -(CH₂)ₙC(O)alkoxy, - (CH₂)ₙOC(O)R⁶, -(CH₂)ₙOR⁶, -(CH₂)ₙR⁴R⁵, -(CH₂)ₙC(O)NR⁴R⁵, - (CH₂)ₙN(R⁴)C(O)R⁶, -(CH₂)ₙS(O)₂NR⁴R⁵, -(CH₂)ₙN(R⁴)S(O)₂R⁶, -ZAr, - (CH₂)ₙS(O)₂R⁶, -(OCH₂)ₙS(O)₂R⁶, -N(R⁴)S(O)₂R⁶, -N(R⁴)C(O)R⁶, - (CH₂)ₙN(R⁴)S(O)₂R⁶, -(CH₂)ₙN(R⁴)C(O)R⁶ or-(CH₂)ₙC(O)alkyl;
R³ is selected from halo, -CF₃, alkyl, alkoxy, -O(CH₂)ₙOR⁶, -O(CH₂)ₙNR⁴R⁵, phenyl, cyclohexyl, benzo[1,3]dioxolyl, morpholinyl, pyridyl, pyrimidinyl, pyrazinyl, piperazinyl, piperidinyl, pyridizinyl, thienyl, furyl, pyrazolyl, pyrrolyl, triazolyl, imidazolyl, oxazolyl, thiazolyl, oxadiazolyl, isothiazolyl, isoxazolyl or thiadiazolyl; which alkyl, alkoxy, phenyl, cyclohexyl, benzo[1,3]dioxolyl, morpholinyl, pyridyl, pyrimidinyl, pyrazinyl, piperazinyl, piperidinyl, pyridizinyl, thienyl, furyl, pyrazolyl, pyrrolyl, triazolyl, imidazolyl, oxazolyl, thiazolyl, oxadiazolyl, isothiazolyl, isoxazolyl and thiadiazolyl groups may be optionally substituted by one or more groups, which may be the same or different, selected from R²;
R⁴ and R⁵ may be the same or different and represent -H or alkyl or R⁴ and R⁵ together with the nitrogen atom to which they are attached form a heterocyclic ring;
R6 is -H, alkyl or aryl;
R⁷ is -H, alkyl or aryl;
Ar is aryl or heteroaryl; each of which may be optionally substituted by R²;
X and Y are selected from CR⁹ and N with the proviso that X and Y may not be the same;
Z is a bond, O, S, NR⁷ or CH₂;
m is 0, 1 or 2;
n is an integer value from 1 to 6;
q and r are independently selected from 0, 1, 2 or 3; and
s is 0, 1, 2 or 3; with the proviso that said compound of formula (IB) is not a compound selected from: 5-amino-*N*-isoquinolin-5-yl-1-[3-(trifluoromethyl)phenyl]-1*H*-pyrazole-3-carboxamide; 5-methyl-*N*-quinolin-8-yl-1-[3-(trifluoromethyl)phenyl]-1*H*-pyrazole-3-carboxamide, 5-methyl-*N*-quinolin-7-yl-1-[3-trifluoromethyl)phenyl]-1*H*-pyrazole-3-carboxam ide, 5-methyl-*N*-quinolin-3-yl-1-[3-(trifluoromethyl)phenyl]-1*H*-pyrazole-3-carboxamide, *N*-isoquinolin-5-yl-5-methyl-1-[3-(trifluoromethyl)phenyl]-1*H*-pyrazole-3-carboxamide, 5-methyl-*N*-quinolin-5-yl-1-[3-(trifluoromethyl)phenyl]-1*H*-pyrazole-3-carboxamide, 1-(3-chlorophenyl)-*N*-isoquinolin-5-yl-5-methyl-1*H*-pyrazole-3-carboxamide, *N*-isoquinolin-5-yl-1-(3-methoxyphenyl)-5-methyl-*H*-pyrazole-3-carboxamide, 1-(3-fuorophenyl)-*N*-isoquinolin-5-yl-5-methyl-1*H*-pyrazole-3-carboxamide, 1-(2-chloro-5-trifluoromethylphenyl)-*N*-isoquinolin-5-yl-5-methyl-1*H*-pyrazole-3-carboxamide, 5-methyl-*N*-(3-methylisoquinolin-5-yl)-1-[3-(trifluoromethyl)phenyl]-1*H*-pyrazole-3-carboxamide; and N-[3-[2-(d iethylam ino)ethyl]-1,2-d ihyd ro-4-methyl-2-oxo-7-q u inol inyl]-4-phenyl-1-piperazinecarboxamide.

4. A compound of formula (I) or a pharmaceutically acceptable salt or solvate thereof, as claimed in claim 1, substantially as hereinbefore described with reference to any one of the Examples.

5. A process for the preparation of a compound of formula (I) or a pharmaceutically acceptable salt or solvate thereof, which process comprises: (a) reacting a compound of formula (II): 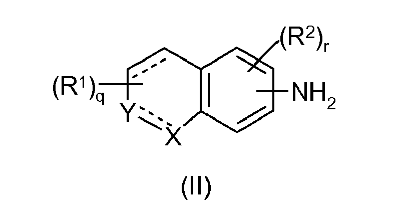 wherein, R¹, R², q, r, X and Y are as defined in relation to formula (I), with a compound of formula (III): 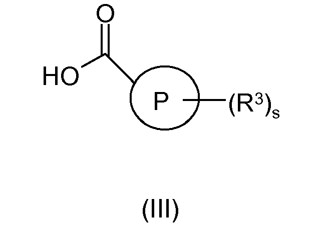 wherein, P, R³ and s are as defined in relation to formula (I) and thereafter, as necessary, carrying out one or more of the following reactions:
(i) converting one compound of formula (I) into another compound of formula (I); (ii) removing any protecting group;
(iii) preparing a salt or a solvate of the compound so formed.

6. A compound of formula (I) or a pharmaceutically acceptable salt or solvate thereof, as claimed in claim 1, for use as an active therapeutic substance.

7. A method for the treatment or prophylaxis of disorders in which antagonism of the Vanilloid (VR1) receptor is beneficialin mammals including humans, which method comprises administering to a mammal in need thereof a therapeutically effective amount of a compound of formula (I) or a pharmaceutically acceptable salt or solvate thereof, as claimed in claim 1.

8. Use of a compound of formula (I) or a pharmaceutically acceptable salt or solvate thereof, as claimed in claim 1, in the manufacture of a medicament for the treatment or prophylaxis of disorders in which antagonism of the Vanilloid (VR1) receptor is beneficial.

9. A pharmaceutical composition, which comprises a compound of formula (I) or a pharmaceutically acceptable salt or solvate thereof, as claimed in claim 1, and a pharmaceutically acceptable carrier or excipient therefor.
